# EUROPÄISCHE PATENTSCHRIFT

(11) **EP 2 766 349 B1**
(45) Veröffentlichungstag und Bekanntmachung des Hinweises auf die Patenterteilung: **01.06.2016**
(21) Anmeldenummer: 12708536.3
(22) Anmeldetag: 07.03.2012
(51) Int. Cl.: C07D 291/08, A61K 31/54, C07D 515/04, A61P 3/10

(54) **MIT CARBOZYKLEN ODER HETEROZYKLEN SUBSTITUIERTE OXATHIAZINDERIVATE, VERFAHREN ZU DEREN HERSTELLUNG, DIESE VERBINDUNGEN ENTHALTENDE ARZNEIMITTEL UND DEREN VERWENDUNG**
OXATHIAZINE DERIVATIVES SUBSTITUTED WITH CARBOCYCLES OR HETEROCYCLES, METHOD FOR PRODUCING SAME, DRUGS CONTAINING SAID COMPOUNDS, AND USE THEREOF
DÉRIVÉS D'OXATHIAZINE SUBSTITUÉS PAR DES CARBOCYCLES OU DES HÉTÉROCYCLES, LEUR PROCÉDÉ DE PRÉPARATION, MÉDICAMENTS CONTENANT CES COMPOSÉS ET LEUR UTILISATION

(30) Priorität: 08.03.2011 EP 11305240
(43) Veröffentlichungstag der Anmeldung: 20.08.2014
(73) Patentinhaber: SANOFI, 75008 Paris (FR)
(72) Erfinder: BOEHME, Thomas, 65926 Frankfurt am Main (DE); ENGEL, Christian, 65926 Frankfurt am Main (DE); GUESSREGEN, Stefan, 65926 Frankfurt am Main (DE); HAACK, Torsten, 65926 Frankfurt am Main (DE); KRETZSCHMAR, Gerhard, 65926 Frankfurt am Main (DE); RITTER, Kurt, 65926 Frankfurt am Main (DE); TSCHANK, Georg, 65926 Frankfurt am Main (DE)
(74) Vertreter: Essler, Frank
(86) Internationale Anmeldenummer: PCT/EP2012/053935
(87) Internationale Veröffentlichungsnummer: WO 2012/120052

(56) Entgegenhaltungen:
- WO-A1-02/11722
- WO-A2-2008/073956
- SUZUE SEIGO ET AL: "Studies on Hypoglycemic Agents. IV. Synthesis of 1,4,3-Benzoxathiazine-4,4-dioxides", CHEMICAL AND PHARMACEUTICAL BULLETIN, PHARMACEUTICAL SOCIETY OF JAPAN, TOKYO, JP, Bd. 16, Nr. 5, 25. Mai 1968 (1968-05-25), Seiten 806-813, XP009109825, ISSN: 0009-2363
- IWAKAWA TSUNEO ET AL: "Cycloaddition in Synthesis of Sulfonamide Derivatives. IV. One-Pot Synthesis of 3-Dimethylamino-4,1,2-benzoxathiazine 1,1-Dioxides, 3-Methoxy-4-methyl-1,2,4-benzothiadiazine 1,1-Dioxide and 3-Dimethylamino-1,4,2-benzodithiazine 1,1-Dioxides", CHEMICAL AND PHARMACEUTICAL BULLETIN, PHARMACEUTICAL SOCIETY OF JAPAN, TOKYO, JP, Bd. 39, Nr. 8, 25. August 1991 (1991-08-25), Seiten 1939-1943, XP009109826, ISSN: 0009-2363

## Beschreibung

Die Erfindung betrifft mit Carbozyklen oder Heterozyklen substituierte Oxathiazinderivate und deren physiologisch verträgliche Salze.

In Suzue Seigo et al., Chem. Phar. Bull. 16(5), 806-813 (1986) sind Oxathiazinverbindungen zur Behandlung von Hypoglykämie beschrieben.

Der Erfindung lag die Aufgabe zugrunde, Verbindungen zur Verfügung zu stellen, die eine therapeutisch verwertbare Wirkung entfalten. Insbesondere bestand die Aufgabe darin, neue Verbindungen zu finden, die zur Behandlung von Diabetes, Hyperglykämie, Insulin Resistenz, Adipositas, Lipidstoffwechselstörungen oder anderen Erkrankungen geeignet sind.

### Die Erfindung betrifft daher die Verbindung der Formel I

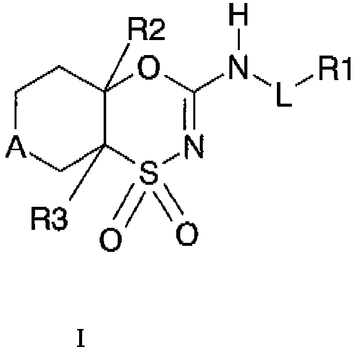

worin bedeuten
- A: Bindung, CH₂, CF₂, O;
- L: Bindung, C(R5)(R6), N(R5);
- R1: (C₃-C₈)-Carbozyklus, wobei der Carbozyklusrest ein bis 3-fach substituiert sein kann mit F, Cl, Br, I, OH, -(C₁-C₆)-Alkylen-OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁- C₆)-Alkyl)₂, SO₂-CH₃, SO₂-NH₂, SO₂-NH(C₁-C₆)-Alkyl, SO₂-N((C₁-C₆)- Alkyl)₂, COOH, COO-(C₁-C₆)-Alkyl, CONH₂, CONH(C₁-C₆)-Alkyl, CON((C₁-C₆)-Alkyl)₂, SF₅;
(C₆-C₁₀)-Aryl, wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁- C₆)-Alkyl, NH₂, NH(C₁-C₆)-Alkyl, N((C₁-C₆)-Alkyl)₂, SO₂-CH₃, SO₂- NH₂, SO₂-NH(C₁-C₆)-Alkyl, SO₂-N((C₁-C₆)-Alkyl)₂, COOH, COO-(C₁- C₆)-Alkyl, CONH₂, CONH(C₁-C₆)-Alkyl, CON((C₁-C₆)-Alkyl)₂, SF₅;
4-12-gliedriger Heterozyklus, wobei der Heterozyklylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO- (C₁-C₆)-Alkyl, CONH₂, CONH(C₁-C₆)-Alkyl, CON((C₁-C₆)-Alkyl)₂, SF₅;
   und wobei der Heterozyklus mit einem weiteren Ring oder Ringsystem anneliert sein kann;
- R2, R3: unabhängig voneinander H, (C₁-C₆)-Alkyl;
- R5, R6: unabhängig voneinander H, (C₁-C₆)-Alkyl, (C₃-C₈)-Cycloalkyl, (C(R7)(R8))ₙ-O-(CO)-N(R7)(R8);
- R7, R8: unabhängig voneinander H, (C₁-C₆)-Alkyl;
- n: 0, 1, 2;
sowie deren pharmazeutisch verträgliche Salze.

Bevorzugt sind Verbindungen der Formel I, worin bedeuten
- A: Bindung, CH₂, CF₂;
- L: Bindung, C(R5)(R6), N(R5);
- R1: (C₃-C₈)-Carbozyklus,
wobei der Carbozyklusrest ein bis 3-fach substituiert sein kann mit F, Cl, Br, I, OH, -(C₁-C₆)-Alkylen-OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁- C₆)-Alkyl)₂, SO₂-CH₃, SO₂-NH₂, SO₂-NH(C₁-C₆)-Alkyl, SO₂-N((C₁-C₆)- Alkyl)₂, COOH, COO-(C₁-C₆)-Alkyl, CONH₂, CONH(C₁-C₆)-Alkyl, CON((C₁-C₆)-Alkyl)₂, SF₅;
Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁- C₆)-Alkyl, NH₂, NH(C₁-C₆)-Alkyl, N((C₁-C₆)-Alkyl)₂, SO₂-CH₃, SO₂- NH₂, SO₂-NH(C₁-C₆)-Alkyl, SO₂-N((C₁-C₆)-Alkyl)₂, COOH, COO-(C₁- C₆)-Alkyl, CONH₂, CONH(C₁-C₆)-Alkyl, CON((C₁-C₆)-Alkyl)₂, SF₅;
ein Heterozyklus, ausgewählt aus der Gruppe Thiophen, Pyran, Tetrahydropyran, Piperidin, Thiopyran, Tetrahydrothiopyran, Pyrrol, Tetrahydropyrrol, Azepan oder (2,3,4,5,6,7,8,9,10,11-Decaborabicyclo[8.1.1.]dodecan,
wobei der Heterozyklylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO- (C₁-C₆)-Alkyl, CONH₂, CONH(C₁-C₆)-Alkyl, CON((C₁-C₆)-Alkyl)₂, SF₅;
und wobei der Heterozyklus mit einem weiteren Ring oder Ringsystem anneliert sein kann;
- R2, R3: unabhängig voneinander H, (C₁-C₆)-Alkyl;
- R5, R6: unabhängig voneinander H, (C₁-C₆)-Alkyl, (C₃-C₈)-Cycloalkyl, (C₁-C₆)-Alkylen-(C₃-C₈)-Cycloalkylen, (C(R7)(R8))ₙ-O-(CO)-N(R7)(R8);
- R7, R8: unabhängig voneinander H, (C₁-C₆)-Alkyl;
- n: 0, 1, 2;
sowie deren pharmazeutisch verträgliche Salze.

Besonders bevorzugt sind Verbindungen der Formel I, worin bedeuten
- A: Bindung, CH₂, CF₂;
- L: Bindung, C(R5)(R6), N(R5);
- R1: (C₃-C₈)-Carbozyklus, wobei der Carbozyklusrest ein bis 3-fach substituiert sein kann mit F, Cl, Br, I, OH, -(C₁-C₆)-Alkylen-OH, CF₃, NO₂, CN, OCF₃, (C₁-C₆)-Alkyl, CONH₂, CONH(C₁-C₆)-Alkyl, CON((C₁-C₆)-Alkyl)₂;
Phenyl,
wobei der Phenylrest ein bis 3-fach substituiert sein kann mit F, Cl, Br, I, OH, -(C₁-C₆)-Alkylen-OH, CF₃, NO₂, CN, OCF₃, (C₁-C₆)-Alkyl, CONH₂, CONH(C₁-C₆)-Alkyl, CON((C₁-C₆)-Alkyl)₂;
ein Heterozyklus, ausgewählt aus der Gruppe Carboran, Thiophen, Tetrahydropyran, Piperidin, Thiochroman, Hexahydro-Cyclopenta [c]pyrrol, Azepan oder (2,3,4,5,6,7,8,9,10,11-Decabora bicyclo[8.1.1.]dodecan, wobei der Heterozyklylrest ein bis 3-fach substituiert sein kann mit F, Cl, Br, I, OH, -(C₁-C₆)-Alkylen-OH, CF₃, NO₂, CN, OCF₃, (C₁-C₆)-Alkyl, CONH₂, CONH(C₁-C₆)-Alkyl, CON((C₁-C₆)-Alkyl)₂;
- R2, R3: unabhängig voneinander H, (C₁-C₆)-Alkyl;
- R5, R6: unabhängig voneinander H, (C₁-C₆)-Alkyl;
sowie deren pharmazeutisch verträgliche Salze.

In einer Ausführungsform sind Verbindungen der Formel I bevorzugt, in denen A eine Bindung ist.

In einer Ausführungsform sind Verbindungen der Formel I bevorzugt, in denen A gleich CH₂ ist.

In einer Ausführungsform sind Verbindungen der Formel I bevorzugt, in denen A gleich CF₂ ist.

In einer Ausführungsform sind Verbindungen der Formel I bevorzugt, in denen A gleich O ist.

In einer Ausführungsform sind Verbindungen der Formel I bevorzugt, in denen L eine Bindung ist.

In einer Ausführungsform sind Verbindungen der Formel I bevorzugt, in denen L gleich C(R5)(R6) ist.

In einer Ausführungsform sind Verbindungen der Formel I bevorzugt, in denen L gleich N(R5) ist.

In einer Ausführungsform sind Verbindungen der Formel I bevorzugt, in denen R1 gleich (C₃-C₈)-Carbozyklus ist.

In einer Ausführungsform sind Verbindungen der Formel I bevorzugt, in denen R1 gleich (C₆-C₁₀)-Aryl ist.

In einer Ausführungsform sind Verbindungen der Formel I bevorzugt, in denen R1 gleich 4-12-gliedriger Heterozyklus ist.

Können Reste oder Substituenten mehrfach in den Verbindungen der Formeln I auftreten (wie z.B. R7), so können sie alle unabhängig voneinander die angegebene Bedeutung haben und gleich oder verschieden sein.

Die Erfindung bezieht sich auf Verbindungen der Formel I, in Form ihrer Tautomere, Racemate, racemischen Mischungen, Stereoisomerengemische, reinen Stereoisomere, Diastereoisomerengemische, reinen Diastereoisomere. Die Trennung der Gemische erfolgt zum Beispiel auf chromatographischem Weg.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate wie hierin beschrieben.

Unter einem Alkylrest wird eine geradkettige oder verzweigte Kohlenwasserstofflkette mit einem bis acht Kohlenstoffen verstanden, wie z.B. Methyl, Ethyl, iso-Propyl, tert.-Butyl, Hexyl, Heptyl, Octyl. Die Alkylreste können einfach oder mehrfach wie oben beschrieben substituiert sein.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen wie oben beschrieben substituiert sein.

Unter Heterozyklus bzw. heterozyklischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterocyclische Rest mit einem weiteren Ring oder Ringsystem anelliert ist. Der Heterocylus bzw. der heterocyclische Rest kann gesättigt, teilweise gesättigt oder aromatisch sein.

Geeignete "Heterocyclen" bzw. "heterocyclische Reste" sind Acridinyl, Azepanyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, 5,6-Dihydro-4H-cyclopentathiazol-2-yl, 4,5-Dihydro-thiazol-2-yl, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, 4,5,6,7-Tetrahydrobenzooxazol-2-yl, 4,5,6,7-Tetrahydro-benzothiazol-2-yl, 4,5,6,7-Tetrahydro-benzoimidazol-2-yl, 4,5,6,7-Tetrahydro-pyrazolo[1,5-a]pyridin-2-yl, Tetrahydrofuranyl, Tetrahydropyranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazinyl, Triazolyl, Tetrazolyl, Thiazolo[4,5-b]pyridinyl, Thieno[2,3-d]thiazol-2-yl, Tropanyl und Xanthenyl.

Die Heterozyklen bzw. heterozyklischen Reste können ein oder mehrfach mit geeigneten Gruppen wie oben beschrieben substituiert sein.,

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im Allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:
Alle Antidiabetika, die in der Roten Liste 2009, Kapitel 12 genannt sind; alle Abmagerungsmittel/Appetitzügler, die in der Roten Liste 2009, Kapitel 1 genannt sind; alle Diuretika, die in der Roten Liste 2009, Kapitel 36 genannt sind; alle Lipidsenker, die in der Roten Liste 2009, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Erfolgt die Gabe der Wirkstoffe durch getrennte Verabreichung der Wirkstoffe, so kann diese gleichzeitig oder nacheinander erfolgen. Die meisten der nachfolgend aufgeführten Wirkstoffe sind in USP Dictionary of USAN and International Drug Names, US Pharmacopeia, Rockville 2006, offenbart.

Antidiabetika umfassen Insulin und Insulinderivate, wie z.B. Lantus^{®} (siehe www.lantus.com) oder HMR 1964 oder Levemir® (insulin detemir), Humalog^{(R)} (Insulin Lispro), Humulin^{(R)}, VIAject™ oder solche, wie sie in WO2005005477 (Novo Nordisk) beschrieben sind, schnell wirkende Insuline (siehe US 6,221,633), inhalierbare Insuline, wie z. B. Exubera^{®}, Nasulin^{™}, oder orale Insuline, wie z. B. IN-105 (Nobex) oder Oral-lyn ^{™} (Generex Biotechnology) oder Technosphere^{(R)} Insulin (MannKind) oder Cobalamin™ orales Insulin oder Insuline, wie sie in WO2007128815, WO2007128817 beschrieben sind oder Insuline, die transdermal verabreicht werden können;

GLP-1-Derivate und GLP-1 Agonisten wie z.B. Exenatide, Liraglutide oder diejenigen die in WO 98/08871, WO2005027978, WO2006037811, WO2006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), AVE-0010, BIM-51077 (R-1583, ITM-077), PC-DAC:Exendin-4 (ein Exendin-4 Analogon, welches kovalent an rekombinantes menschliches Albumin gebunden ist), CVX-73, CVX-98 und CVx-96 (GLP-1 Analoga, welche kovalent an einen monoklonalen Antikörper gebunden sind, der spezifische Bindungsstellen für das GLP-1 Peptid aufweist), CNTO-736 (ein GLP-1 Analogon, welches an eine Domäne gebunden ist, welche den Fc-Teil eines Antikörpers beinhaltet), PGC-GLP-1 (GLP-1 gebunden an einen Nanocarrier), Agonisten wie sie z.B. bei D. Chen et al., Proc. Natl. Acad. Sci. USA 104 (2007) 943 beschrieben sind, solche wie sie in WO2006124529, WO2007124461 beschrieben sind, Peptide wie z.B. Obinepitide (TM-30338), Amylinrezeptor Agonisten, wie sie z.B. in WO2007104789 beschrieben sind, Analoga des humanen GLP-1, wie sie in WO2007120899 beschrieben sind, sowie oral wirksame hypoglykämische Wirkstoffe.

Antidiabetika umfassen auch Agonisten des Glukose-abhängigen insulinotropen Polypeptids (GIP) Rezeptors wie sie z.B. in WO2006121860 beschrieben sind.

Antidiabetika umfassen auch Analoga und Derivate des Fibroblastenwachstumsfaktors 21 (FGF-21, fibroblast growth factor 21).

Die oral wirksamen hypoglykämischen Wirkstoffe umfassen vorzugsweise Sulfonylharnstoffe,
Biguanidine,
Meglitinide,
Oxadiazolidindione,
Thiazolidindione,
PPAR- und RXR-Modulatoren,
Glukosidase-Inhibitoren,
Hemmstoffe der Glykogenphosphorylase,
Glukagonrezeptor-Antagonisten,
Glukokinaseaktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT),
GLP-1 -Agonisten,
Kaliumkanalöffner, wie z.B. Pinacidil, Cromakalim, Diazoxid oder solche wie sie bei R. D. Carr et al., Diabetes 52, 2003, 2513.2518, bei J. B. Hansen et al, Current Medicinal Chemistry 11, 2004, 1595-1615, bei T. M. Tagmose et al., J. Med. Chem. 47, 2004, 3202-3211 oder bei M. J. Coghlan et al., J. Med. Chem. 44, 2001, 1627-1653 beschrieben sind, oder diejenigen, die in WO 97/26265 und WO 99/03861 von Novo Nordisk A/S offenbart wurden,
Wirkstoffe, die auf den ATP-abhängigen Kaliumkanal der Betazellen wirken,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption,
Modulatoren der natrium-abhängigen Glukosetransporter 1 oder 2 (SGLT1, SGLT2),
Hemmstoffe der 11-beta-Hydroxysteroid-Dehydrogenase-1 (11β-HSD1),
Inhibitoren der Protein-Tyrosin-Phosphatase-1B (PTP-1B),
Nikotinsäurerezeptoragoni sten,
Inhibitoren der hormon-sensitiven bzw. endothelialen Lipasen,
Hemmstoffen der Acetyl-CoA Carboxylase (ACC1 und/oder ACC2) oder
Inhibitoren der GSK-3 beta.
Weiterhin sind umfasst den Fettstoffwechsel verändernde Verbindungen wie antihyperlipidämische Wirkstoffe und antilipidämische Wirkstoffe,
HMGCoA-Reduktase-Inhibitoren,
Farnesoid X Rezeptor (FXR) Antagonisten,
Fibrate,
Cholesterinresreptionsinhibitoren,
CETP-Inhibitoren,
Gallensäureresorptionsinhibitoren,
MTP-Inhibitoren,
Agonisten des Estrogenrezeptors gamma (ERR Agonisten),
Sigma-1 Rezeptorantagonisten,
Antagonisten des Somatostatin 5 Rezeptors (SST5 Rezeptor);
Verbindungen, die die Nahrungsmitteleinnahme verringern und
Verbindungen, die die Thermogenese erhöhen.

Bei einer Ausführungsform der Erfindung wird die Verbindung der Formel I in Kombination mit Insulin verabreicht.

Bei einer Ausführungsform wird die Verbindung der Formel I in Kombination mit einem Wirkstoff, der auf den ATP-abhängigen Kaliumkanal der Betazellen wirkt, z.B. Sulfonylharnstoffe, wie z.B. Tolbutamid, Glibenclamid, Glipizid, Gliclazide oder Glimepirid, verabreicht.

Bei einer Ausführungsform wird die Verbindung der Formel I in Kombination mit einer Tablette verabreicht, die sowohl Glimeprid enthält, welches schnell freigesetzt wird wie auch Metformin enthält, welches über einen längeren Zeitraum freigesetzt wird (wie z.B. in US2007264331 beschrieben).

Bei einer Ausführungsform wird die Verbindung der Formel I in Kombination mit einem Biguanid, wie z.B. Metformin, verabreicht.

Bei wieder einer Ausführungsform wird die Verbindung der Formel I in Kombination mit einem Meglitinid, wie z.B. Repaglinide, Nateglinid oder Mitiglinide verabreicht.

Bei einer weiteren Ausführungsform wird die Verbindung der Formel I mit einer Kombination von Mitiglinide mit einem Glitazon, z.B. Pioglitazon Hydrochlorid, verabreicht.

Bei einer weiteren Ausführungsform wird die Verbindung der Formel I mit einer Kombination von Mitiglinide mit einem alpha-Glukosidaseinhibitor verabreicht.

Bei einer weiteren Ausführungsform wird die Verbindung der Formel I in Kombination mit antidiabetischen Verbindungen, wie sie in WO2007095462, WO2007101060, WO2007105650 beschrieben sind, verabreicht.

Bei einer weiteren Ausführungsform wird die Verbindung der Formel I in Kombination mit antihypoglykämischen Verbindungen, wie sie in WO2007137008 beschrieben sind, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483, CS-011 (Rivoglitazon), DRL-17564, DRF-2593 (Balaglitazon) oder solchen, wie sie in WO2007060992, WO2007100027, WO2007103252, WO2007122970 beschrieben sind, verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.

Bei einer Ausführungsform der Erfindung wird die Verbindung der Formel I in Kombination mit Tandemact™, einer festen Kombination von Pioglitazon mit Glimeprid, verabreicht.

Bei einer weiteren Ausführungsform der Erfindung wird die Verbindung der Formel I in Kombination mit einer festen Kombination von Pioglitazon Hydrochlorid mit einem Angiotensin II Agonisten, wie z.B. TAK-536, verabreicht.

Bei einer Ausführungsform der Erfindung wird die Verbindung der Formel I in Kombination mit einem PPAR alpha Agonisten bzw. gemischten PPAR alpha/PPAR delta Agonisten, wie z.B. GW9578, GW-590735, K-111, LY-674, KRP-101, DRF-10945, LY-518674, CP-900691, BMS-687453, BMS-711939 oder solchen wie sie in WO2001040207, WO2002096894, WO2005097076, WO2007056771, WO2007087448, WO2007089667, WO2007089557, WO2007102515, WO2007103252, JP2007246474, WO2007118963, WO2007118964, WO2007126043 beschrieben sind, verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, CKD-501 (Lobeglitazon Sulfat), MBX-213 oder wie in WO 00/64888, WO 00/64876, WO03/020269, WO2007099553, US2007276041, WO2007085135, WO2007085136 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 oder wie sie in WO2006059744, WO2006084176, WO2006029699, WO2007039172-WO2007039178, WO2007071766, WO2007101864, US2007244094, WO2007119887 beschrieben sind, verabreicht.

Bei einer Ausführungsform der Erfindung wird die Verbindung der Formel I in Kombination mit einem pan-SPPARM (selective PPAR modulator alpha, gamma, delta), wie z.B. GFT-505, verabreicht.

Bei einer Ausführungsform wird die Verbindung der Formel I in Kombination mit Metaglidasen oder mit MBX-2044 oder anderen partiellen PPAR gamma Agonisten/Antagonisten verabrei cht.

Bei einer Ausführungsform wird die Verbindung der Formel I in Kombination mit einem α-Glukosidase-Inhibitor, wie z.B. Miglitol oder Acarbose oder solchen, wie sie z.B. in WO2007114532, WO2007140230 beschrieben sind, verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932 beschrieben, verabreicht.

Bei einer Ausführungsform wird die Verbindung der Formel I in Kombination mit GlukagonRezeptor-Antagonisten, wie z.B. A-770077 oder NNC-25-2504 oder wie in WO2004100875, WO2005065680, WO2006086488, WO2007106181, WO2007111864, WO2007120270, WO2007120284, WO2007123581, WO2007136577 beschrieben, verabreicht.

Bei einer weiteren Ausführungsform wird die Verbindung der Formel I in Kombination mit einer Antisense-Verbindung, z.B. ISIS-325568, verabreicht, welche die Produktion des Glukagonrezeptors inhibiert.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031, WO2004072066, WO2005080360, WO2005044801, WO2006016194, WO2006058923, WO2006112549, WO2006125972, WO2007017549, WO2007017649, WO2007007910, WO2007007040-42, WO2007006760-61, WO2007006814, WO2007007886, WO2007028135, WO2007031739, WO2007041365, WO2007041366, WO2007037534, WO2007043638, WO2007053345, WO2007051846, WO2007051845, WO2007053765, WO2007051847, WO2007061923, WO2007075847, WO2007089512, WO2007104034, WO2007117381, WO2007122482, WO2007125103, WO2007125105, US2007281942 beschrieben sind, verabreicht.

Bei einer Ausführungsform wird die Verbindung der Formel I in Kombination mit einem Inhibitor der Glukoneogenese, wie z. B. FR-225654, verabreicht.

Bei einer Ausführungsform wird die Verbindung der Formel I in Kombination mit Inhibitoren der Fructose-1,6-bisphosphatase (FBPase) wie z.B. CS-917 (MB-06322) oder MB-07803 oder solchen wie sie in WO2006023515, WO2006104030, WO2007014619, WO2007137962 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Sitagliptin Phosphat, Saxagliptin ((BMS-477118), GSK-823093, PSN-9301, SYR-322, SYR-619, TA-6666, TS-021, GRC-8200, GW-825964X, KRP-104, DP-893, ABT-341, ABT-279 oder ein anderes Salz davon, S-40010, S-40755, PF-00734200, BI-1356, PHX-1149, Alogliptin oder solchen Verbindungen wie sie in WO2003074500, WO2003106456, WO2004037169, WO200450658, WO2005037828, WO2005058901, WO2005012312, WO2005/012308, WO2006039325, WO2006058064, WO2006015691, WO2006015701, WO2006015699, WO2006015700, WO2006018117, WO2006099943, WO2006099941, JP2006160733, WO2006071752, WO2006065826, WO2006078676, WO2006073167, WO2006068163, WO2006085685, WO2006090915, WO2006104356, WO2006127530, WO2006111261, WO2007015767 (LY-2463665), WO2007024993, WO2007029086, WO2007063928, WO2007070434, WO2007071738, WO2007077508, WO2007087231, WO2007097931, WO2007099385, WO2007100374, WO2007112347, WO2007112669, WO2007113226, WO2007113634, WO2007115821, WO2007116092, US2007259900, EP1852108, US2007270492, WO2007126745, WO2007136603 beschrieben sind, verabreicht.

Bei einer Ausführungsform wird die Verbindung der Formel I in Kombination mit Janumet™, einer festen Kombination von Sitagliptin Phosphat mit Metformin Hydrochlorid, verabreicht.

Bei einer Ausführungsform wird die Verbindung der Formel I in Kombination mit Eucreas^{(R)}, einer festen Kombination von Vildagliptin mit Metformin Hydrochlorid, verabreicht.

Bei einer Ausführungsform wird die Verbindung der Formel I in Kombination mit einer Kombination eines DPP-IV-Inhibitors mit omega-3-Fettsäuren oder omega-3-Fettsäureestern, wie z.B. in WO2007128801 beschrieben, verabreicht.

Bei einer Ausführungsform wird die Verbindung der Formel I in Kombination mit einer die Insulinsekretion verstärkende Substanz, wie z. B. KCP-265 (WO2003097064), oder solchen wie sie in WO2007026761 beschrieben sind, verabreicht.

Bei einer Ausführungsform wird die Verbindung der Formel I in Kombination mit Agonisten des glucose-abhängigen insulinotropischen Rezeptors (GDIR) wie z. B. APD-668 verabreicht. Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268, SAR 7226, SGL-5083, SGL-5085, SGL-5094, ISIS-388626, Sergliflozin oder Dapagliflozin oder wie sie z. B. in WO2004007517, WO200452903, WO200452902, PCT/EP2005/005959, WO2005085237, JP2004359630, WO2005121161, WO2006018150, WO2006035796, WO2006062224, WO2006058597, WO2006073197, WO2006080577, WO2006087997, WO2006108842, WO2007000445, WO2007014895, WO2007080170, WO2007093610, WO2007126117, WO2007128480, WO2007129668, US2007275907, WO2007136116 oder von A. L. Handlon in Expert Opin. Ther. Patents (2005) 15(11), 1531-1540 beschrieben sind, verabreicht.

Bei einer Ausführungsform wird die Verbindung der Formel I in Kombination mit Hemmstoffen der 11-beta-Hydroxysteroid-Dehydrogenase-1 (11β-HSD1), wie z. B. BVT-2733, JNJ-25918646, INCB-13739, DIO-92 ((-)-Ketoconazol) oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005063247, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109, WO2006074244, WO2006078006, WO2006106423, WO2006132436, WO2006134481, WO2006134467, WO2006135795, WO2006136502, WO2006138508, WO2006138695, WO2006133926, WO2007003521, WO2007007688, US2007066584, WO2007029021, WO2007047625, WO2007051811, WO2007051810, WO2007057768, WO2007058346, WO2007061661, WO2007068330, WO2007070506, WO2007087150, WO2007092435, WO2007089683, WO2007101270, WO2007105753, WO2007107470, WO2007107550, WO2007111921, US2007207985, US2007208001, WO2007115935, WO2007118185, WO2007122411, WO2007124329, WO2007124337, WO2007124254, WO2007127688, WO2007127693, WO2007127704, WO2007127726, WO2007127763, WO2007127765, WO2007127901, US2007270424, JP2007291075, WO2007130898, WO2007135427 beschrieben sind, verabreicht.

Bei einer Ausführungsform wird die Verbindung der Formel I in Kombination mit Inhibitoren der Protein-Tyrosin-Phosphatase-1B (PTP-1B), wie sie z. B. in WO200119830-31, WO200117516, WO2004506446, WO2005012295, WO2005116003, WO2005116003, WO2006007959, DE 10 2004 060542.4, WO2007009911, WO2007028145, WO2007067612-615, WO2007081755, WO2007115058 beschrieben sind, verabreicht.

Bei einer Ausführungsform der Erfindung wird die Verbindung der Formel I in Kombination mit einem Agonisten des GPR109A (HM74A Rezeptor Agonisten; NAR-Agonisten (Nikotinsäurerezeptoragonisten)), wie z.B. Nicotinsäure oder "extended release niacin" in Verbindung mit MK-0524A (Laropiprant) oder MK-0524 oder solchen Verbindungen, wie sie in WO2006045565, WO2006045564, WO02006069242, WO2006085108, WO2006085112, WO2006085113, WO2006124490, WO2006113150, WO2007017261, WO2007017262, WO2007017265, WO2007015744, WO2007027532, WO2007092364, WO2007120575, WO2007134986 beschrieben sind, verabreicht.

Bei einer anderen Ausführungsform der Erfindung wird die Verbindung der Formel I in Kombination mit einer festen Kombination von Niacin mit Simvastatin verabreicht.

Bei einer anderen Ausführungsform der Erfindung wird die Verbindung der Formel I in Kombination mit Nicotinsäure oder "extended release niacin" in Verbindung mit MK-0524A (Laropiprant) verabreicht.

Bei einer weiteren Ausführungsform der Erfindung wird die Verbindung der Formel I in Kombination mit Nicotinsäure oder "extended release niacin" in Verbindung mit MK-0524A (Laropiprant) und mit Simvastatin verabreicht.

Bei einer anderen Ausführungsform der Erfindung wird die Verbindung der Formel I in Kombination mit einem Agonisten des GPR116, wie sie z.B. in WO2006067531, WO2006067532 beschrieben sind, verabreicht.

Bei einer Ausführungsform wird die Verbindung der Formel I in Kombination mit Modulatoren des GPR40, wie sie z.B. in WO2007013689, WO2007033002, WO2007106469, US2007265332, WO2007123225, WO2007131619, WO2007131620, WO2007131621, US2007265332, WO2007131622, WO2007136572 beschrieben sind, verabreicht.

Bei einer Ausführungsform wird die Verbindung der Formel I in Kombination mit Modulatoren des GPR119b wie sie z. B. in WO2004041274 beschrieben sind, verabreicht.

Bei einer Ausführungsform wird die Verbindung der Formel I in Kombination mit Modulatoren des GPR119 (G-Protein-gekoppelter Glukose-abhängiger insulinotroper Rezeptor), wie z.B. PSN-119-1 oder solchen wie sie z. B. in WO2005061489 (PSN-632408), WO2004065380, WO2006018662, WO2007003960-62 und WO2007003964, WO2007116229, WO2007116230 beschrieben sind, verabreicht.

Bei einer weiteren Ausführungsform wird die Verbindung der Formel I in Kombination mit Modulatoren des GPR120, wie sie z.B. in EP1688138 beschrieben sind, verabreicht.

Bei einer Ausführungsform wird die Verbindung der Formel I in Kombination mit Inhibitoren der hormon-sensitiven Lipase (HSL) und/oder Phospholipasen, wie z. B. in WO2005073199, WO2006074957, WO2006087309, WO2006111321, WO2007042178, WO2007119837 beschrieben, verabreicht.

Bei einer Ausführungsform wird die Verbindung der Formel I in Kombination mit Inhibitoren der endothelialen Lipase, wie z. B. in WO2007110216 beschrieben, verabreicht.

Bei einer Ausführungsform wird die Verbindung der Formel I in Kombination mit einem Phospholipase A2 Inhibitor wie z.B. Darapladib oder A-002 verabreicht.

Bei einer Ausführungsform wird die Verbindung der Formel I in Kombination mit Myricitrin, einem Lipase-Inhibitor (WO2007119827), verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, WO2005111018, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117, WO2007073117, WO2007083978, WO2007120102, WO2007122634, WO2007125109, WO2007125110 beschrieben.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Serum/Glucocorticoid regulierten Kinase (SGK), wie z. B. in WO2006072354, WO2007093264 beschrieben, verabreicht.

Bei einer Ausführungsform wird die Verbindung der Formel I in Kombination mit einem Inhibitor der Protein Kinase C beta (PKC beta), wie z. B. Ruboxistaurin, verabreicht.

Bei einer weiteren Ausführungsform wird die Verbindung der Formel I in Kombination mit einem Aktivator der AMP-aktivierten Proteinkinase (AMPK), wie sie z. B. in WO2007062568 beschrieben sind, verabreicht.

Bei einer Ausführungsform wird die Verbindung der Formel I in Kombination mit einem Inhibitor der Ceramidkinase, wie sie z. B. in WO2007112914 beschrieben sind, verabreicht.

Bei einer weiteren Ausführungsform wird die Verbindung der Formel I in Kombination mit einem Inhibitor der MAPK-interagierenden Kinase 2 (MNK2), wie sie z.B. in WO2007104053, WO2007115822 beschrieben sind, verabreicht.

Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022057, WO2004022553, WO2005097129, WO2005113544, US2007244140 beschrieben sind, verabreicht.

Bei einer Ausführungsform der Erfindung wird die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin, L-659699 oder solchen, wie sie in US2007249583 beschrieben sind, verabreicht.

Bei einer weiteren Ausführungsform der Erfindung wird die Verbindung der Formel I in Kombination mit einem Farnesoid X Rezeptor (FXR) Antagonisten, wie z.B. in WO2007052843, WO2007070796, WO2007092751, JP2007230909, WO2007095174, WO2007140174, WO2007140183 beschrieben, verabreicht.

Bei einer anderen Ausführungsform der Erfindung wird die Verbindung der Formel I in Kombination mit einem Liganden des Leber X Rezeptors (liver X receptor; LXR), wie z.B. in WO2007092965 beschrieben, verabreicht.

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit Fibraten, wie z.B. dem Cholinsalz von Fenofibrat (SLV-348), verabreicht.

Bei einer Ausführungsform der Erfindung wird die Verbindung der Formel I in Kombination mit Fibraten, wie z.B. dem Cholinsalz von Fenofibrat und einem HMGCoA Reduktase Inhibitor, wie z.B. Rosuvastatin, verabreicht.

Bei einer weiteren Ausführungsform der Erfindung wird die Verbindung der Formel I in Kombination mit Bezafibrat und Diflunisal verabreicht.

Bei einer weiteren Ausführungsform der Erfindung wird die Verbindung der Formel I in Kombination mit einer festen Kombination von Fenofibrat oder einem Salz davon mit Simvastatin, Rosuvastatin, Fluvastatin, Lovastatin, Cerivastatin, Pravastatin oder Atorvastatin verabreicht.

Bei einer weiteren Ausführungsform der Erfindung wird die Verbindung der Formel I in Kombination mit Synordia (R), einer festen Kombination von Fenofibrat mit Metformin, verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692, WO2005005453), MD-0727 (Microbia Inc., WO2005021497, WO2005021495) oder mit Verbindungen, wie in WO2002066464, WO2005000353 (Kotobuki Pharmaceutical Co. Ltd.) oder WO2005044256 oder WO2005062824 (Merck & Co.) oder WO2005061451 und WO2005061452 (AstraZeneca AB) und WO2006017257 (Phenomix) oder WO2005033100 (Lipideon Biotechnology AG) oder wie in WO2002050060, WO2002050068, WO2004000803, WO2004000804, WO2004000805, WO2004087655, WO2004097655, WO2005047248, WO2006086562, WO2006102674, WO2006116499, WO2006121861, WO2006122186, WO2006122216, WO2006127893, WO2006137794, WO2006137796, WO2006137782, WO2006137793, WO2006137797, WO2006137795, WO2006137792, WO2006138163, WO2007059871, US2007232688, WO2007126358 beschrieben, verabreicht.

Bei einer Ausführungsform der Erfindung wird die Verbindung der Formel I in Kombination mit Vytorin™, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Atorvastatin, verabreicht.

Bei einer Ausführungsform der Erfindung wird die Verbindung der Formel I in Kombination mit einer festen Kombination von Ezetimibe mit Fenofibrat verabreicht.

Bei einer Ausführungsform der Erfindung ist der weitere Wirkstoff ein Diphenylazetidinonderivat, wie z.B. in US 6,992,067 oder US 7,205,290 beschrieben.

Bei einer weiteren Ausführungsform der Erfindung ist der weitere Wirkstoff ein Diphenylazetidinonderivat, wie z.B. in US 6,992,067 oder US 7,205,290 beschrieben, kombiniert mit einem Statin, wie z.B. Simvastatin, Fluvastatin, Pravastatin, Lovastatin, Cerivastatin, Atorvastatin oder Rosuvastatin.

Bei einer Ausführungsform der Erfindung wird die Verbindung der Formel I in Kombination mit einer festen Kombination von Lapaquistat, einem Squalensynthase-Inhibitor, mit Atorvastatin verabreicht.

Bei einer Ausführungsform der Erfindung wird die Verbindung der Formel I in Kombination mit einem CETP-Inhibitor, wie z.B. Torcetrapib, Anacetrapib oder JTT-705 oder solchen wie sie in WO2006002342, WO2006010422, WO2006012093, WO2006073973, WO2006072362, WO2007088996, WO2007088999, US2007185058, US2007185113, US2007185154, US2007185182, WO2006097169, WO2007041494, WO2007090752, WO2007107243, WO2007120621, US2007265252, US2007265304, WO2007128568, WO2007132906 beschrieben sind, verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9, WO2007009655-56 beschrieben, verabreicht.

Bei einer Ausführungsform wird die Verbindung der Formel I in Kombination mit Agonisten des GPBAR1 (G-protein-coupled-bile-acid-receptor-1; TGR5), wie sie z.B. in WO2007110237, WO2007127505 beschrieben sind, verabreicht.

Bei einer Ausführungsform der Erfindung wird die Verbindung der Formel I in Kombination mit einem polymeren Gallensäureadsorber, wie z.B. Cholestyramin, Colesevelam Hydrochlorid, verabreicht.

Bei einer Ausführungsform der Erfindung wird die Verbindung der Formel I in Kombination mit einem Phytosterole enthaltenden Kaugummi (Reductol^{™}) verabreicht.

Bei einer Ausführungsform der Erfindung wird die Verbindung der Formel I in Kombination mit einem Inhibitor des mikrosomalen Triglycerid-Transfer-Proteins (MTP-Inhibitor), wie z.B. Implitapide , BMS-201038, R-103757, AS-1552133, SLx-4090, AEGR-733 oder solchen wie in WO2005085226, WO2005121091, WO2006010423, WO2006113910 beschrieben, verabreicht.

Bei einer anderen Ausführungsform der Erfindung wird die Verbindung der Formel I in Kombination mit einem Antagonisten des Somatostatin 5 Rezeptors (SST5 Rezeptor), wie z.B. solchen wie sie in WO2006094682 beschrieben sind, verabreicht.

Bei einer Ausführungsform der Erfindung wird die Verbindung der Formel I in Kombination mit einem ACAT-Inhibitor, wie z.B. Avasimibe, SMP-797 oder KY-382, verabreicht.

Bei einer weiteren Ausführungsform der Erfindung wird die Verbindung der Formel I in Kombination mit einem Inhibitor der Leber-Carnitin Palmitoyltransferase-1 (L-CPT1), wie sie z.B. in WO2007063012, WO2007096251 (ST-3473) beschrieben sind, verabreicht.

Bei einer Ausführungsform der Erfindung wird die Verbindung der Formel I in Kombination mit einem Squalen Synthetase Inhibitor, wie z.B. BMS-188494, TAK-475 (Lapaquistat Acetat) oder wie in WO2005077907, JP2007022943 beschrieben, verabreicht.

Bei einer Ausführungsform der Erfindung wird die Verbindung der Formel I in Kombination mit ISIS-301012 (Mipomersen), einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in WO2005097738 beschrieben, verabreicht.

Bei einer Ausführungsform der Erfindung wird die Verbindung der Formel I in Kombination mit einem ABCA1 Expressionsverstäker, wie sie z.B. in WO2006072393 beschrieben, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit einem Adenosin A2B Rezeptor Agonisten (Adenosin A2B R) wie z.B. ATL-801 verabreicht.

Bei einer anderen Ausführungsform der Erfindung wird die Verbindung der Formel I in Kombination mit einem Modulator der Adenosin A2A und/oder Adenosin A3 Rezeptoren, wie z.B. in WO2007111954, WO2007121918, WO2007121921, WO2007121923 beschrieben, verabreicht.

Bei einer Ausführungsform der Erfindung wird die Verbindung der Formel I in Kombination mit einem Adenosin A2B Rezeptor Antagonisten (Adenosin A2B R), wie sie in US2007270433 beschrieben sind, verabreicht.

Bei einer Ausführungsform wird die Verbindung der Formel I in Kombination mit Hemmstoffen der Acetyl-CoA Carboxylase (ACC1 und/oder ACC2) wie z. B. solchen wie in WO199946262, WO200372197, WO2003072197, WO2005044814, WO2005108370, JP2006131559, WO2007011809, WO2007011811, WO2007013691, WO2007095601-603, WO2007119833 beschrieben, verabreicht.

Bei einer anderen Ausführungsform wird die Verbindung der Formel I in Kombination mit Modulatoren der mikrosomalen Acyl-CoA:Glycerol-3-Phosphat-Acyltransferase 3 (GPAT3, beschrieben in WO2007100789) oder mit Modulatoren der mikrosomalen Acyl-CoA:Glycerol-3-Phosphat-Acyltransferase 4 (GPAT4, beschrieben in WO2007100833) verabreicht.

Bei einer weiteren Ausführungsform wird die Verbindung der Formel I in Kombination mit Modulatoren der Xanthin-Oxidoreductase (XOR) verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
NPY-5 Rezeptorantagonisten wie L-152804 oder die Verbindung "NPY-5-BY" der Firma Banyu oder wie sie z. B. in WO2006001318, WO2007103295, WO2007125952 beschrieben sind;
NPY-4-Rezeptorantagonisten wie sie z. B. in WO2007038942 beschrieben sind;
NPY-2-Rezeptorantagonisten wie sie z. B. in WO2007038943 beschrieben sind;
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424, WO2006095166 beschrieben sind;
Derivaten des Peptids Obestatin wie sie WO2006096847 beschrieben sind;
CB1R (Cannabinoid Rezeptor 1) Antagonisten (wie z.B. Rimonabant, Surinabant (SR147778), SLV-319, AVE-1625, Taranabant (MK-0364) oder Salze davon, V-24343 oder solche Verbindungen wie sie in z. B. EP 0656354, WO 00/15609, WO2001/64632-64634, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006047516, WO2006060461, WO2006067428, WO2006067443, WO2006087480, WO2006087476, WO2006100208, WO2006106054, WO2006111849, WO2006113704, WO2007009705, WO2007017124, WO2007017126, WO2007018459, WO2007018460, WO2007016460, WO2007020502, WO2007026215, WO2007028849, WO2007031720, WO2007031721, WO2007036945, WO2007038045, WO2007039740, US20070015810, WO2007046548, WO2007047737, WO2007057687, WO2007062193, WO2007064272, WO2007079681, WO2007084319, WO2007084450, WO2007086080, EP1816125, US2007213302, WO2007095513, WO2007096764, US2007254863, WO2007119001, WO2007120454, WO2007121687, WO2007123949, US2007259934, WO2007131219, WO2007133820, WO2007136607, WO2007136571, US7297710, WO2007138050, WO2007140385, WO2007140439 beschrieben sind);
Cannabinoid Rezeptor 1 / Cannabinoid Rezeptor 2 (CB1/CB2) modulierende Verbindungen wie z.B. delta-9-Tetrahydrocannabivarin oder solchen wie sie z.B. in WO2007001939, WO2007044215, WO2007047737, WO2007095513, WO2007096764, WO2007112399, WO2007112402 beschrieben sind;
Modulatoren der FAAH (fatty acid amide hydrolase) wie sie z.B. in WO2007140005 beschrieben sind;
Vanilloid-1-Rezeptor Modulatoren (Modulatoren des TRPV1), wie sie z.B. in WO2007091948, WO2007129188, WO2007133637 beschrieben sind;
Aktivatoren des Capsaicinrezeptors, wie sie z.B. in JP2007210969 beschrieben sind;
Agonisten des Prostaglandinrezeptors, wie z.B. Bimatoprost oder solchen Verbindungen wie sie in WO2007111806 beschrieben sind;
MC4-Rezeptor Agonisten (Melanocortin-4 Rezeptor Agonisten, MC4R Agonisten wie z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chlor-phenyl)-2-oxo-ethyl]-amid; (WO 01/91752)) oder LB53280, LB53279, LB53278 oder THIQ, MB243, RY764, CHIR-785, PT-141, MK-0493 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, WO2005118573, EP1538159, WO2004072076, WO2004072077, WO2006021655-57, WO2007009894, WO2007015162, WO2007041061, WO2007041052, JP2007131570, EP-1842846, WO2007096186, WO2007096763 beschrieben sind;
Orexin-Rezeptor 1 Antagonisten (OX1R Antagonisten), Orexin-Rezeptor 2 Antagonisten (OX2R Antagonisten) oder gemischte OX1R/OX2R Antagonisten (z.B. 1-(2-Methylbenzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224, WO2007085718, WO2007088276, WO2007116374;WO2007122591, WO2007126934, WO2007126935 beschrieben sind);
Histamin H3 Rezeptor Antagonisten/inverse Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893, US2005171181 (z.B. PF-00389027), WO2006107661, WO2007003804, WO2007016496, WO2007020213, WO2007049798, WO2007055418, WO2007057329, WO2007065820, WO2007068620, WO2007068641, WO2007075629, WO2007080140, WO2007082840, WO2007088450, WO2007088462, WO2007094962, WO2007099423, WO2007100990, WO2007105053, WO2007106349, WO2007110364, WO2007115938, WO2007131907, WO2007133561, US2007270440, WO2007135111 beschrieben sind);
Histamin H1 / Histamin H3 Modulatoren, wie z. B. Betahistin bzw. seinem Dihydrochlorid;
CRF-Antagonisten (z.B. [2-Methyl-9-(2,4,6-trimethyl-phenyl)-9H-1,3,9-triaza-fluoren-4-yl]-dipropyl-amin (WO 00/66585) oder solche CF1-Antagonisten, wie sie in WO2007105113, WO2007133756 beschrieben sind);
CRF BP-Antagonisten (z.B. Urocortin);
Urocortin-Agonisten;
Agonisten des beta-3 Adrenoceptors wie z.B. 1-(4-Chlor-3-methanesulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553, WO2002038543, WO2002038544, WO2007048840-843 beschrieben sind;
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2005085200, WO2005019240, WO2004011438, WO2004012648, WO2003015769, WO2004072025, WO2005070898, WO2005070925, WO2004039780, WO2004092181, WO2003033476, WO2002006245, WO2002089729, WO2002002744, WO2003004027, FR2868780, WO2006010446, WO2006038680, WO2006044293, WO2006044174, JP2006176443, WO2006018280, WO2006018279, WO2006118320, WO2006130075, WO2007018248, WO2007012661, WO2007029847, WO2007024004, WO2007039462, WO2007042660, WO2007042668, WO2007042669, US2007093508, US2007093509, WO2007048802, JP2007091649, WO2007092416; WO2007093363-366, WO2007114902, WO2007114916 beschrieben sind);
CCK-A (CCK-1) Agonisten (wie z.B. {2-[4-(4-Chlor-2,5-dimethoxy-phenyl)-5-(2-cyclohexylethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180) oder solchen, wie sie in WO2005116034, WO2007120655, WO2007120688, WO2007120718 beschrieben sind;
Serotonin-Wiederaufnahme-Inhibitoren (z.B. Dexfenfluramine);
gemischte Serotonin-/Dopamin-Wiederaufnahme-Inhibitoren (z.B. Bupropion) oder feste Kombinationen von Bupropion mit Naltrexon oder Bupropion mit Zonisamid;
gemischte Wiederaufnahmeinhibitoren wie z.B. DOV-21947;
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
gemischte Dopamin/Norepinephrin/Acetylcholin-Wiederaufnahme-Inhibitoren (z.B. Tesofensine) oder solchen wie sie z.B. in WO2006085118 beschrieben sind;
5-HT2C Rezeptor Agonisten (wie z.B. Lorcaserin Hydrochlorid (APD-356) oder BVT-933 oder solche, wie sie in WO200077010, WO200077001-02, WO2005019180, WO2003064423, WO200242304, WO2005035533, WO2005082859, WO2006004937, US2006025601, WO2006028961, WO2006077025, WO2006103511, WO2007028132, WO2007084622, US2007249709; WO2007132841, WO2007140213 beschrieben sind);
5-HT6 Rezeptor Modulatoren, wie z.B. E-6837, BVT-74316 oder PRX-07034 oder solche wie sie z.B. in WO2005058858, WO2007054257, WO2007107373, WO2007108569, WO2007108742-744 beschrieben sind;
Agonisten des Estrogenrezeptors gamma (ERR Agonisten), wie sie z.B. in WO2007131005 beschrieben sind;
Sigma-1 Rezeptorantagonisten, wie sie z.B. in WO2007098953, WO2007098961 beschrieben sind;
Muscarin 3 Rezeptor (M3R) Antagonisten, wie sie z.B. in WO2007110782 beschrieben sind;
Bombesin-Rezeptor Agonisten (BRS-3 Agonisten);
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695));
Growth Hormone Secretagogue Receptor Antagonisten (Ghrelin Antagonisten) wie z. B. A-778193 oder solchen, wie sie in WO2005030734, WO2007127457 beschrieben sind;
Growth Hormone Secretagogue Receptor Modulatoren wie z.B. JMV-2959, JMV-3002, JMV-2810, JMV-2951 oder solchen, wie sie in WO2006012577 (z.B. YIL-781 oder YIL-870), WO2007079239 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
Dopaminagonisten (DA-Agonisten, z.B. Bromocriptin, Doprexin);
Lipase/Amylase-Inhibitoren (z.B. WO 00/40569);
Inhibitoren der Diacylglycerol O-Acyltransferasen (DGATs) wie z. B. BAY-74-4113 oder wie z. B. in US2004/0224997, WO2004094618, WO200058491, WO2005044250, WO2005072740, JP2005206492, WO2005013907, WO2006004200, WO2006019020, WO2006064189, WO2006082952, WO2006120125, WO2006113919, WO2006134317, WO2007016538, WO2007060140, JP2007131584, WO2007071966, WO2007126957, WO2007137103, WO2007137107, WO2007138304, WO2007138311 beschrieben;
Inhibitoren der Fettsäuresynthase (FAS) wie z.B. C75 oder solchen, wie in WO2004005277 beschrieben;
Inhibitoren der Stearoyl-CoA delta9 Desaturase (SCD1) wie sie z.B. in WO2007009236, WO2007044085, WO2007046867, WO2007046868, WO20070501124, WO2007056846, WO2007071023, WO2007130075, WO2007134457, WO2007136746 beschrieben sind;
Inhibitoren des "Adipocyte fatty acid-binding protein aP2" wie z.B. BMS-309403; Aktivatoren der Adiponectinsekretion, wie z.B. in WO2006082978 beschrieben;
Promotoren der Adiponectinproduktion, wie z.B. in WO2007125946 beschrieben;
Oxyntomodulin;
Oleoyl-Estron
oder Agonisten oder partiellen Agonisten des Schilddrüsenhormonrezeptors (thyroid hormone receptor agonists) wie z. B: KB-2115 oder DITPA oder solche, wie in WO20058279, WO200172692, WO200194293, WO2003084915, WO2004018421, WO2005092316, WO2007003419, WO2007009913, WO2007039125, WO2007110225, WO2007110226, WO2007128492, WO2007132475, WO2007134864 beschrieben
oder Agonisten des Schilddrüsenhormonrezeptors beta (TR-beta) wie z. B. MB-07811 oder MB-07344, verabreicht.

Bei einer Ausführungsform der Erfindung wird die Verbindung der Formel I in Kombination mit einem Inhibitor der Site-1 Protease (S1P), wie z.B. PF-429242, verabreicht.

Bei einer weiteren Ausführungsform der Erfindung wird die Verbindung der Formel I in Kombination mit einem RNAi Therapeutikum, welches gegen PCSK9 (Proprotein Convertase Subtilisin/Kexin Typ 9) gerichtet ist, verabreicht.

Bei einer Ausführungsform wird die Verbindung der Formel I in Kombination mit Omacor® oder Lovaza™ (Omega-3-Fettsäureester; hochkonzentrierte Ethylester der Eicosapentaensäure und der Docosahexaensäure) verabreicht.

Bei einer Ausführungsform wird die Verbindung der Formel I in Kombination mit Lycopin verabreicht.

Bei einer Ausführungsform der Erfindung wird die Verbindung der Formel I in Kombination mit einem Antioxidans, wie z.B. OPC-14117, Succinobucol, Probucol, Tocopherol, Ascorbinsäure, ß-Caroten oder Selen verabreicht.

Bei einer Ausführungsform der Erfindung wird die Verbindung der Formel I in Kombination mit einem Vitamin, wie z. B. Vitamin B6 oder Vitamin B 12 verabreicht.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer anderen Ausführungsform wird die Verbindung der Formel I in Kombination mit einem Inhibitor der Carboanhydrase Typ 2 (Carbonic anhydrase type 2), wie z.B. solchen, wie in WO2007065948 beschrieben, verabreicht.

Bei einer anderen Ausführungsform wird die Verbindung der Formel I in Kombination mit Topiramat verabreicht.

Bei einer weiteren Ausführungsform wird die Verbindung der Formel I in Kombination mit einer festen Kombination von Topiramat mit Phentermin (Qnexa^{™}) verabreicht.

Bei einer weiteren Ausführungsform wird die Verbindung der Formel I in Kombination mit einer Antisense-Verbindung, z.B. ISIS-377131, verabreicht, welche die Produktion des Glukokortikoidrezeptors inhibiert.

Bei einer Ausführungsform wird die Verbindung der Formel I in Kombination mit einem Agonisten des RUP3 Rezeptors, wie z. B. in WO2007035355 beschrieben, verabreicht.

Bei einer anderen Ausführungsform wird die Verbindung der Formel I in Kombination mit einem Aktivator des Gens, welches für die Ataxia Telangiectasia Mutated (ATM) Proteinkinase kodiert, wie z. B. Chloroquin, verabreicht.

Bei einer Ausführungsform wird die Verbindung der Formel I in Kombination mit einem Tau-Protein-Kinase-1-Inhibitor (TPK1 Inhibitor), wie z. B. in WO2007119463 beschrieben, verabreicht.

Bei einer Ausführungsform wird die Verbindung der Formel I in Kombination mit einem "c-Jun N-terminal kinase" Inhibitor (JNK-Inhibitor), wie z. B. in WO2007125405 beschrieben, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Modulatoren des Glukokortikoidrezeptors (GR), wie z.B. KB-3305 oder solchen Verbindungen wie sie z. B. in WO2005090336, WO2006071609, WO2006135826, WO2007105766 beschrieben sind, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIRT1 (einer NAD⁺-abhängigen Proteindeacetylase); dieser Wirkstoff kann z.B. Resveratrol in geeigneten Formulierungen sein, oder solche Verbindungen wie sie in WO2007019416 (z.B. SRT-1720) genannt sind.

Bei einer Ausführungsform der Erfindung ist der weitere Wirkstoff DM-71 (N-Acetyl-L-Cystein mit Bethanechol).

Bei einer Ausführungsform wird die Verbindung der Formel I in Kombination mit antihypercholesterolemisch wirkenden Verbindungen, wie sie z.B. in WO2007107587, WO2007111994 beschrieben sind, verabreicht.

Bei einer anderen Ausführungsform wird die Verbindung der Formel I in Kombination mit einem cyclischen Peptidagonisten des VPAC2 Rezeptors, wie sie z.B. in WO2007101146, WO2007133828 beschrieben sind, verabreicht.

Bei einer weiteren Ausführungsform wird die Verbindung der Formel I in Kombination mit einem Agonisten des Endothelinrezeptors, wie sie z.B. in WO2007112069 beschrieben sind, verabreicht.

Bei einer weiteren Ausführungsform wird die Verbindung der Formel I in Kombination mit AKP-020 (Bis(ethylmaltolato)oxovanadium-IV) verabreicht.

Bei einer anderen Ausführungsform wird die Verbindung der Formel I in Kombination mit gewebe-selektiven Androgenrezeptor Modulatoren ("tissue-selective androgen receptor modulators"; SARM), wie sie z.B. in WO2007099200 beschrieben sind, verabreicht.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer anderen Ausführungsform der Erfindung ist der weitere Wirkstoff Metreleptin (rekombinantes Methionyl-Leptin) kombiniert mit Pramlintide.

Bei einer weiteren Ausführungsform der Erfindung ist der weitere Wirkstoff das Tetrapeptid ISF-402.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer weiteren Ausführungsform ist der weitere Wirkstoff Geniposidinsäure (geniposidic acid; WO2007100104).

Bei einer Ausführungsform wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

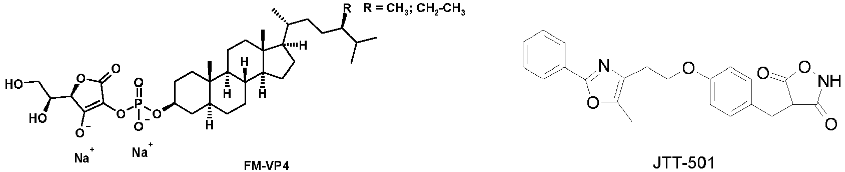

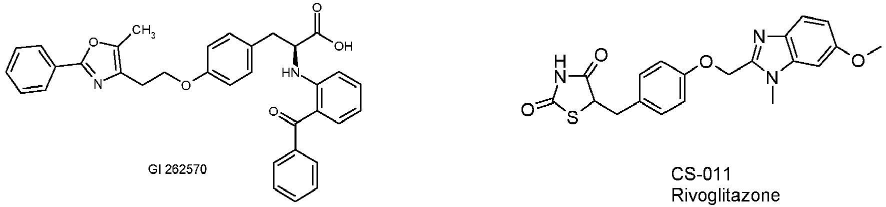

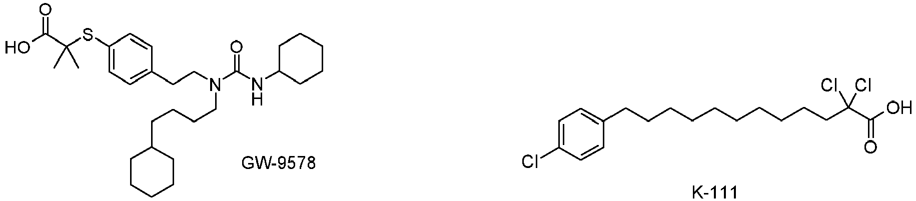

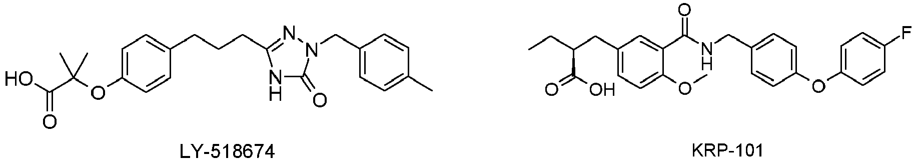

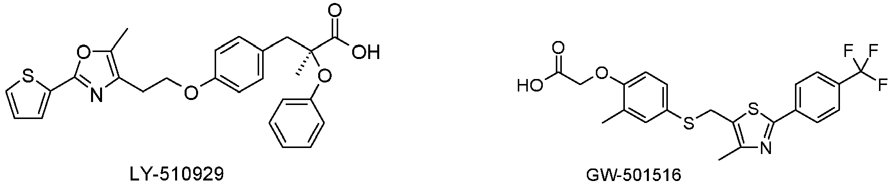

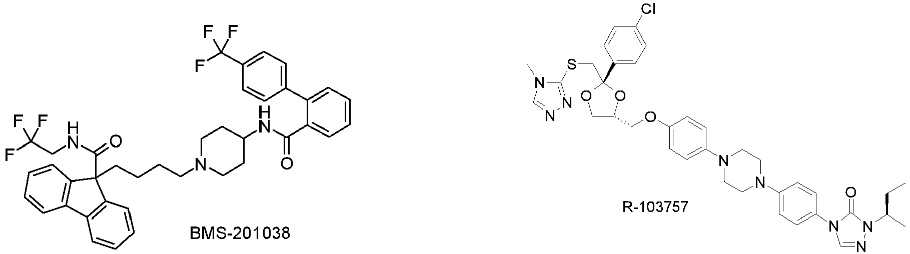

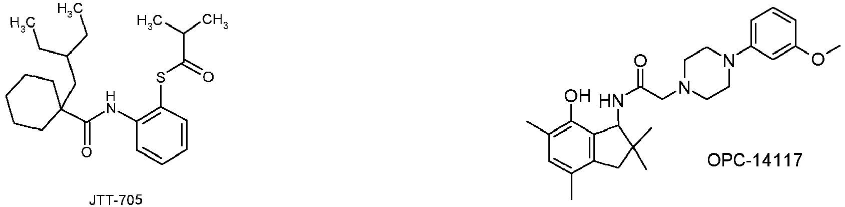

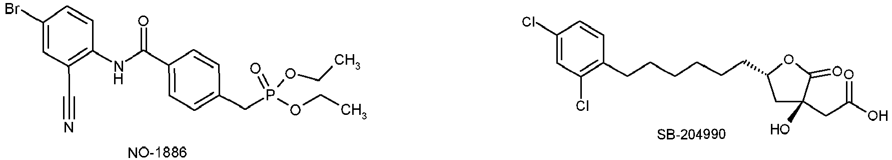

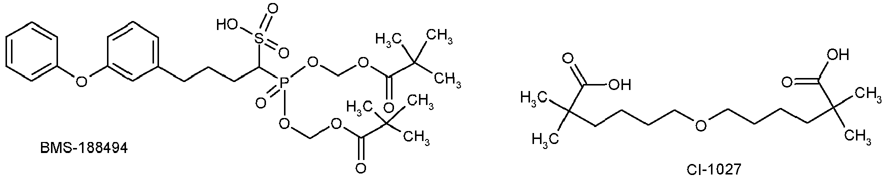

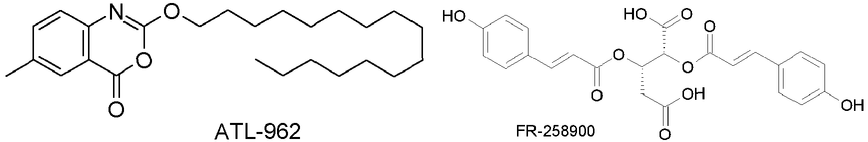

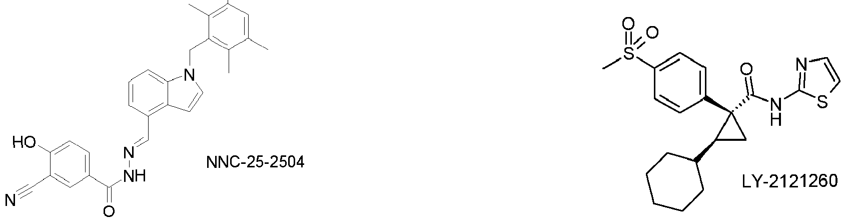

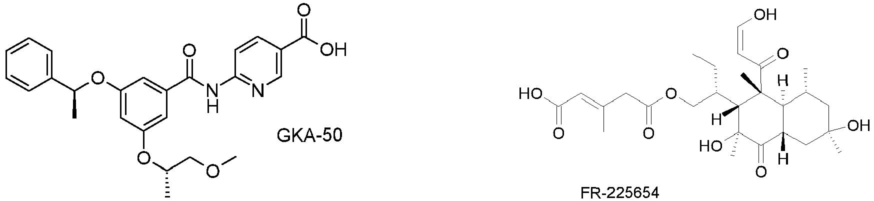

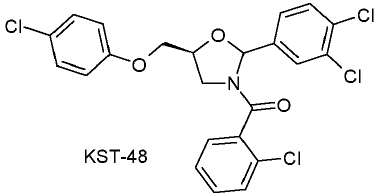

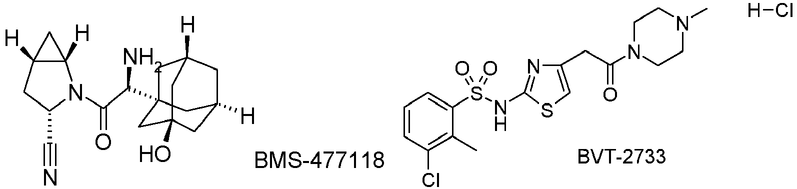

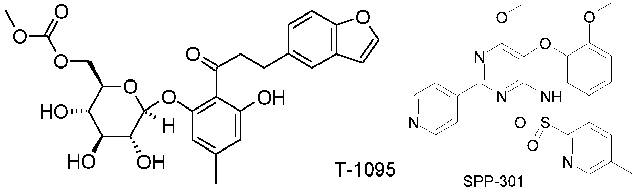

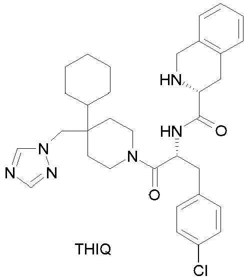

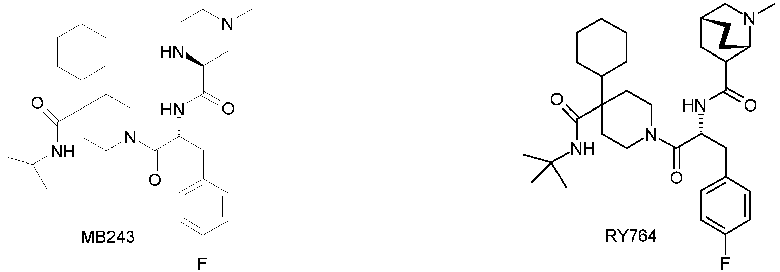

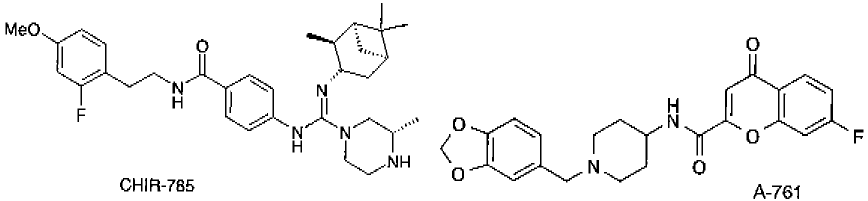

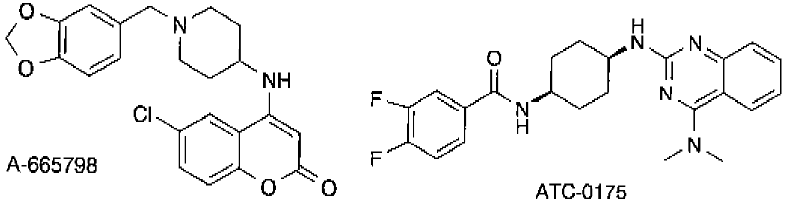

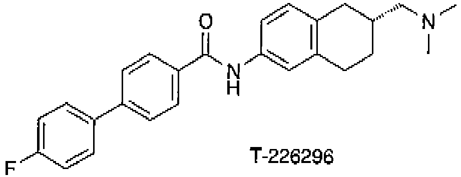

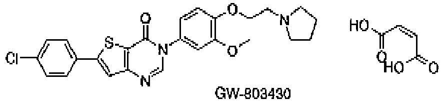

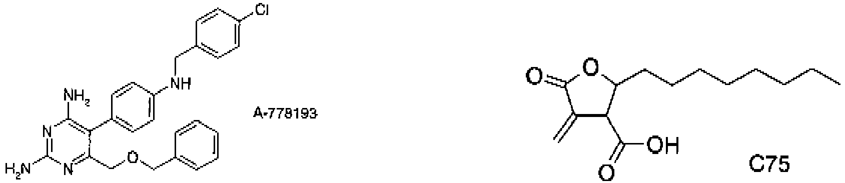

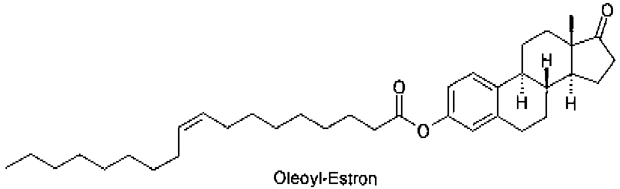

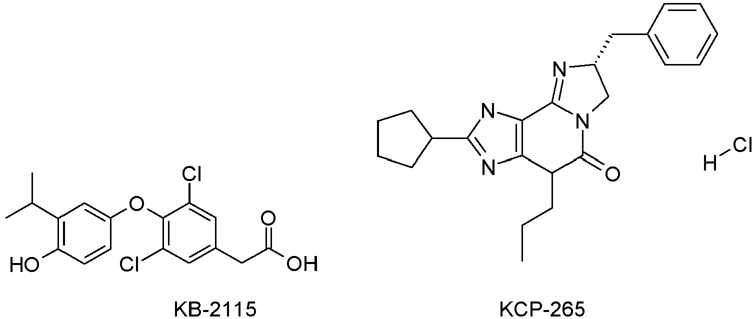

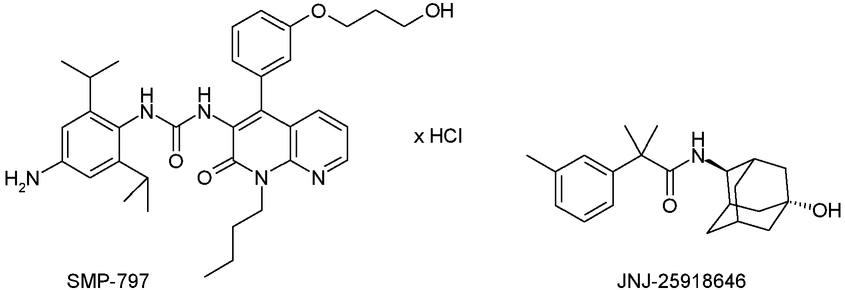

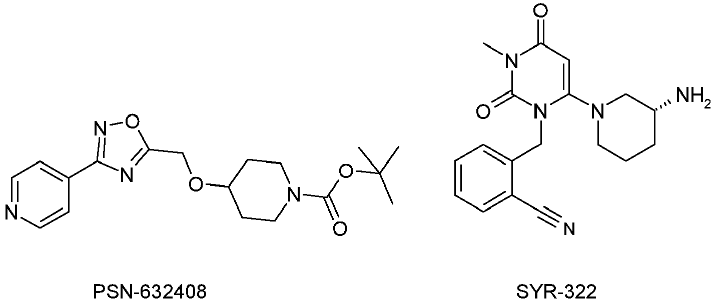

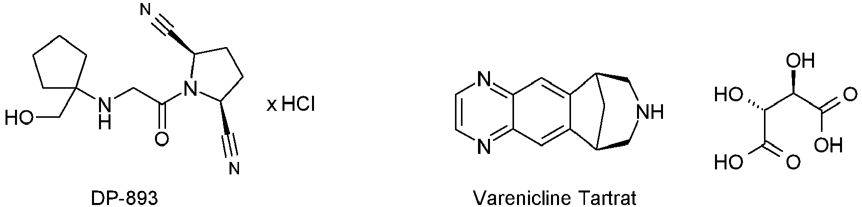

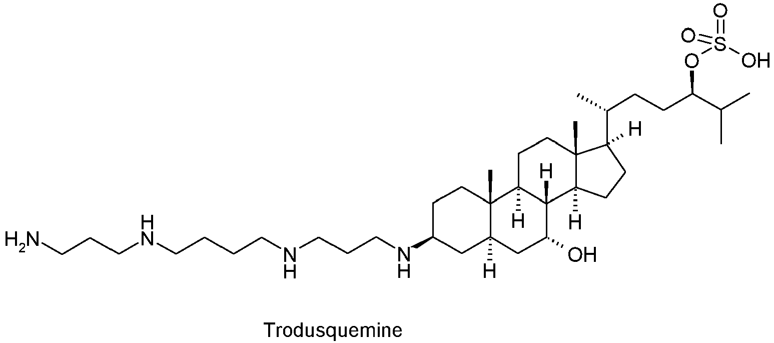

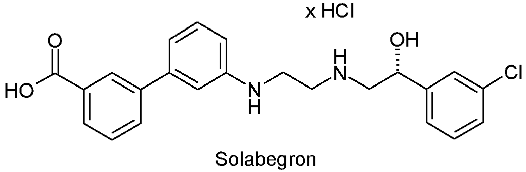

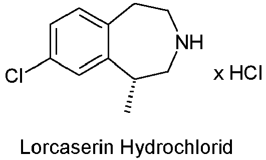

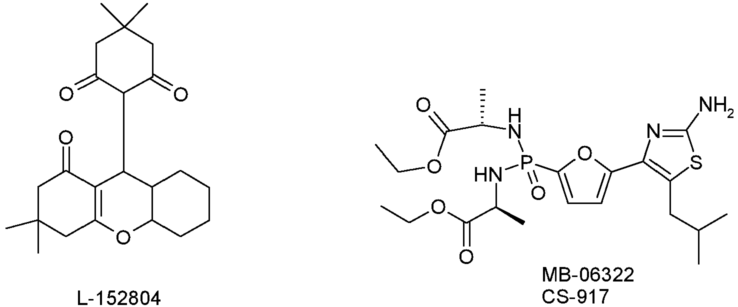

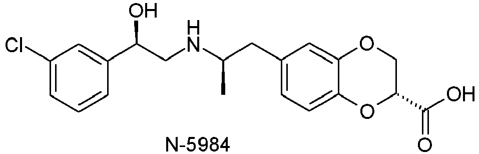

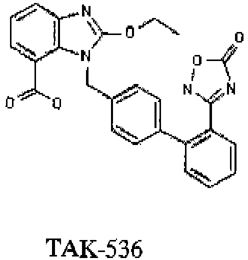

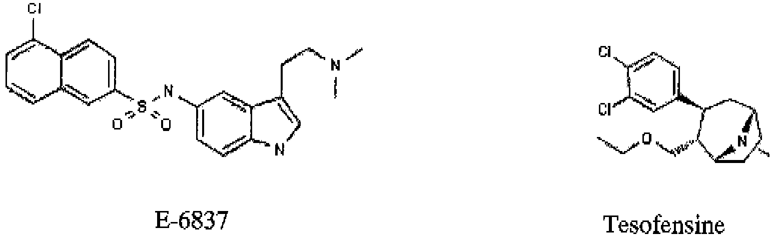

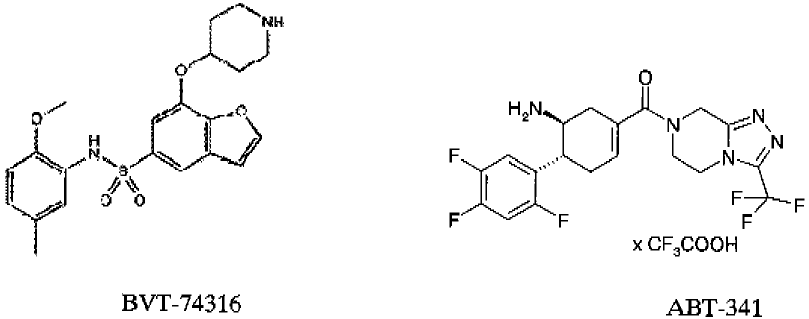

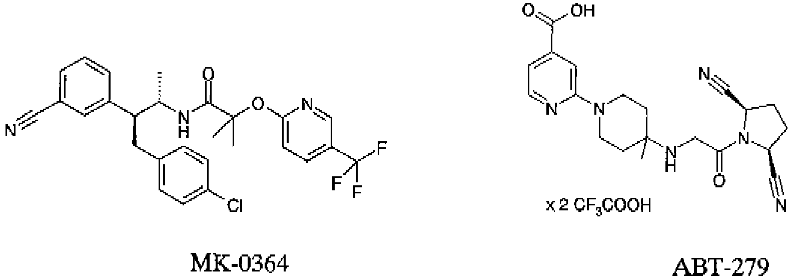

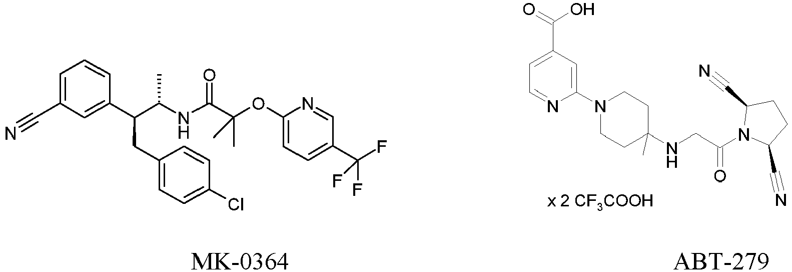

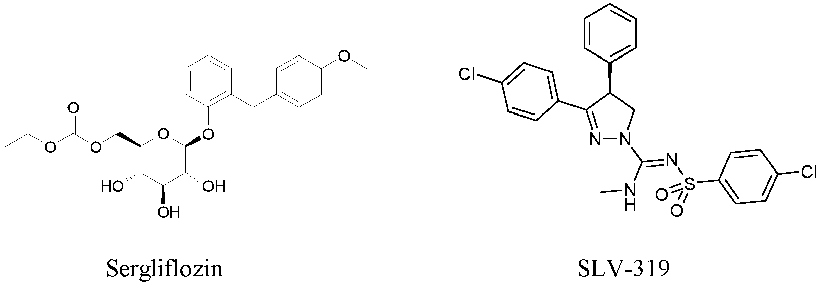

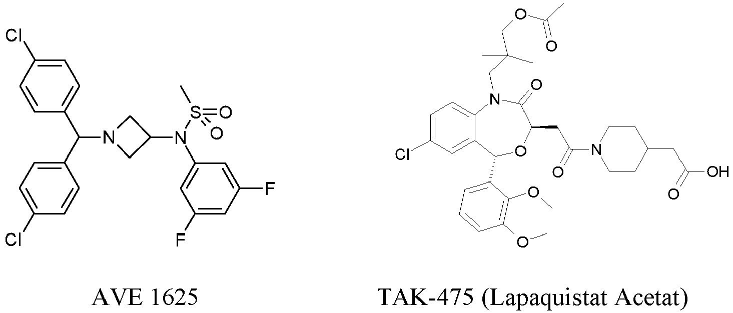

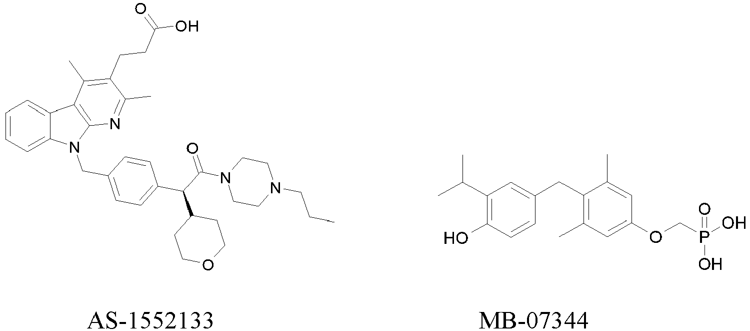

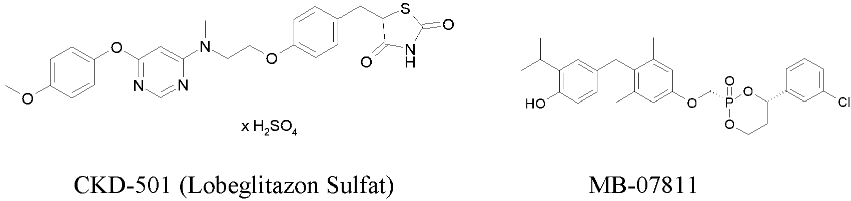

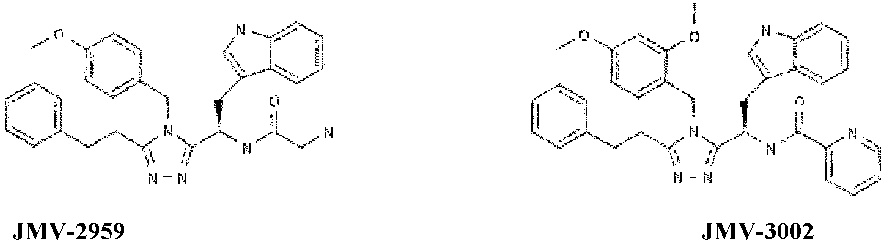

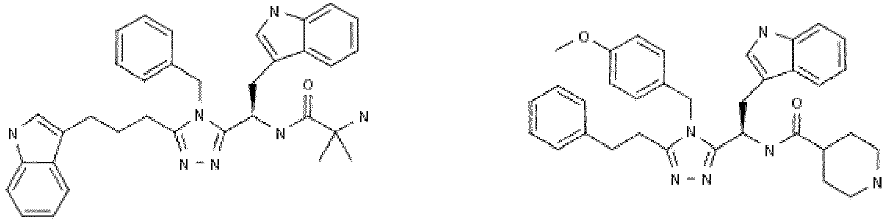

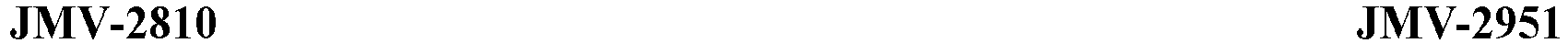

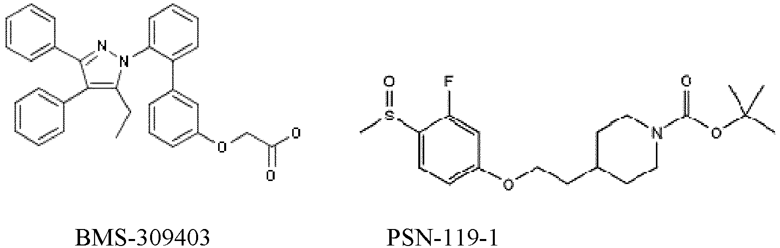

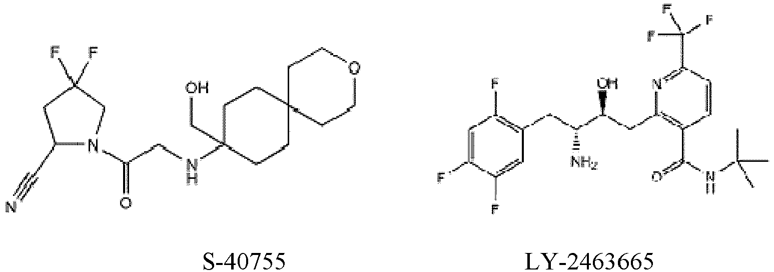

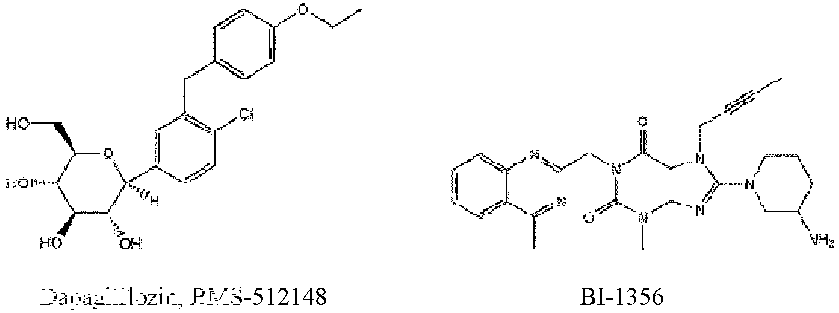

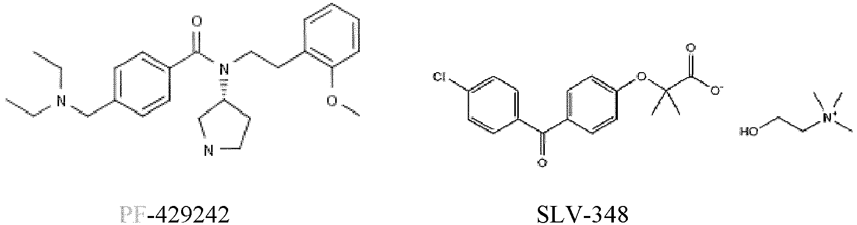

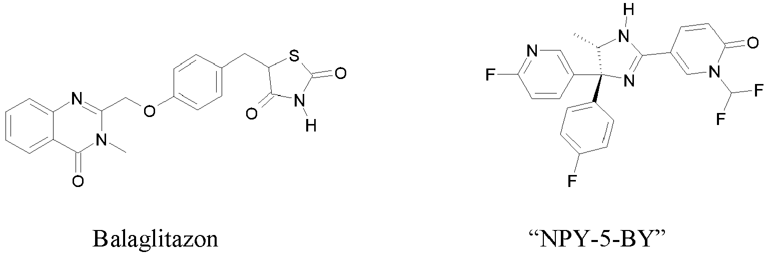

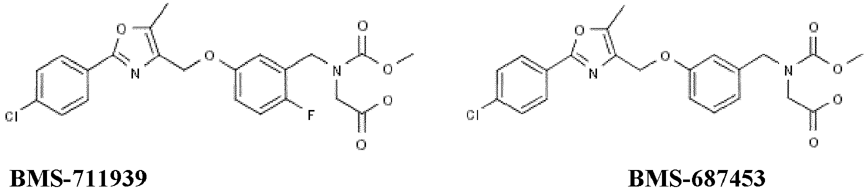

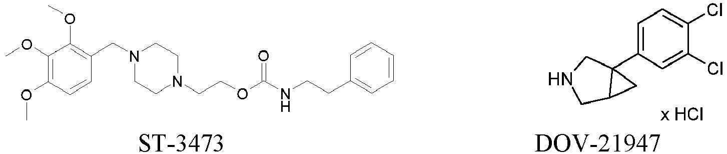

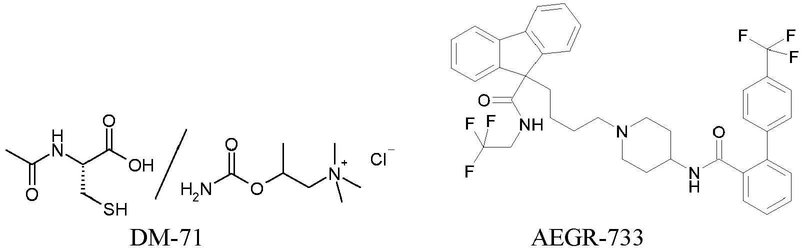

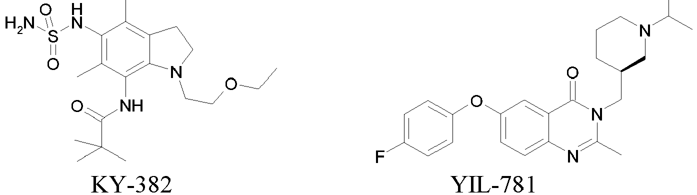

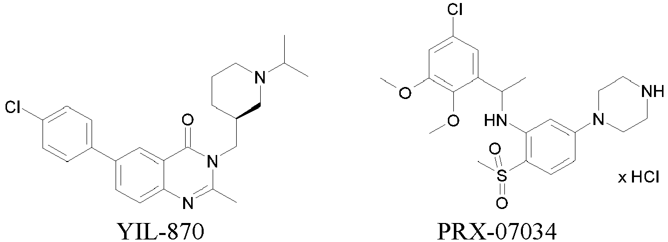

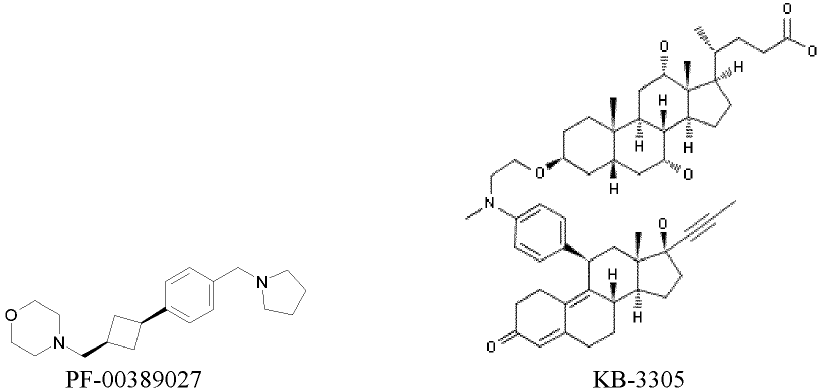

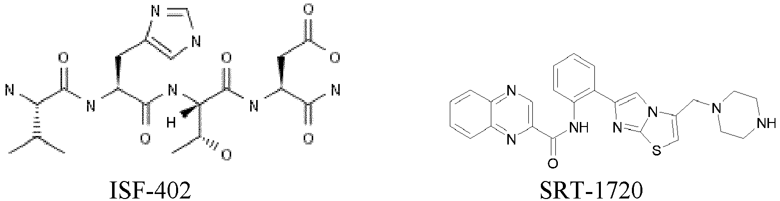

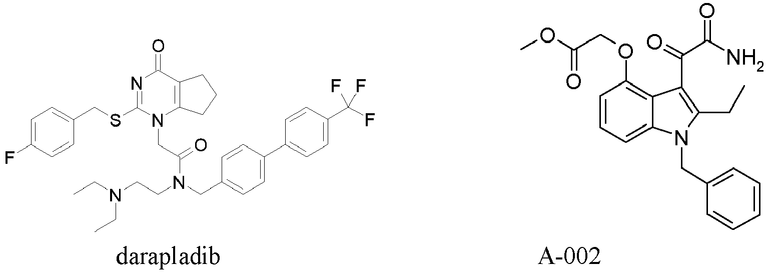

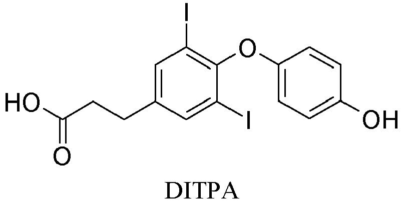

### Beispiele

Die nachfolgend aufgeführten Beispiele und Herstellungsmethoden dienen zur Erläuterung der Erfindung, ohne diese jedoch einzuschränken.

Die erfindungsgemäßen Verbindungen der Formel I können mit Hilfe von im Prinzip bekannten Reaktionen hergestellt werden. Beispielsweise wurden die Verbindungen nach folgenden allgemeinen Reaktionsschemata erhalten.

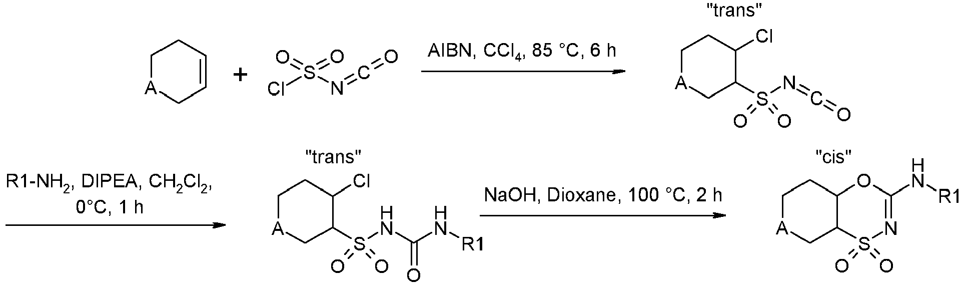

Aus einem cyclischen Alken kann ein entsprechendes Chlorsulfonylisocyanat in einer radikalischen Reaktion hergestellt werden. Dabei wird ausschließlich die *cis*-Konfiguration erhalten. Anschließend wird mit primären Aminen zu Chlorsulfonylharnstoffen umgesetzt. Die entsprechenden Natriumsalze zyklisieren in der Wärme zu *cis*-4,4-Dioxo-oxathiazinen.

In den Fällen, in denen während der Synthese Diastereomere oder Racemate entstehen, können diese durch präparative HPLC voneinander getrennt wreden.

Andere erfindungsgemäße Verbindungen können auf weiteren Wegen erhalten werden, die im folgenden Schema beispielhaft skizziert sind.

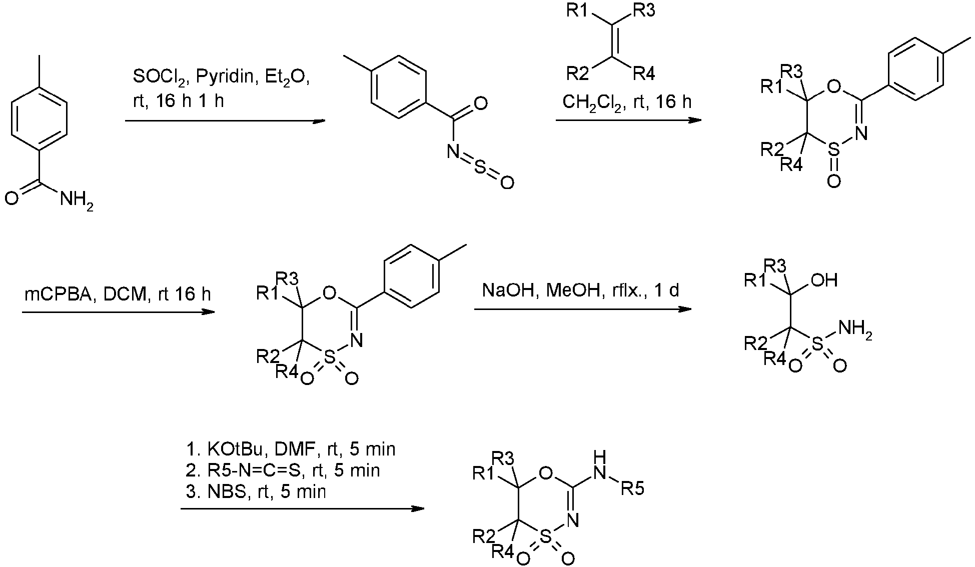

4-Tolylamid wird mit Thionylchlorid und Pyridin zum N-Sulfinyl-4-tolylamid umgesetzt. Dieses reagiert in einer Hetero-Diels-Alder Reaktion mit einem Alken zum 2-Tolyl-4-oxathiazin. Bei cyclischen Alkenen wird dabei ausschließlich die *cis*-Konfiguration erhalten. Nach Oxidation zum 2-Tolyl-4,4-dioxathiazin mit 3-Chlorperbenzoesäure wird mit Natronlauge zum 1,2-Dimethyl-2-hydroxycyclohexylsulfonamid hydrolysiert. Die weitere Behandlung mit Base und einem Isothiocyanat sowie nachfolgendem oxidativen Ringschluss mit NBS liefert die gewünschten 4,4-Dioxo-oxathiazine.

In den Fällen, in denen während der Synthese Diastereomere oder Racemate entstehen, können diese durch präparative HPLC voneinander getrennt wreden.

Wieder andere Beispiele wurden erhalten wie im folgenden Schema angedeutet.

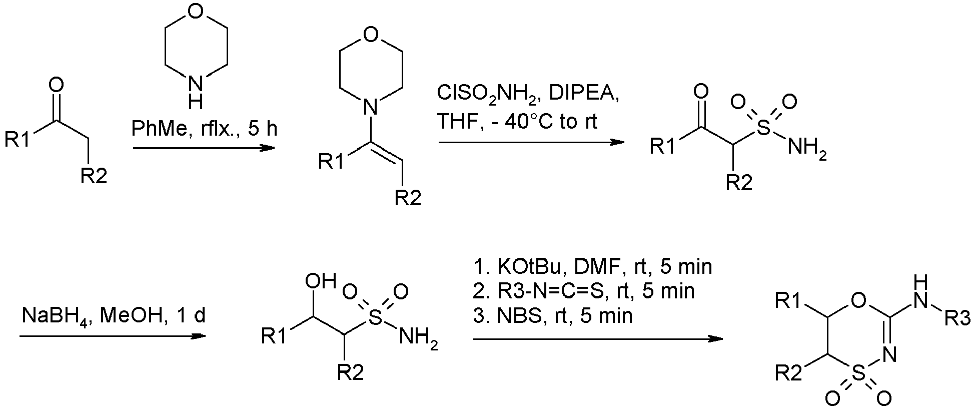

Ein Keton wird mit Morpholin zum Enamin umgesetzt. Dieses wird dann mit Sulfamoylchlorid behandelt. Das entstehende Ketosulfonamid kann mit einem geeigneten Reduktionsmittel, z.B. Natriumborhydrid, zum Hydroxysulfonamid reduziert werden. Dabei kommt es zur Bildung von Diastereomeren und im Falle von cyclischen Ketonen zu cis/trans Isomeren, die voneinander getrennt werden können. Das Verhältnis der Diastereomeren bzw. cis/trans Isomeren ist abhängig von der Natur der Reste R1 und R2 sowie von der Wahl des Reduktionsmittels. Die Behandlung des Hydroxysulfonamids mit Base und einem Isothiocyanat sowie nachfolgendem oxidativen Ringschluss mit NBS liefert die gewünschten 4,4-dioxo-oxathiazine.

In den Fällen, in denen während der Synthese Diastereomere oder Racemate entstehen, können diese durch präparative HPLC voneinander getrennt wreden.

Wieder andere Beispiele wurden erhalten wie im folgenden Schema angedeutet.

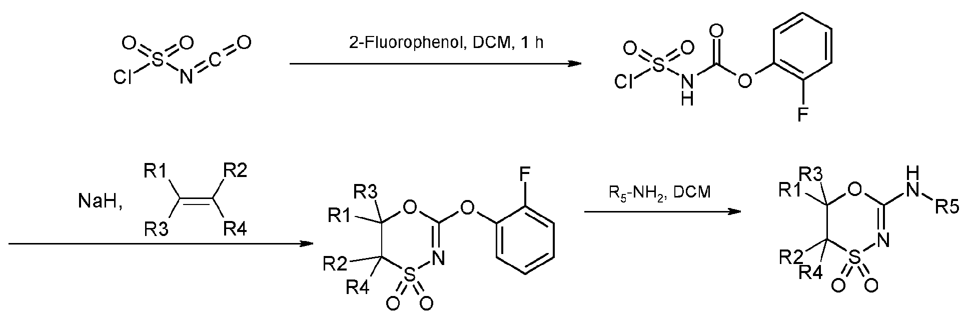

Chlorsulfonylisocyanat wird zunächst mit 2-Fluorphenol zum entsprechenden Chlorsulfonylcarbamat umgesetzt. Dieses reagiert in einer 2+4-Cycloaddition mit einem Alken zum entsprechenden 2-(2-Fluorphenoxy)-4,4-dioxothiazin. Bei cyclischen Alkenen wird dabei ausschließlich die *cis*-Konfiguration erhalten. Die Reaktion mit Aminen liefert die gewünschten 4,4-Dioxo-oxathiazine.

In den Fällen, in denen während der Synthese Diastereomere oder Racemate entstehen, können diese durch präparative HPLC voneinander getrennt werden.

Ab hier überarbeiten und Beispiele mit >C₈ löschen.

| Beispiel | CHEMISTRY | Methode | Retentionszeit | Molgewicht (g/mol) | Name |
|---|---|---|---|---|---|
| 1 | 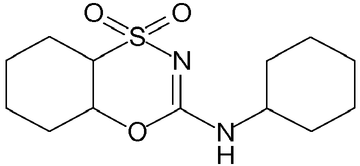 | A | 1,244 | 286,4 | Cyclohexyl-(1,1-dioxo-4a,5,6,7,8,8a-hexahydro-1H-1lambda6-benzo[1,4,3]oxathiazin-3-yl)-amin |
| 2 | 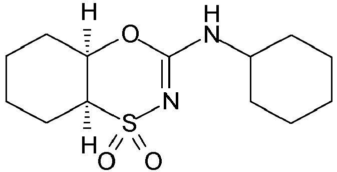 | C | 4,648 | 286,4 | (-)-Cyclohexyl-((4aR,8aS)-1,1-dioxo-4a,5,6,7,8,8a-hexahydro-1H-1lambda6-benzo[1,4,3]oxathiazin-3-yl)-amin * |
| 3 | 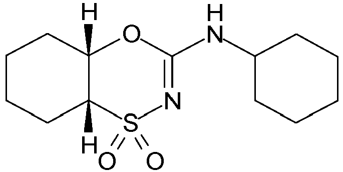 | C | 6,145 | 286,4 | (+)-Cyclohexyl-((4aS,8aR)-1,1-dioxo-4a,5,6,7,8,8a-hexahydro-1H-1lambda6-benzo[1,4,3]oxathiazin-3-yl)-amin * |
| 5 | 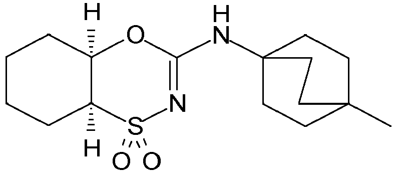 | B | 9.494 | 326.5 | ((4aR,8aS)-1,1-Dioxo-4a,5,6,7,8,8a-hexahydro-1H-1lambda6-benzo[1,4,3]oxathiazin-3-yl)-(4-methyl-bicyclo[2.2.2]oct-1-yl)-amin * |
| 7 | 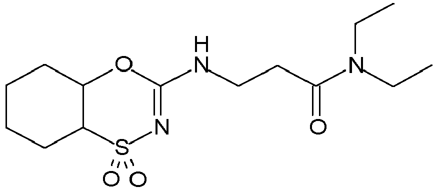 | D | 9.283 | 331.4 | 3-((4aS,8aR)-1,1-Dioxo-4a,5,6,7,8, 8a-hexahydro-1H-1lambda6-benzo[1,4,3]oxathiazin-3-ylamino)-N, N-diethyl propionamid * |
| 8 | 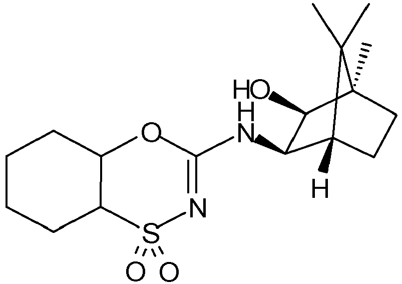 | B | 6.973 | 356.5 | (-)-(1R,2S,3R,4S)-3-(-1,1-Dioxo-4a,5,6,7,8,8a hexahydro-1H-1lambda6-benzo[1,4,3]oxathiazin-3-ylamino)-1,7,7-trimethyl-bicyclo[2.2.1]heptan-2-ol * |
| 9 | 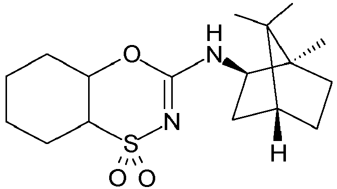 | C | 5.81 | 340.5 | (-1,1-Dioxo-4a,5,6,7,8,8a-hexahydro-1 H-1lambda6-benzo[1,4,3]oxathiazin-3-yl)-((1R,2R,4R)-1,7,7-trimethyl-bicyclo[2.2.1]hept-2-yl)-amin * |
| 10 | 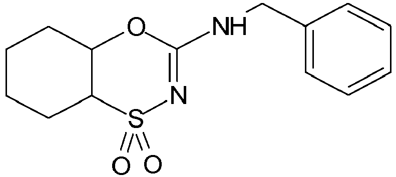 | C | 6.152 | 294.4 | (-)-Benzyl-(-1,1-dioxo-4a,5,6,7,8,8a-hexahydro-1H-1lambda6-benzo[1,4,3]oxathiazin-3-yl)-amin * |
| 11 | 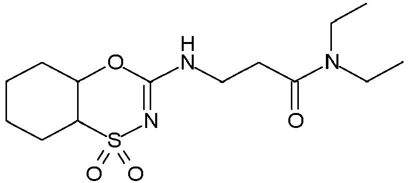 | D | 7.222 | 331.4 | 3-(-1,1-Dioxo-4a,5,6,7,8,8a-hexahydro-1 H-1lambda6-benzo[1,4,3]oxathiazin-3-ylamino)-N,N-diethyl-propionamid * |
| 13 | 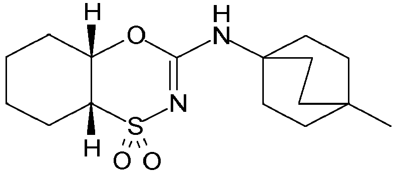 | B | 6.461 | 326.5 | ((4aS,8aR)-1,1-Dioxo-4a,5,6,7,8,8a-hexahydro-1H-1lambda6-benzo[1,4,3]oxathiazin-3-yl)-(4-methyl-bicyclo[2.2.2]oct-1-yl)-amin * |
| 14 | 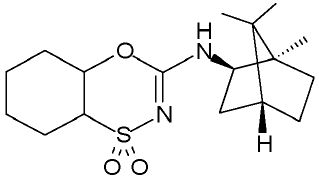 | C | 4.337 | 340.5 | (-1,1-Dioxo-4a,5,6,7,8,8a-hexahydro-1H-1lambda6-benzo[1,4,3]oxathiazin-3-yl)-((1R,2R,4R)-1,7,7-trimethyl-bicyclo[2.2.1]hept-2-yl)-amin * |
| 15 | 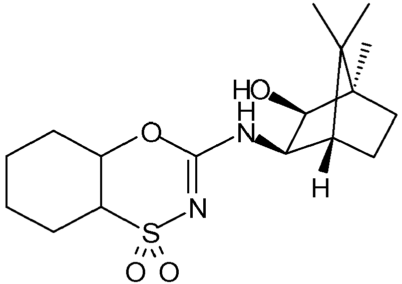 | B | 6.256 | 356.5 | (-)-(1R,2S,3R,4S)-3-(-1,1-Dioxo-4a,5,6,7,8,8a hexahydro-1H-1lambda6-benzo[1,4,3]oxathiazin-3-ylamino)-1,7,7-trimethyl-bicyclo[2.2.1]heptan-2-ol * |
| 16 | 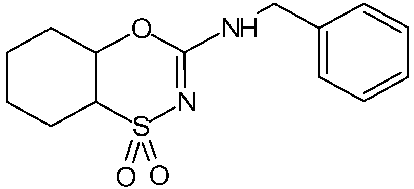 | C | 9.237 | 294.4 | (+)-Benzyl-(-1,1-dioxo-4a,5,6,7,8,8a-hexahydro-1H-1lambda6-benzo[1,4,3]oxathiazin-3-yl)-amin * |
| 20 | 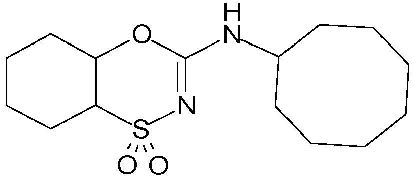 | A | 1.472 | 314.4 | Cyclooctyl-(1,1-dioxo-4a,5,6,7,8,8a-hexahydro-1H-1lambda6-benzo[1,4,3]oxathiazin-3-yl)-amin |
| 21 | 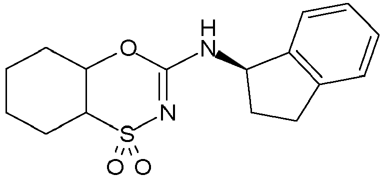 | A | 1.336 | 320.4 | (1,1-Dioxo-4a,5,6,7,8,8a-hexahydro-1 H-1lambda6-benzo[1,4,3]oxathiazin-3-yl)-(R)-indan-1-yl-amin |
| 22 | 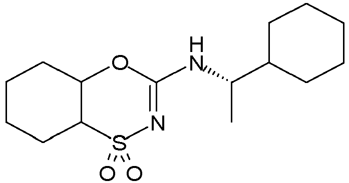 | A | 1.477 | 314.4 | ((S)-1-Cyclohexyl-ethyl)-(1,1-dioxo-4a,5,6,7,8,8a-hexahydro-1H-1lambda6-benzo[1,4,3]oxathiazin-3-yl)-amin |
| 23 | 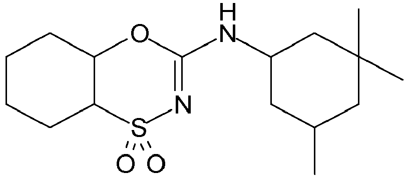 | A | 1.58 | 328.5 | (1,1-Dioxo-4a,5,6,7,8,8a-hexahydro-1H-1lambda6-benzo[1,4,3]oxathiazin-3-yl)-(3,3,5-trimethyl-cyclohexyl)-amin |
| 24 | 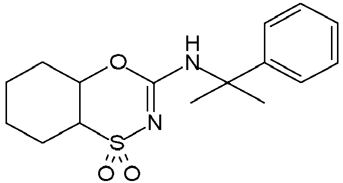 | A | 1.351 | 322.4 | (1,1-Dioxo-4a,5,6,7,8,8a-hexahydro-1 H-1lambda6-benzo[1,4,3]oxathiazin-3-yl)-(1-methyl-1-phenyl-ethyl)-amin |
| 25 | 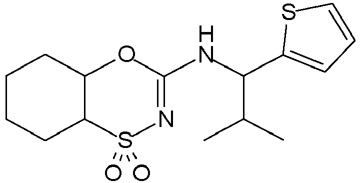 | A | 1.424 | 342.5 | (1,1-Dioxo-4a,5,6,7,8,8a-hexahydro-1 H-1lambda6-benzo[1,4,3]oxathiazin-3-yl)-(2-methyl-1-thiophen-2-yl-propyl)-amin |
| 26 | 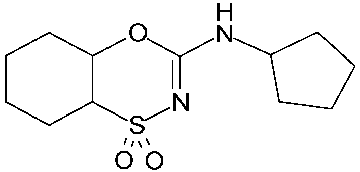 | A | 1.177 | 272.4 | Cyclopentyl-(1,1-dioxo-4a,5,6,7,8,8a-hexahydro-1H-1lambda6-benzo[1,4,3]oxathiazin-3-yl)-amin |
| 27 | 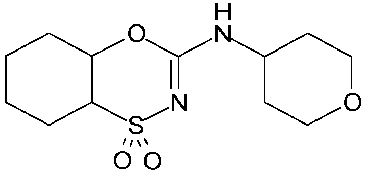 | A | 0.852 | 288.4 | (1,1-Dioxo-4a,5,6,7,8,8a-hexahydro-1 H-1lambda6-benzo[1,4,3]oxathiazin-3-yl)-(tetrahydro-pyran-4-yl)-amin |
| 28 | 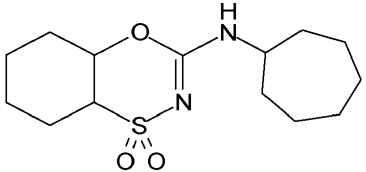 | A | 1.357 | 300.4 | Cycloheptyl-(1,1-dioxo-4a,5,6,7,8,8a-hexahydro-1H-1lambda6-benzo[1,4,3]oxathiazin-3-yl)-amin |
| 29 | 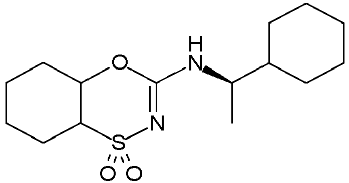 | A | 1.475 | 314.4 | ((R)-1-Cyclohexyl-ethyl)-(1,1-dioxo-4a,5,6,7,8,8a-hexahydro-1H-1lambda6-benzo[1,4,3]oxathiazin-3-yl)-amin |
| 31 | 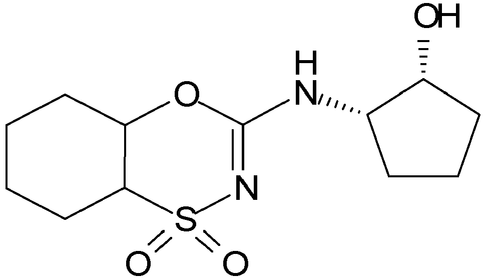 | A | 0.817 | 288.4 | (1R,2S)-2-(1,1-Dioxo-4a,5,6,7,8,8a-hexahydro 1H-1lambda6-benzo[1,4,3]oxathiazin-3-ylamino)-cyclopentanol |
| 35 | 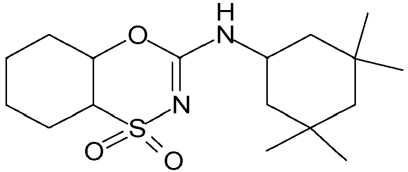 | A | 1.771 | 342.5 | (1,1-Dioxo-4a,5,6,7,8,8a-hexahydro-1 H-1lambda6-benzo[1,4,3]oxathiazin-3-yl)-(3,3,5,5-tetramethyl-cyclohexyl)-amin |
| 36 | 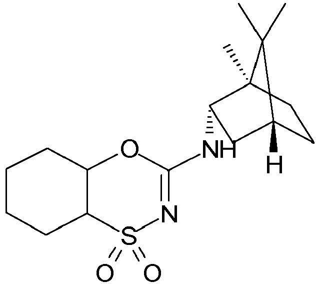 | A | 1.553 | 340.5 | (1,1-Dioxo-4a,5,6,7,8,8a-hexahydro-1H-1lambda6-benzo[1,4,3]oxathiazin-3-yl)-((1R,2S,4R)-1,7,7-trimethyl-bicyclo[2.2.1 ]hept-2-yl)-amin |
| 38 | 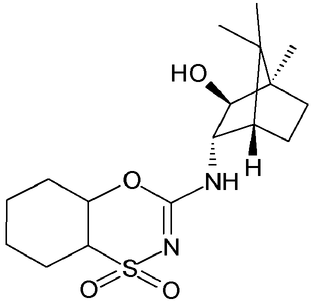 | A | 1.289 | 356.5 | (1R,2S,3S,4S)-3-(1,1-Dioxo-4a,5,6,7,8,8a-hexahydro-1H-1lambda6-benzo[1,4,3]oxathiazin-3-ylamino)-1,7,7-trimethyl-bicyclo[2.2.1]heptan-2-ol |
| 39 | 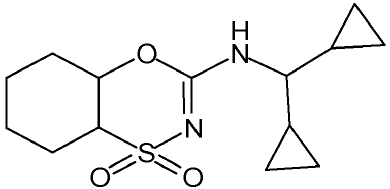 | A | 1.264 | 298.4 | Dicyclopropylmethyl-(1,1-dioxo-4a,5,6,7,8,8a-hexahydro-1H-1lambda6-benzo[1,4,3]oxathiazin-3-yl)-amin |
| 40 | 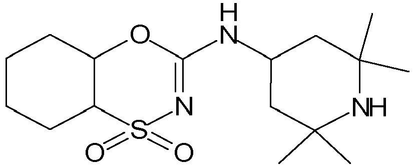 | A | 0.786 | 343.5 | (1,1-Dioxo-4a,5,6,7,8,8a-hexahydro-1 H-1lambda6-benzo[1,4,3]oxathiazin-3-yl)-(2,2,6,6-tetramethyl-piperidin-4-yl)-amin |
| 41 | 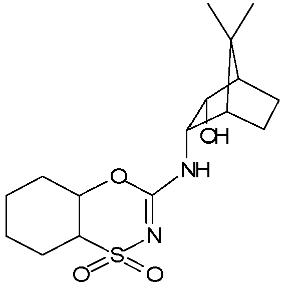 | A | 1.144 | 342.5 | 3-(1,1-Dioxo-4a,5,6,7,8,8a-hexahydro-1 H-1lambda6-benzo[1,4,3]oxathiazin-3-ylamino)-7,7-dimethyl-bicyclo[2.2.1]heptan-2-ol |
| 42 | 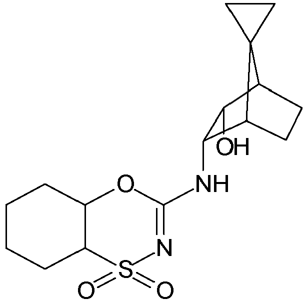 | A | 1.145 | 340.4 | 3-(1,1-Dioxo-4a,5,6,7,8,8a-hexahydro-1H-1lambda6-benzo[1,4,3]oxathiazin-3-ylamino)-7-cyclopropyl-bicyclo[2.2.1]heptan-2-ol |
| 43 | 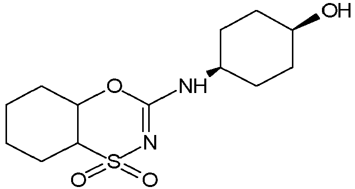 | A | 0.831 | 302.4 | 4-(1,1-Dioxo-4a,5,6,7,8,8a-hexahydro-1H-1lambda6-benzo[1,4,3]oxathiazin-3-ylamino)-cyclohexanol |
| 44 | 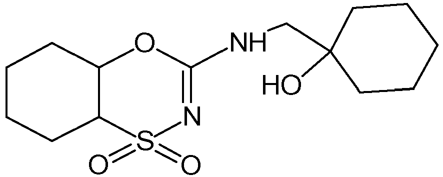 | A | 1.034 | 316.4 | 1-[(1,1-Dioxo-4a,5,6,7,8,8a-hexahydro-1 H-1lambda6-benzo[1,4,3]oxathiazin-3-ylamino)-methyl]-cyclohexanol |
| 45 | 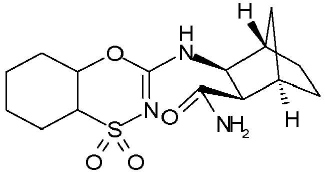 | A | 0.916 | 341.4 | (1R,2R,3S,4S)-3-(-1,1-Dioxo-4a,5,6,7,8,8a-hexahydro-1H-1lambda6-benzo[1,4,3]oxathiazin-3-ylamino)-bicyclo[2.2.1]heptane-2-carboxylic acid amid |
| 46 | 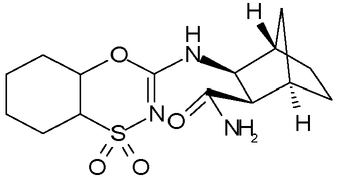 | A | 0.98 | 341.4 | (1R,2R,3S,4S)-3-(-1,1-Dioxo-4a,5,6,7,8,8a-hexahydro-1H-1lambda6-benzo[1,4,3]oxathiazin-3-ylamino)-bicyclo[2.2.1]heptane-2-carboxylic acid amid |
| 47 | 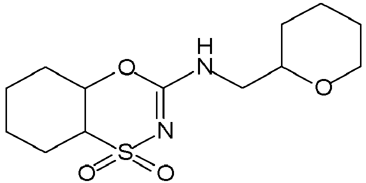 | A | 1.043 | 302.4 | (1,1-Dioxo-4a,5,6,7,8,8a-hexahydro-1H-1lambda6-benzo[1,4,3]oxathiazin-3-yl)-(tetrahydro-pyran-2-ylmethyl)-amin |
| 48 | 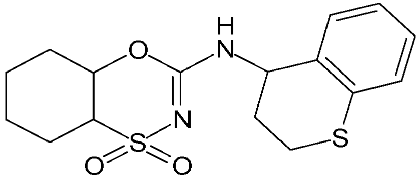 | A | 1.343 | 352.5 | (1,1-Dioxo-4a,5,6,7,8,8a-hexahydro-1 H-1lambda6-benzo[1,4,3]oxathiazin-3-yl)-thiochroman-4-yl-amin |
| 49 | 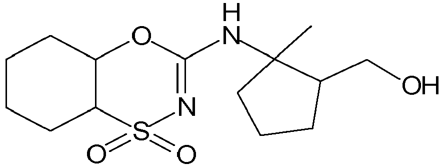 | A | 0.903 | 316.4 | [2-(1,1-Dioxo-4a,5,6,7,8,8a-hexahydro-1 H-1lambda6-benzo[1,4,3]oxathiazin-3-ylamino)-2-methyl-cyclopentyl]-methanol |
| 50 | 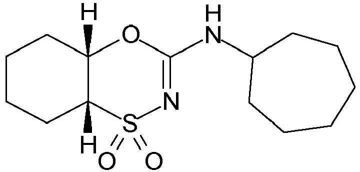 | M | 11.255 | 300.4 | Cycloheptyl-((4aS,8aR)-1,1-dioxo-4a,5,6,7,8,8a-hexahydro-1H-1lambda6-benzo[1,4,3]oxathiazin-3-yl)-amine |
| 51 | 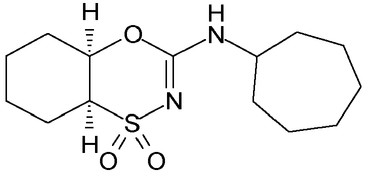 | M | 14.565 | 300.4 | Cycloheptyl-((4aR,8aS)-1,1-dioxo-4a,5,6,7,8,8a-hexahydro-1H-1lambda6-benzo[1,4,3]oxathiazin-3-yl)-amine |
| 53 | 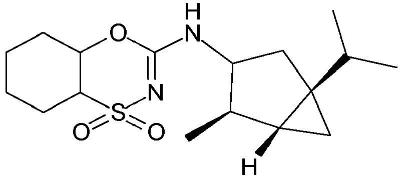 | A | 1.615 | 340.5 | (1,1-Dioxo-4a,5,6,7,8,8a-hexahydro-1 H-1lambda6-benzo[1,4,3]oxathiazin-3-yl)-((1S,4R,5R)-1-isopropyl-4-methyl-bicyclo[3.1.0]hex-3-yl)-amin |
| 54 | 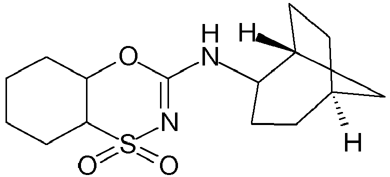 | A | 1.398 | 312.4 | (1R,5R)-Bicyclo[3.2.1]oct-2-yl-(1,1-dioxo-4a,5,6,7,8,8a-hexahydro-1H-1lambda6-benzo[1,4,3]oxathiazin-3-yl)-amin |
| 55 | 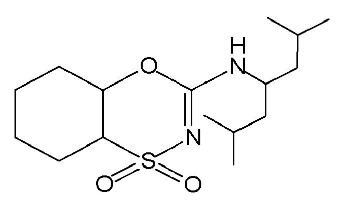 | A | 1.66 | 330.5 | (1,1-Dioxo-4a,5,6,7,8,8a-hexahydro-1 H-1lambda6-benzo[1,4,3]oxathiazin-3-yl)-(1-isobutyl-3-methyl-butyl)-amin |
| 56 | 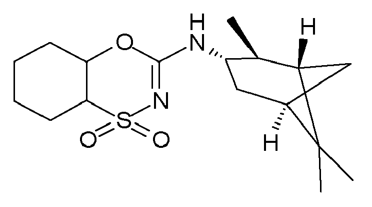 | A | 1.603 | 340.5 | (1,1-Dioxo-4a,5,6,7,8,8a-hexahydro-1 H-1lambda6-benzo[1,4,3]oxathiazin-3-yl)-((1S,2S,3S,5R)-2,6,6-trimethyl-bicyclo[3.1.1]hept-3-yl)-amin |
| 57 | 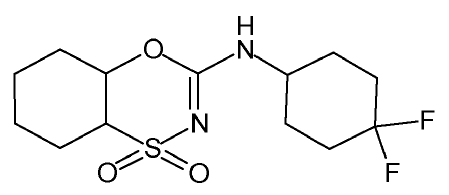 | A | 1.217 | 322.4 | (4,4-Difluoro-cyclohexyl)-(1,1-dioxo-4a,5,6,7,8,8a-hexahydro-1 H-1lambda6-benzo[1,4,3]oxathiazin-3-yl)-amin |
| 58 | 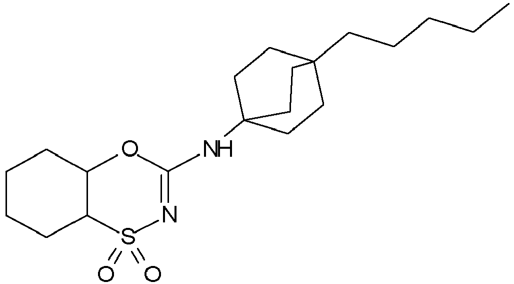 | A | 1.958 | 382.6 | ((4aS,8aR)-1,1-Dioxo-4a,5,6,7,8,8a-hexahydro-1H-1lambda6-benzo[1,4,3]oxathiazin-3-yl)-(4-pentyl-bicyclo[2.2.2]oct-1-yl)-amin |
| 59 | 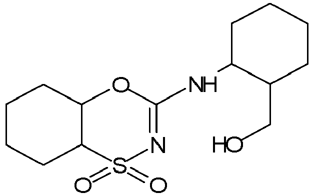 | A | 1.01 | 316.4 | [(1R,2R)-2-((4aS,8aR)-1,1-Dioxo-4a,5,6,7,8,8a-hexahydro-1H-1lambda6-benzo[1,4,3]oxathiazin-3-ylamino)-cyclohexyl] methanol |
| 61 | 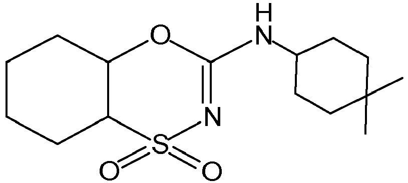 | A | 1.472 | 314.5 | (4,4-Dimethyl-cyclohexyl)-(1,1-dioxo-4a,5,6,7,8,8a-hexahydro-1H-1lambda6-benzo[1,4,3]oxathiazin-3-yl)-amin |
| 62 | 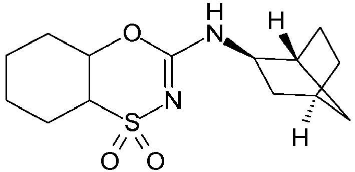 | A | 1.297 | 298.4 | (1R,2R,4S)-Bicyclo[2.2.1]hept-2-yl-((5S,6R)-1,1-dioxo-4a,5,6,7,8,8a-hexahydro-1 H-1lambda6-benzo[1,4,3]oxathiazin-3-yl)-amin |
| 64 | 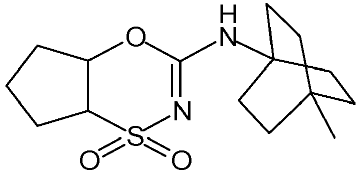 | A | 1.391 | 312.4 | (1,1-Dioxo-1,4a,5,6,7,7a-hexahydro-1lambda6-cyclopenta[1,4,3]oxathiazin-3-yl)-(4 methyl-bicyclo[2.2.2]oct-1-yl)-amin |
| 65 | 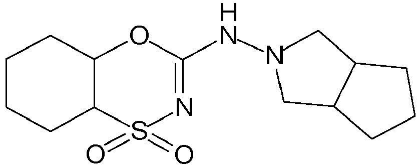 | A | 1.1 | 313.4 | (1,1-Dioxo-4a,5,6,7,8,8a-hexahydro-1H-1lambda6-benzo[1,4,3]oxathiazin-3-yl)-(hexahydro-cyclopenta[c]pyrrol-2-yl)-amin |
| 66 | 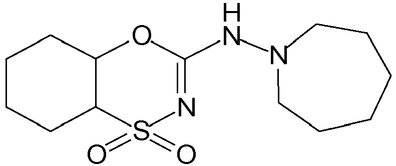 | A | 1.021 | 301.4 | Azepan-1-yl-(1,1-dioxo-4a,5,6,7,8,8a-hexahydro-1H-1lambda6-benzo[1,4,3]oxathiazin-3-yl)-amin |
| 67 | 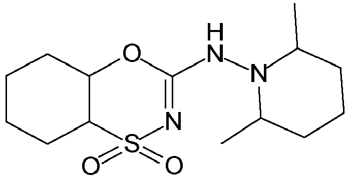 | A | 1.071 | 315.4 | (2,6-Dimethyl-piperidin-1-yl)-(1,1-dioxo-4a,5,6,7,8,8a-hexahydro-1 H-1lambda6-benzo[1,4,3]oxathiazin-3-yl)-amin |
| 68 | 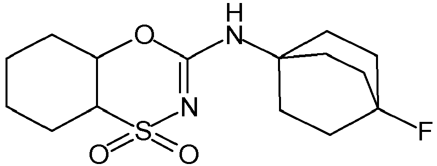 | A | 1.213 | 330.4 | (1,1-Dioxo-4a,5,6,7,8,8a-hexahydro-1 H-1lambda6-benzo[1,4,3]oxathiazin-3-yl)-(4-fluoro-bicyclo[2.2.2]oct-1-yl)-amin |
| 71 | 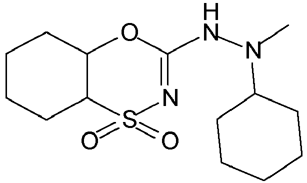 | A | 1.241 | 315.4 | N-Cyclohexyl-N'-(1,1-dioxo-4a,5,6,7,8,8a-hexahydro-1H-1lambda6-benzo[1,4,3]oxathiazin-3-yl)-N-methyl-hydrazin |
| 72 | 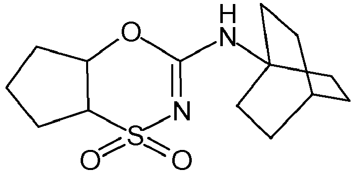 | A | 1.202 | 298.4 | Bicyclo[2.2.2]oct-1-yl-(1,1-dioxo-1,4a,5,6,7,7a hexahydro-1lambda6-cyclopenta[1,4,3]oxathiazin-3-yl)-amin |
| 73 | 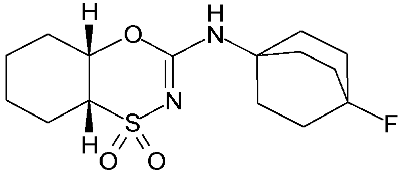 | B | 5.707 | 330.4 | ((4aS,8aR)-1,1-Dioxo-4a,5,6,7,8,8a-hexahydro-1H-1lambda6-benzo[1,4,3]oxathiazin-3-yl)-(4-fluoro-bicyclo[2.2.2]oct-1-yl)-amin * |
| 74 | 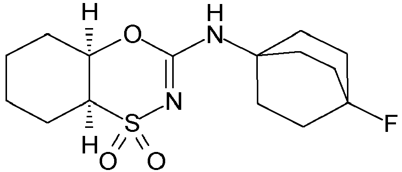 | B | 7.45 | 330.4 | ((4aR,8aS)-1,1-Dioxo-4a,5,6,7,8,8a-hexahydro-1H-1lambda6-benzo[1,4,3]oxathiazin-3-yl)-(4-fluoro-bicyclo[2.2.2]oct-1-yl)-amin * |
| 75 | 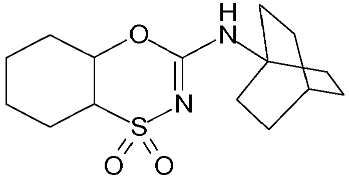 | F | 24.152 | 312.4 | (+)-Bicyclo[2.2.2]oct-1-yl-(-1,1-dioxo-4a,5,6,7,8,8a-hexahydro-1 H-1lambda6-benzo[1,4,3]oxathiazin-3-yl)-amin * |
| 76 | 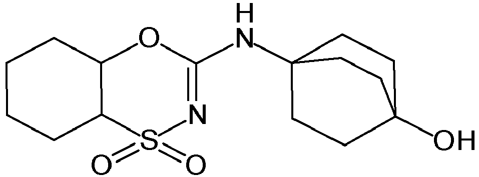 | A | 0.882 | 328.4 | 4-(1,1-Dioxo-4a,5,6,7,8,8a-hexahydro-1H-1lambda6-benzo[1,4,3]oxathiazin-3-ylamino)-bicyclo[2.2.2]octan-1-ol |
| 77 | 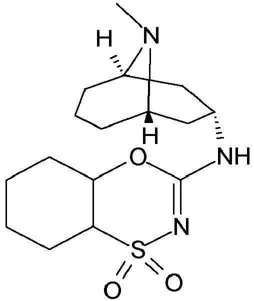 | L | 0.717 | 341.5 | (-1,1-Dioxo-4a,5,6,7,8,8a-hexahydro-1 H-1lambda6-benzo[1,4,3]oxathiazin-3-yl)-((1S,3R,5R)-9-methyl-9-aza-bicyclo[3.3.1]non-3-yl)-amin |
| 78 | 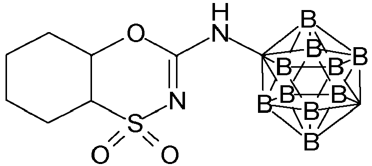 | A | 1.587 | 346.4 | (2,3,4,5,6,7,8,9,10,11-Decabora-bicyclo[8.1.1]dodec-1-yl)-(1,1-dioxo-4a,5,6,7,8,8a-hexahydro-1H-1lambda6-benzo[1,4,3]oxathiazin-3-yl)-amin |
| 79 | 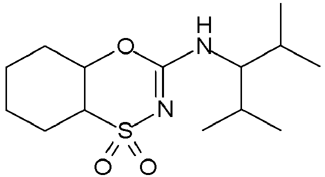 | A | 1.383 | 300.4 | (1,1-Dioxo-4a,5,6,7,8,8a-hexahydro-1 H-1lambda6-benzo[1,4,3]oxathiazin-3-yl)-(1-isopropyl-2-methyl-propyl)-amin |
| 80 | 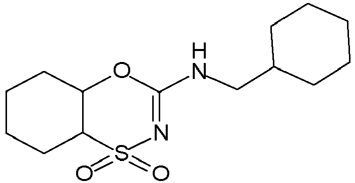 | A | 1.37 | 300.4 | Cyclohexylmethyl-(1,1-dioxo-4a,5,6,7,8,8a-hexahydro-1H-1lambda6-benzo[1,4,3]oxathiazin-3-yl)-amin |
| 81 | 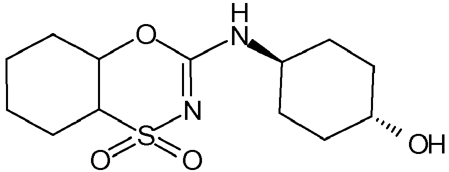 | A | 0.789 | 302.4 | 4-(1,1-Dioxo-4a,5,6,7,8,8a-hexahydro-1H-1lambda6-benzo[1,4,3]oxathiazin-3-ylamino)-cyclohexanol |
| 82 | 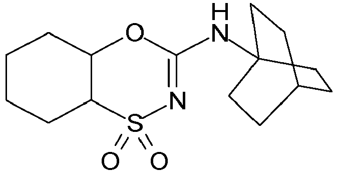 | F | 15.169 | 312.4 | (-)-Bicyclo[2.2.2]oct-1-yl-(-1,1-dioxo-4a,5,6,7,8,8a-hexahydro-1H-1lambda6-benzo[1,4,3]oxathiazin-3-yl)-amin * |
| 83 | 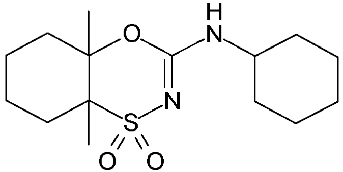 | A | 1.354 | 314.4 | Cyclohexyl-(4a,8a-dimethyl-1,1-dioxo-4a,5,6,7,8,8a-hexahydro-1H-1lambda6-benzo[1,4,3]oxathiazin-3-yl)-amin |
| 84 | 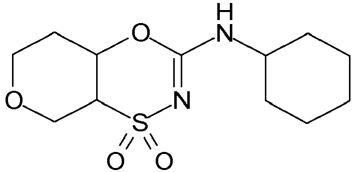 | N | 0.689 | 288.4 | Cyclohexyl-(-1,1-dioxo-5,6,8,8a-tetrahydro-1H,4aH-4,7-dioxa-1lambda6-thia-2-aza-naphthalen-3-yl)-amin ** |
| 85 | 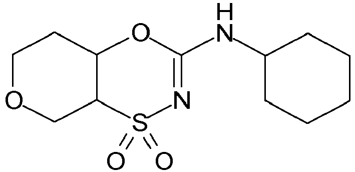 | N | 0.703 | 288.4 | Cyclohexyl-(-1,1-dioxo-5,6,8,8a-tetrahydro-1H,4aH-4,7-dioxa-1lambda6-thia-2-aza-naphthalen-3-yl)-amin |
| 86 | 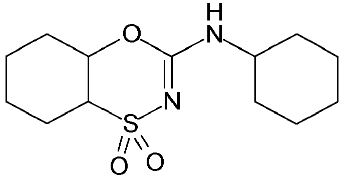 | P | 39.602 | 286.4 | Cyclohexyl-(-1,1-dioxo-4a,5,6,7,8,8a-hexahydro-1H-1lambda6-benzo[1,4,3]oxathiazin-3-yl)-amin *,** |
| 87 | 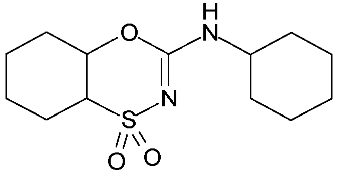 | P | 44.273 | 286.4 | Cyclohexyl-(-1,1-dioxo-4a,5,6,7,8,8a-hexahydro-1H-1lambda6-benzo[1,4,3]oxathiazin-3-yl)-amin *, ** |
| 88 | 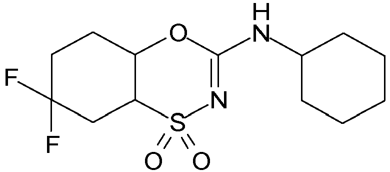 | N | 0.849 | 322.4 | Cyclohexyl-(7,7-difluoro-1,1-dioxo-4a,5,6,7,8,8a-hexahydro-1 H-1lambda6-benzo[1,4,3]oxathiazin-3-yl)-amin |
| 89 | 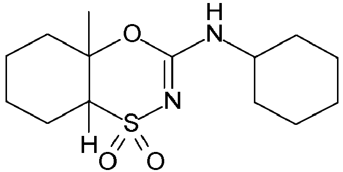 | L | 1.005 | 300.4 | Cyclohexyl-(-4a-methyl-1,1-dioxo-4a,5,6,7,8,8a-hexahydro-1 H-1lambda6-benzo[1,4,3]oxathiazin-3-yl)-amin |

| | | | | | |
|---|---|---|---|---|---|
| * Isomerenreine Verbindung ** trans | | | | | |

### Chromatographie-Methoden:

### Methode A

Säule: YMC J'spere ODS H80, 80 Ǻ, S-4 µm, 20 x 2.1 mm
Laufmittel: 0 min 90 % H₂O (0.05 % TFA) - 1.9 min 95 % Acetonitril - 2.4 min 95 % Acetonitril - 2.45 min 10% Acetonitril (30 °C, Fluss 1 ml / min)

### Methode B

Säule: Chiralpak AS-H/75, 5 µm, 250 x 4.6 mm
Laufmittel: Heptan : Ethanol : Methanol 5 : 1 : 1 (30 °C, Fluss 1 ml / min)

### Methode C

Säule: Chiralpak AD-H/44, 5 µm, 250 x 4.6 mm
Laufmittel: Heptan : Ethanol : Methanol 5 : 1 : 1 (30 °C, Fluss 1 ml / min)

### Methode D

Säule: Chiralpak AD-H/39, 5 µm, 250 x 4.6 mm
Laufmittel: Heptan : Ethanol : Methanol 5 : 1 : 1 (30 °C, Fluss 1 ml / min)

### Methode E

Säule: Chiralpak AS-H/52, 5 µm, 250 x 4.6 mm
Laufmittel: Heptan : Ethanol : Methanol 5 : 1 : 1 (30 °C, Fluss 1 ml / min)

### Methode F

Säule: Chiralpak AS-H/52, 5 µm, 250 x 4.6 mm
Laufmittel: Heptan : Ethanol : Methanol 10 : 1 : 1 (30 °C, Fluss 1 ml / min)

### Methode K

Säule: YMC J'spere 33 x 2 mm 4 µM
Laufmittel: 0 min 95 % H₂O(0.05 % TFA) - 0.5 min 95 % Acetonitril (0.05 % TFA) - 3.5 min 95 % Acetonitril - 4.0 min 95 % Acetonitril (30 °C, Fluss 1 ml / min)

### Methode L

Säule: YMC J'spere ODS H80, 80 Ǻ, S-4 µm, 20 x 2.1 mm
Laufmittel: 0 min 96 % H₂O (0.05 % TFA) - 2.0 min 95 % Acetonitril - 2.4 min 95 % Acetonitril - 2.45 min 4% Acetonitril (30 °C, Fluss 1 ml / min)

### Methode M

Säule: Chiralpak AS-H/52, 5 µm, 250 x 4.6 mm
Laufmittel: Heptan : Ethanol : Methanol 10 : 1 : 1 , vorkonditioniert mit TFA (30 °C, Fluss 1 ml / min)

### Methode N

Säule: Mercury MS, Luna C18(2), S-3 µm, 10 x 2.0 mm
Laufmittel: 0 min 93 % H₂O (0.05 % TFA) - 1.2 min 95 % Acetonitril - 1.4 min 95 % Acetonitril - 1.45 min 7% Acetonitril (30 °C, Fluss 1.1 ml / min)

### Methode O

Säule: Mercury MS, Luna C18(2), S-3 µm, 10 x 2.0 mm
Laufmittel: 0 min 93 % H₂O (0.05 % TFA) - 1.0 min 95 % Acetonitril - 1.45 min 95 % Acetonitril - 1.5 min 7% Acetonitril (30 °C, Fluss 1.1 ml / min)

### Methode P

Säule: Chiralpak IA/103
Laufmittel: Heptan : Ethanol : Methanol 40 : 1 : 1 + 0,1 % Trifluoressigsäure (30 °C, Fluss 1 ml / min)

### Die Wirksamkeit der Verbindungen wurde wie folgt getestet:

### Enzymatischer 11beta-HSD1 Test:

Zum Messen der Wirkung der Verbindungen wurde ein auf SPA basierendes Nachweisverfahren (Solly *et al.* 2005) angewendet. Zunächst wurden 20 µl der humanen 11β-HSD1-Mikrosomenfraktion (0,2 µg Protein), zubereitet in 50 mM HEPES, 0,1% BSA (w/v), in eine Platte mit 384 Vertiefungen gegeben. Die Testverbindungen (0,09 µl) wurden in 100% DMSO auf die Assayplatte übertragen. Die Reaktion wurde durch Zugabe von 20 µl [1,2-³H] Cortison (0,1 µCi/100 mM) in Assaypuffer mit 25 mM HEPES, 100 mM KCl, 5 mM NaCl, 2 mM MgCl₂ und 0,25 mM NADPH gestartet. Die Platte wurde 1 Stunde lang bei 37°C geschüttelt. Gleichzeitig wurde eine Stopplösung mit 20 mg/ml SPA-PVT-Perlen, 1,6 mg/ml monoklonalem Cortisol-Antikörper und 0,01 mM SSR110887 (Inhibitor aus dem Biovitrium-Patent) in 50 mM HEPES, 1 M NaCl und 1 M KCl bei Raumtemperatur gerührt. Zum Abbruch der Reaktion wurden in alle Vertiefungen jeweils 25 µl der Stopplösung gegeben. Die Platte wurde 1 weitere Stunde lang sachte bei Raumtemperatur geschüttelt und dann 1 min bei 500 gₐᵥ zentrifugiert, damit sich die SPA-Perlen absetzen konnten. Die Platte wurde dann in einem Wallac-1450-Microbeta-Gerät mit einem Standard-SPA-Programm gelesen (1 min Zählzeit/Vertiefung). Die Vergleichsverbindung war Glycyrrhetinsäure.

Protein und radioaktives Substrat wurden mit einem Biomek FX-Gerät (Beckman Coulter) zur Handhabung von Flüssigkeiten dispensiert. Die Testverbindungen wurden mit einem mit einem 90-nl-Pin-Tool ausgestatteten Cybi-Well (CyBio) zugesetzt.

Lit.: Solly S, Mundt SS, Zokian HJ, Juy-Fang Ding G, Hermanowski-Vosatka A, Strulovici B and Zheng W. High-throughput screening of 11β-Hydroxysteroid dehydrogenase type 1 in scintillation proximity format. Assay Drug Dev Technol 2005;3:377-384.

**Tabelle 2: Biologische Aktivität in Nanomolar (nM)**

| Beispiel | 1C₅₀ (nM) |
|---|---|
| 1 | 31 |
| 2 | 575 |
| 3 | 24 |
| 5 | 8 |
| 9 | 5 |
| 10 | 104 |
| 13 | 5 |
| 14 | 236 |
| 20 | 13 |
| 23 | 20 |
| 24 | 215 |
| 25 | 121 |
| 26 | 425 |
| 27 | 671 |
| 28 | 22 |
| 29 | 275 |
| 35 | 11 |
| 36 | 59 |
| 38 | 570 |
| 39 | 440 |
| 41 | 497 |
| 47 | 165 |
| 48 | 93 |
| 50 | 24 |
| 51 | 300 |
| 53 | 54 |
| 54 | 29 |
| 55 | 670 |
| 56 | 16 |
| 57 | 405 |
| 59 | 658 |
| 61 | 64 |
| 62 | 43 |
| 64 | 38 |
| 65 | 308 |
| 66 | 148 |
| 68 | 23 |
| 72 | 74 |
| 73 | 13 |
| 74 | 18 |
| 75 | 11 |
| 76 | 49 |
| 78 | 2 |
| 80 | 98 |
| 81 | 355 |
| 82 | 9 |
| 83 | 18 |
| 84 | 779 |
| 86 | 655 |
| 87 | 197 |
| 88 | 234 |
| 89 | 49 |

Aus den Messdaten ist abzulesen, dass die Verbindungen der Formel I 11beta-HSD1 (11beta-Hydroxysteroid Dehydrogenase Type 1) inhibieren und dadurch gut zur Behandlung von Hyperglykämie, Insulin Resistenz, Diabetes, Adipositas, Lipidstoffwechselstörungen, Bluthochdruck, Verbesserung der Kognition, erhöhtem Augeninnendruck, der Förderung der Wundheilung und anderen Erkrankungen geeignet sind.

Nachfolgend wird die Herstellung einiger Beispiele detailliert beschrieben, die übrigen Verbindungen der Formel I wurden analog erhalten:

### Experimenteller Teil:

### Cyclohexyl-(1,1-dioxo-4a,5,6,7,8,8a-hexahydro-1H-1lambda6-benzo[1,4,3]oxathiazin-3-yl)-amin

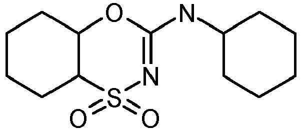

Unter Inertgas wurden 10 mmol Cyclohexylamin und 50 mmol Diisopropyl-ethyl-amin in 5 ml Dichlormethan vorgelegt und anschließend unter Eiskühlung 10 mmol 2-Chlor-cyclohexansulfonylisocyanat in 5 ml Dichlormethan gelöst tropfenweise zugegeben und 1 Stunde gerührt. Die Vollständigkeit der Umsetzung wurde mit LCMS geprüft. Falls noch Cyclohexylamin vorhanden war, wurden nochmals 0.4 eq 2-Chlor-cyclohexansulfonylisocyanat zugegeben und über Nacht unter Rühren auf Raumtemperatur kommen gelassen. Die Reaktionslösung wurde mit 50 ml Dichlormethan verdünnt und 2 x mit 20 ml 10 %iger KHSO₄- Lösung extrahiert, die organische Phase über MgSO₄ getrocknet und einrotiert (3.08 g). Der Rückstand wurde dann mit 0.5 eq (bezogen auf das Amin) 0.1 N NaOH versetzt. Zugabe von Dioxan (z.B. bei 20 ml 0.1 N NaOH / 5ml Dioxan) und auf 100 °C erhitzt. Bei 100 °C entsteht eine klare Lösung. Falls noch etwas Ausgangsmaterial vorhanden ist, wurden 1-2 Tropfen 1 N NaOH zugegeben. Nach weiteren 30 Minuten bei 100 °C vollständige Umsetzung. Nach dem Abkühlen wurde mit 30 ml Dichlormethan versetzt und 2 x mit 10 ml gesättigter Na₂CO₃-Lösung gewaschen. Die wässrige Lösung wurde noch 1 x mit 20 ml Dichlormethan extrahiert. Die vereinigten organischen Phasen wurden über MgSO₄ getrocknet und einrotiert. Der Rückstand wurde in DMF gelöst und im Reinigungslabor über präparative HPLC gereinigt. Man erhielt so das Produkt (244 mg) mit dem Molekulargewicht 286.4 g / mol (C₁₃H₂₂N₂O₃S); MS (ESI): m / e = 287 (M+H+).

Die Verbindungen 1-82 wurden mit dieser Herstellungsvorschrift synthetisiert:
(-)-Cyclohexyl-((4aR,8aS)-1,1-dioxo-4a,5,6,7,8,8a-hexahydro-1H-1lambda6-benzo[1,4,3]oxathiazin-3-yl)-amin *
(+)-Cyclohexyl-((4aS,8aR)-1,1-dioxo-4a,5,6,7,8,8a-hexahydro-1H-1lambda6-benzo[1,4,3]oxathiazin-3-yl)-amin *
((4aR,8aS)-1,1-Dioxo-4a,5,6,7,8,8a-hexahydro-1H-1lambda6-benzo[1,4,3]oxathiazin-3-yl)-(4-methyl-bicyclo[2.2.2]oct-1-yl)-amin *
3-((4aS,8aR)-1,1-Dioxo-4a,5,6,7,8,8a-hexahydro-1H-1lambda6-benzo[1,4,3]oxathiazin-3-ylamino)-N,N-diethyl-propionamid *
(-)-(1R,2S,3R,4S)-3-(-1,1-Dioxo-4a,5,6,7,8,8a-hexahydro-1H-1lambda6-benzo[1,4,3]oxathiazin-3-ylamino)-1,7,7-trimethyl-bicyclo[2.2.1]heptan-2-ol *
(-1,1-Dioxo-4a,5,6,7,8,8a-hexahydro-1H-llambda6-benzo[1,4,3]oxathiazin-3-yl)-((1R,2R,4R)-1,7,7-trimethyl-bicyclo[2.2.1]hept-2-yl)-amin *
(-)-Benzyl-(-1,1-dioxo-4a,5,6,7,8,8a-hexahydro-1H-1lambda6-benzo[1,4,3]oxathiazin-3-yl)-amin *
3-(-1,1-Dioxo-4a,5,6,7,8,8a-hexahydro-1H-1lambda6-benzo[1,4,3]oxathiazin-3-ylamino)-N,N-diethyl-propionamid *
((4aS,8aR)-1,1-Dioxo-4a,5,6,7,8,8a-hexahydro-1H-1lambda6-benzo[1,4,3]oxathiazin-3-yl)-(4-methyl-bicyclo[2.2.2]oct-1-yl)-amin *
(-1,1-Dioxo-4a,5,6,7,8,8a-hexahydro-1H-1lambda6-benzo[1,4,3]oxathiazin-3-yl)-((1R,2R,4R)-1,7,7-trimethyl-bicyclo[2.2.1]hept-2-yl)-amin *
(-)-(1R,2S,3R,4S)-3-(-1,1-Dioxo-4a,5,6,7,8,8a-hexahydro-1H-1lambda6-benzo[1,4,3]oxathiazin-3-ylamino)-1,7,7-trimethyl-bicyclo[2.2.1]heptan-2-ol *
(+)-Benzyl-(-1,1-dioxo-4a,5,6,7,8,8a-hexahydro-1H-1lambda6-benzo[1,4,3]oxathiazin-3-yl)-amin *
(1,1-Dioxo-4a,5,6,7,8,8a-hexahydro-1H-1lambda6-benzo[1,4,3]oxathiazin-3-yl)-(hexahydro-2,5-methano-pentalen-3a-yl)-amin
Cyclooctyl-(1,1-dioxo-4a,5,6,7,8,8a-hexahydro-1H-1lambda6-benzo[1,4,3]oxathiazin-3-yl)-amin
(1,1-Dioxo-4a,5,6,7,8,8a-hexahydro-1H-1lambda6-benzo[1,4,3]oxathiazin-3-yl)-(R)-indan-1-yl-amin
((S)-1-Cyclohexyl-ethyl)-(1,1-dioxo-4a,5,6,7,8,8a-hexahydro-1H-1lambda6-benzo[1,4,3]oxathiazin-3-yl)-amin
(1,1-Dioxo-4a,5,6,7,8,8a-hexahydro-1H-1lambda6-benzo[1,4,3]oxathiazin-3-yl)-(3,3,5-trimethyl-cyclohexyl)-amin
(1,1-Dioxo-4a,5,6,7,8,8a-hexahydro-1H-1lambda6-benzo[1,4,3]oxathiazin-3-yl)-(1-methyl-1-phenyl-ethyl)-amin
(1,1-Dioxo-4a,5,6,7,8,8a-hexahydro-1H-1lambda6-benzo[1,4,3]oxathiazin-3-yl)-(2-methyl-1-thiophen-2-yl-propyl)-amin
Cyclopentyl-(1,1-dioxo-4a,5,6,7,8,8a-hexahydro-1H-1lambda6-benzo[1,4,3]oxathiazin-3-yl)-amin
(1,1-Dioxo-4a,5,6,7,8,8a-hexahydro-1H-1lambda6-benzo[1,4,3]oxathiazin-3-yl)-(tetrahydro-pyran-4-yl)-amin
Cycloheptyl-(1,1-dioxo-4a,5,6,7,8,8a-hexahydro-1H-1lambda6-benzo[1,4,3]oxathiazin-3-yl)-amin
((R)-1-Cyclohexyl-ethyl)-(1,1-dioxo-4a,5,6,7,8,8a-hexahydro-1H-1lambda6-benzo[1,4,3]oxathiazin-3-yl)-amin
(1R,2S)-2-(1,1-Dioxo-4a,5,6,7,8,8a-hexahydro-1H-1lambda6-benzo[1,4,3]oxathiazin-3-ylamino)-cyclopentanol
(1,1-Dioxo-4a,5,6,7,8,8a-hexahydro-1H-1lambda6-benzo[1,4,3]oxathiazin-3-yl)-(3,3,5,5-tetramethyl-cyclohexyl)-amin
(1,1-Dioxo-4a,5,6,7,8,8a-hexahydro-1H-1lambda6-benzo[1,4,3]oxathiazin-3-yl)-((1R,2S,4R)-1,7,7-trimethyl-bicyclo[2.2.1]hept-2-yl)-amin
(1R,2S,3S,4S)-3-(1,1-Dioxo-4a,5,6,7,8,8a-hexahydro-1H-1lambda6-benzo[1,4,3]oxathiazin-3-ylamino)-1,7,7-trimethyl-bicyclo[2.2.1]heptan-2-ol
Dicyclopropylmethyl-(1,1-dioxo-4a,5,6,7,8,8a-hexahydro-1H-1lambda6-benzo[1,4,3]oxathiazin-3-yl)-amin
(1,1-Dioxo-4a,5,6,7,8,8a-hexahydro-1H-1lambda6-benzo[1,4,3]oxathiazin-3-yl)-(2,2,6,6-tetramethyl-piperidin-4-yl)-amin
3-(1,1-Dioxo-4a,5,6,7,8,8a-hexahydro-1H-1lambda6-benzo[1,4,3]oxathiazin-3-ylamino)-7,7-dimethyl-bicyclo[2.2.1]heptan-2-ol
3-(1,1-Dioxo-4a,5,6,7,8,8a-hexahydro-1H-1lambda6-benzo[1,4,3]oxathiazin-3-ylamino)-7-cyclopropyl-bicyclo[2.2.1]heptan-2-ol
4-(1,1-Dioxo-4a,5,6,7,8,8a-hexahydro-1H-1lambda6-benzo[1,4,3]oxathiazin-3-ylamino)-cyclohexanol
1-[(1,1-Dioxo-4a,5,6,7,8,8a-hexahydro-1H-1lambda6-benzo[1,4,3]oxathiazin-3-ylamino)-methyl]-cyclohexanol
(1R,2R,3S,4S)-3-(-1,1-Dioxo-4a,5,6,7,8,8a-hexahydro-1H-1lambda6-benzo[1,4,3]oxathiazin-3-ylamino)-bicyclo[2.2.1]heptane-2-carboxylic acid amid
(1R,2R,3S,4S)-3-(-1,1-Dioxo-4a,5,6,7,8,8a-hexahydro-1H-1lambda6-benzo[1,4,3]oxathiazin-3-ylamino)-bicyclo[2.2.1]heptane-2-carboxylic acid amid
(1,1-Dioxo-4a,5,6,7,8,8a-hexahydro-1H-1lambda6-benzo[1,4,3]oxathiazin-3-yl)-(tetrahydro-pyran-2-ylmethyl)-amin
(1,1-Dioxo-4a,5,6,7,8,8a-hexahydro-1H-1lambda6-benzo[1,4,3]oxathiazin-3-yl)-thiochroman-4-yl-amin
[2-(1,1-Dioxo-4a,5,6,7,8,8a-hexahydro-1H-1lambda6-benzo[1,4,3]oxathiazin-3-ylamino)-2-methyl-cyclopentyl]-methanol
Cycloheptyl-((4aS,8aR)-1,1-dioxo-4a,5,6,7,8,8a-hexahydro-1H-1lambda6-benzo[1,4,33]oxathiazin-3-yl)-amine
Cycloheptyl-((4aR,8aS)-1,1-dioxo-4a,5,6,7,8,8a-hexahydro-1H-1lambda6-benzo[1,4,33]oxathiazin-3-yl)-amine
(1,1-Dioxo-4a,5,6,7,8,8a-hexahydro-1H-1lambda6-benzo[1,4,3]oxathiazin-3-yl)-((1S,4R,5R)-1-isopropyl-4-methyl-bicyclo[3.1.0]hex-3-yl)-amin
(1R,5R)-Bicyclo[3.2.1]oct-2-yl-(1,1-dioxo-4a,5,6,7,8,8a-hexahydro-1H-1lambda6-benzo[1,4,3]oxathiazin-3-yl)-amin
(1,1-Dioxo-4a,5,6,7,8,8a-hexahydro-1H-1lambda6-benzo[1,4,3]oxathiazin-3-yl)-(1-isobutyl-3-methyl-butyl)-amin
(1,1-Dioxo-4a,5,6,7,8,8a-hexahydro-1H-1lambda6-benzo[1,4,3]oxathiazin-3-yl)-((1S,2S,3S,5R)-2,6,6-trimethyl-bicyclo[3.1.1]hept-3-yl)-amin
(4,4-Difluor-cyclohexyl)-(1,1-dioxo-4a,5,6,7,8,8a-hexahydro-1H-1lambda6-benzo[1,4,3]oxathiazin-3-yl)-amin
((4aS,8aR)-1,1-Dioxo-4a,5,6,7,8,8a-hexahydro-1H-1lambda6-benzo[1,4,3]oxathiazin-3-yl)-(4-pentyl-bicyclo[2.2.2]oct-1-yl)-amin
[(1R,2R)-2-((4aS,8aR)-1,1-Dioxo-4a,5,6,7,8,8a-hexahydro-1H-1lambda6-benzo[1,4,3]oxathiazin-3-ylamino)-cyclohexyl]-methanol
(4,4-Dimethyl-cyclohexyl)-(1,1-dioxo-4a,5,6,7,8,8a-hexahydro-1H-1lambda6-benzo[1,4,3]oxathiazin-3-yl)-amin
(1R,2R,4S)-Bicyclo[2.2.1]hept-2-yl-((5S,6R)-1,1-dioxo-4a,5,6,7,8,8a-hexahydro-1H-1lambda6-benzo[1,4,3]oxathiazin-3-yl)-amin
(1,1-Dioxo-1,4a,5,6,7,7a-hexahydro-1lambda6-cyclopenta[1,4,3]oxathiazin-3-yl)-(4-methyl-bicyclo[2.2.2]oct-1-yl)-amin
(1,1-Dioxo-4a,5,6,7,8,8a-hexahydro-1H-1lambda6-benzo[1,4,3]oxathiazin-3-yl)-(hexahydro-cyclopenta[c]pyrrol-2-yl)-amin
Azepan-1-yl-(1,1-dioxo-4a,5,6,7,8,8a-hexahydro-1H-1lambda6-benzo[1,4,3]oxathiazin-3-yl)-amin
(2,6-Dimethyl-piperidin-1-yl)-(1,1-dioxo-4a,5,6,7,8,8a-hexahydro-1H-1lambda6-benzo[1,4,3]oxathiazin-3-yl)-amin
(1,1-Dioxo-4a,5,6,7,8,8a-hexahydro-1H-1lambda6-benzo[1,4,3]oxathiazin-3-yl)-(4-fluor-bicyclo[2.2.2]oct-1-yl)-amin
N-Cyclohexyl-N'-(1,1-dioxo-4a,5,6,7,8,8a-hexahydro-1H-1lambda6-benzo[1,4,3]oxathiazin-3-yl)-N-methyl-hydrazin
Bicyclo[2.2.2]oct-1-yl-(1,1-dioxo-1,4a,5,6,7,7a-hexahydro-1lambda6-cyclopenta[1,4,3]oxathiazin-3-yl)-amin
((4aS,8aR)-1,1-Dioxo-4a,5,6,7,8,8a-hexahydro-1H-1lambda6-benzo[1,4,3]oxathiazin-3-yl)-(4-fluor-bicyclo[2.2.2]oct-1-yl)-amin *
((4aR,8aS)-1,1-Dioxo-4a,5,6,7,8,8a-hexahydro-1H-1lambda6-benzo[1,4,3]oxathiazin-3-yl)-(4-fluor-bicyclo[2.2.2]oct-1-yl)-amin *
(+)-Bicyclo[2.2.2]oct-1-yl-(-1,1-dioxo-4a,5,6,7,8,8a-hexahydro-1H-1lambda6-benzo[1,4,3]oxathiazin-3-yl)-amin *
4-(1,1-Dioxo-4a,5,6,7,8,8a-hexahydro-1H-1lambda6-benzo[1,4,3]oxathiazin-3-ylamino)-bicyclo[2.2.2]octan-1-ol
(-1,1-Dioxo-4a,5,6,7,8,8a-hexahydro-1H-1lambda6-benzo[1,4,3]oxathiazin-3-yl)-((1S,3R,5R)-9-methyl-9-aza-bicyclo[3.3.1]non-3-yl)-amin
(2,3,4,5,6,7,8,9,10,11-Decabora-bicyclo[8.1.1]dodec-1-yl)-(1,1-dioxo-4a,5,6,7,8,8a-hexahydro-1H-1lambda6-benzo[1,4,3]oxathiazin-3 -yl)-amin
(1,1-Dioxo-4a,5,6,7,8,8a-hexahydro-1H-1lambda6-benzo[1,4,3]oxathiazin-3-yl)-(1-isopropyl-2-methyl-propyl)-amin
Cyclohexylmethyl-(1,1-dioxo-4a,5,6,7,8,8a-hexahydro-1H-1lambda6-benzo[1,4,3]oxathiazin-3-yl)-amin
4-(1,1-Dioxo-4a,5,6,7,8,8a-hexahydro-1H-1lambda6-benzo[1,4,3]oxathiazin-3-ylamino)-cyclohexanol
(-)-Bicyclo[2.2.2]oct-1-yl-(-1,1-dioxo-4a,5,6,7,8,8a-hexahydro-1H-1lambda6-benzo[1,4,3]oxathiazin-3-yl)-amin *
Cyclohexyl-(4a,8a-dimethyl-1,1-dioxo-4a,5,6,7,8,8a-hexahydro-1H-1lambda6-benzo[1,4,3]oxathiazin-3-yl)-amin
* Isomerenreine Verbindung
* trans

### 2-Chlor-cyclohexansulfonyl isocyanat

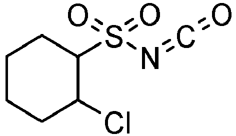

Chemische Berichte 1970, 103(3), 663-669

### 2-Chlor-cyclopentansulfonylisocyanat wurde analog hergestellt:

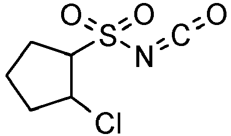

### 1-Isobutyl-3-methyl-butylamin wurde analog hergestellt:

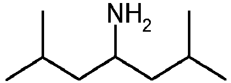

### 1-Isopropyl-2-methyl-propylamin

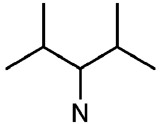

2,4-Dimethyl-3-pentanon (5 g) und Titan(IV)isopropoxid (24 g) wurden in methanolischer Ammoniak-Lösung 7 N (30 ml) 5 Stunden bei Raumtemperatur gerührt. Natriumborhydrid (2.5 g) wurden unter Kühlung der Lösung zugesetzt und es wurde weiter 2 Stunden bei Raumtemperatur gerührt. Anschließend wurde mit Ammoniumhydroxid-Lösung 10 N (5 ml) versetzt, filtriert, und dann die Lösung mit Salzsäure 2 N (pH < 2) versetzt. Die Lösung wurde mit Ethylacetat gewaschen und anschliessend zur Trockene eingedampft. Man erhielt so das Hydrochlorid des Produkts mit dem Molekulargewicht 115.2 g / mol (C₇H₁₇N), MS (ESI): m / e = 116 (M+H+)

### 4-Pentyl-bicyclo[2.2.2]oct-1-ylamin

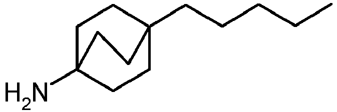

1.00 g 4-Pentyl-bicyclo[2.2.2]octan-1- carbonsäure wurden in einer Mischung aus 20 ml Toluol und 0.81 ml N,N-Diisopropylamin unter Argonatmosphäre gelöst und anschließend 1.06 ml Diphenylphosphorylazid zugetropft. Nach 2 Stunden Rühren bei 100 °C und Abkühlen auf Raumtemperatur wurde das Lösungsmittel im Vakuum entfernt. Der Rückstand wurde in Dichlormethan aufgenommen, mit 5 % iger wässriger Zitronensäure und gesättiger wässriger Natriumhydrogencarbonat-Lösung gewaschen, die organische Phase getrocknet und das Lösungsmittel im Vakuum entfernt. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (406 mg) mit dem Molekulargewicht 221.4 g / mol (C₁₄H₂₃NO). 390 mg 1-Isocyanato-4-pentyl-bicyclo[2.2.2]octan wurden in 10 ml einer wässrigen 6 N Salzsäure suspendiert und über 16 h bei 100 °C gerührt. Nach dem Abkühlen auf Raumtemperatur wurde der Niederschlag abgesaugt und anschließend durch Koevaporation mit Toluol im Vakuum getrocknet. Das Produkt (305 mg) mit dem Molekulargewicht 195.4 g / mol (C₁₃H₂₅N); MS (ESI): m / e = 196 (M+H+) wurde in Form eines Hydrochlorid-Salzes erhalten.

### 3-Acetyl-5-chlor-oxazolidin-2-on

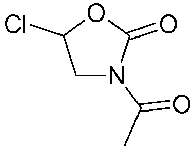

5.00 g 3-Acetyl-oxazolidin-2-on wurden in 25 ml Tetrachlorkohlenstoff vorgelegt und unter Argonatmosphäre auf 70 °C erwärmt. Dann wurde abwechselnd eine Lösung von 3.46 ml Sulfurylchlorid in 5 ml Tetrachlorkohlenstoff und 0.10 g 2,2'Azobis(2-methylpropionitril) zugegeben und anschließend 2 Stunden bei gleichbleibender Temperatur gerührt. Nach dem Abkühlen auf Raumtemperatur wurde die klare Lösung von den festen Rückständen dekantiert und das Lösungsmittel unter vermindertem Druck entfernt. Man erhielt so das Produkt (6.80 g) mit dem Molekulargewicht 163.6 g / mol (C₅H₆ClNO₃).

### 3-Acetyl-3H-oxazol-2-on

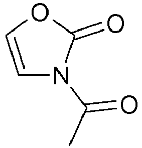

6.80 g 3-Acetyl-5-chlor-oxazolidin-2-on wurden in der Kugelrohrdestillation unter vermindertem Druck (50 mbar) bei einer Temperatur von 180 °C destilliert. Man erhielt so das Produkt (3.80 g) mit dem Molekulargewicht 127.1 g / mol (C₅H₅NO₃).

### 5-Acetyl-10-spiro-cyclopropyl-3-oxa-5-aza-tricyclo[5.2.1.0<2,6>]dec-8-en-4-on

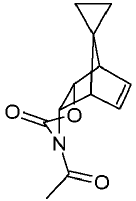

Ein Vial mit 3.00 g 3-Acetyl-3H-oxazol-2-on und 2.89 ml Spiro[2.4]hepta-4,6-dien in 6 ml 1,2-Dichlorethan wurde mit einem Teflon-Septum verschlossen und 90 Minuten bei 200 °C in der Mikrowelle (Emrys Optimizer, Personal Chemistry) gerührt. Anschließend wurde das Lösungsmittel unter vermindertem Druck entfernt und der Rückstand mittels zweimaliger Normalphasenchromatographie über den Flashmaster mit einem n-Heptan/Ethylacetat-Gradienten gereinigt. Die produkthaltigen Fraktionen wurden vereinigt und einrotiert.
Man erhielt so das Produkt (3.27 g) mit dem Molekulargewicht 219.2 g / mol (C₁₂H₁₃NO₃); MS (ESI): m / e = 220 (M+H+).

### 5-Acetyl-10-spiro-cyclopropyl-3-oxa-5-aza-tricyclo[5.2.1.0<2,6>]decan-4-on

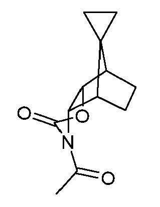

1.50 g 5-Acetyl-10-spiro-cyclopropyl-3-oxa-5-aza-tricyclo[5.2.1.0<2,6>]dec-8-en-4-on wurden in 30 ml Ethanol gelöst und unter Zusatz von 0.15 g Pd/C 5 % für 1 Stunde unter einem Wasserstoff-Überdruck von 0.5 bar gerührt. Nach Filtration von den festen Rückständen wurde das Filtrat unter vermindertem Druck vom Lösungsmittel befreit.
Man erhielt so das Produkt (1.47 g) mit dem Molekulargewicht 221.3 g / mol (C₁₂H₁₅NO₃); MS (ESI): m / e = 222 (M+H+).

### 3-Amino-7-spiro-cyclopropyl-bicyclo[2.2.1 ]heptan-2-ol

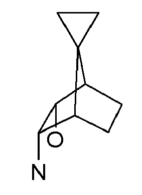

In einem Mikrowellen-Vial wurden 1.86 g Kaliumhydroxid in 4 ml Wasser gelöst und mit einer Lösung aus 1.47 g 5-Acetyl-10-spiro-cyclopropyl-3-oxa-5-aza-tricyclo[5.2.1.0<2,6>]decan-4-on in 2 ml Ethanol versetzt. Nach Verschließen des Vials mit einem Teflon-Septum wurde die Reaktionsmischung für 90 Minuten bei 120 °C in der Mikrowelle (Emrys Optimizer, Personal Chemistry) gerührt. Anschließend wurde das Lösungsmittel unter vermindertem Druck entfernt und der Rückstand zwischen Ethylacetat und Wasser verteilt. Die wässrige Phase wurde noch 2 x mit 50 ml Ethylacetat gewaschen und die vereinigten organischen Phasen mit Natriumsulfat getrocknet und das Lösungsmittel unter vermindertem Druck entfernt.
Man erhielt so das Produkt (930 mg) mit dem Molekulargewicht 153.2 g / mol (C₉H₁₅NO); MS (ESI): m / e = 154 (M+H+).

### 5-Acetyl-10,10-dimethyl-3-oxa-5-aza-tricyclo[5.2.1.0 *2,6*]decan-4-on

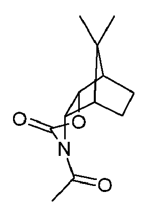

1.75 g 5-Acetyl-10-spiro-cyclopropyl-3-oxa-5-aza-tricyclo[5.2.1.0<2,6>]decan-4-on wurden in 20 ml Eisessig gelöst und unter Zusatz von 0.33 g Platindioxid für 16 Stunden unter einem Wasserstoff-Überdruck von 0.5 bar gerührt. Nach Filtration von den festen Rückständen wurde das Filtrat unter vermindertem Druck vom Lösungsmittel befreit.
Man erhielt so das Produkt (1.62 g) mit dem Molekulargewicht 223.3 g / mol (C₁₂H₁₇NO₃); MS (ESI): m / e = 224 (M+H+).

### 3-Amino-7,7-dimethyl-cyclopropyl-bicyclo[2.2.1]heptan-2-ol

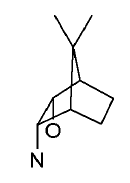

In einem Mikrowellen-Vial wurden 2.04 g Kaliumhydroxid in 4 ml Wasser gelöst und mit einer Lösung aus 1.62 g 5-Acetyl-10,10-dimethyl-3-oxa-5-aza-tricyclo[5.2.1.0*2,6*]decan-4-on in 2 ml Ethanol versetzt. Nach Verschließen des Vials mit einem Teflon-Septum wurde die Reaktionsmischung für 90 Minuten bei 120 °C in der Mikrowelle (Emrys Optimizer, Personal Chemistry) gerührt. Anschließend wurde das Lösungsmittel unter vermindertem Druck entfernt und der Rückstand zwischen Ethylacetat und Wasser verteilt. Die wässrige Phase wurde noch 2 x mit 50 ml Ethylacetat gewaschen und die vereinigten organischen Phasen mit Natriumsulfat getrocknet und das Lösungsmittel unter vermindertem Druck entfernt.
Man erhielt so das Produkt (920 mg) mit dem Molekulargewicht 155.2 g / mol (C₉H₁₇NO); MS (ESI): m / e = 156 (M+H+).

### Adamantan-1-yl-hydrazin

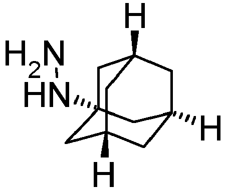

Helvetica Chimica Acta 1967, 50(7), 2008-2010

### Amino-p-carboran

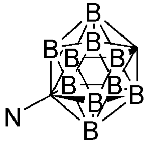

Inorganic Chemistry 1999, 38(12), 2936-2940

### Sulfamoylchlorid

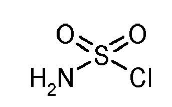

Journal of the American Chemical Society 1962, 94, 1994

### N-Sulfinyl-4-toluamid

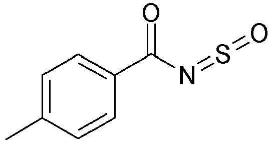

10 g p-Toluamid wurden in 100 ml Ether und 12.5 ml Pyridin unter Argon suspendiert. Die in 10 ml Ether gelösten 5.8 ml Thionylchlorid wurden bei 5 °C zugetropft und noch 5 Minuten nachgerührt. Anschließend wurde die Kühlung entfernt und der Ansatz weitere 16 Stunden bei Raumtemperatur gerührt. Unter Argon wurde dann das feste Pyridiniumhydrochlorid abfiltriert und dann die Lösung konzentriert. Der Rückstand wurde ohne weitere Aufarbeitung verwendet.

### 4a,8a-Dimethyl-3-p-tolyl-4a,5,6,7,8,8a-hexahydro-benzo[1,4,3]oxathiazin 1-oxid

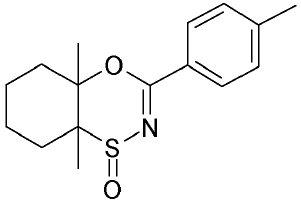

Die 3.17 g N-Sulfinyl-4-toluamid wurden in 20 ml trockenem Dichlormethan gelöst und 3.85 g 1,2-Dimethylcyclohexen bei 5 °C langsam zugetropft. Nach 16 Stunden wurde im Vakuum konzentriert und über Kieselgel chromatographiert. Man erhielt so das Produkt (1.57 g) mit dem Molekulargewicht 291.4 g / mol (C₁₆H₂₁NO₂S); MS (ESI): m / e = 292 (M+H+)
4a,8a-Dimethyl-3-p-tolyl-4a,5,6,7,8,8a-hexahydro-benzo[1,4,3]oxathiazin 1,1-dioxid

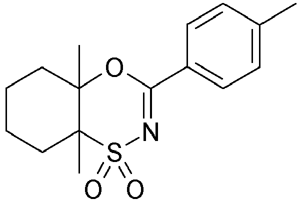

228 mg 4a,8a-Dimethyl-3-p-tolyl-4a,5,6,7,8,8a-hexahydro-benzo[1,4,3]oxathiazin 1-oxid wurden in 4 ml Dichlormethan gelöst und 190 mg m-Chlorperbenzoesäure bei 5 °C zugegeben und 16 Stunden gerührt. Die entstandene m-Chlorbenzoesäure wurde abfiltriert und das Flitrat zweimal mit 0.1 N wässriger NaOH-Lösung, einmal mit gesättigter NaCl-Lösung gewaschen, mit Natriumsulfat getrocknet, filtriert und konzentriert. Der Rückstand wurde aus n-Pentan/Diethylether umkristallisiert. Man erhielt so das Produkt (128 mg) mit dem Molekulargewicht 307.4 g / mol (C₁₆H₂₁NO₃S); MS (ESI): m / e = 308 (M+H+)

### 2-Hydroxy-1,2-dimethyl-cyclohexansulfonsäureamid

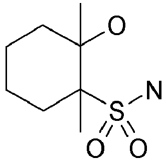

137 mg 4a,8a-Dimethyl-3-p-tolyl-4a,5,6,7,8,8a-hexahydro-benzo[1,4,3]oxathiazin 1,1-dioxid wurden in Methanol gelöst, mit 1.5 ml 6 N NaOH versetzt und über Nacht gekocht. Nach dem Abkühlen wurde mit konzentrierter Salzsäure angesäuert und das Methanol im Vakuum abgedampft. Der Rückstand wurde dann mit 10 ml Wasser versetzt und zweimal mit 10 ml n-Butanol extrahiert. Das n-Butanol wurde im Vakuum verdampft und der Rückstand (96 mg) ohne weitere Aufarbeitung verwendet.

### 4-(3,6-Dihydro-2H-pyran-4-yl)-morpholin

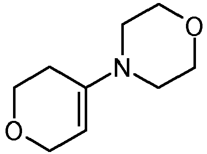

650 mg Tetrahydro-4H-pyran-4-on wurden zusammen mit 950 mg Morpholin in 10 ml Toluol gelöst und 5 Stunden in einem leichten Wasserabscheider unter Rückfluss erhitzt. Anschließend wurde im Vakuum konzentriert und Rückstand (1.3 g) ohne weitere Aufarbeitung weiterverwendet.

### 4-Oxo-tetrahydro-pyran-3-sulfonamid

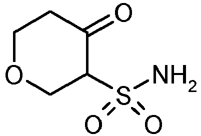

1.3 g 4-(3,6-Dihydro-2H-pyran-4-yl)-morpholin wurden unter Argon in 12 ml THF gelöst, auf -40 °C abgekühlt und dann 1.3 g Sulfamoylchlorid zugegeben gefolgt von 6 ml Diisopropylamin. Die Kühlung wurde entfernt und nachdem der Ansatz auf Raumtemperatur gekommen war, wurde noch 2 Stunden gerührt. Es schied sich ein Öl ab. Das THF wurde abdekantiert, der Rückstand in Methanol aufgenommen und durch Kieselgel filtriert. Es wurde im Vakuum konzentriert und der Rückstand, gebunden auf 12 g Celite, über eine Kieselgelsäule (50 g) gereinigt: Lösungsmittel A: Dichlormethan, B: Methanol, Gradient: 0 min 2 % B, 3 min 2 % B, 5 min 5 % B, 35 min 10 % B, 45 min 10 % B; Fluss 20 ml / min; Detektion: 220 nm, Fraktiongröße: 20 ml. Nach Entfernen des Lösungsmittels im Vakuum erhielt man so das Produkt mit Verunreinigungen. Der Rückstand (1.09 g) wurde ohne weitere Aufarbeitung weiterverwendet.

### 4-Hydroxy-tetrahydro-pyran-3-sulfonamid

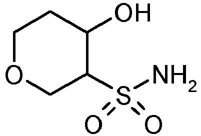

1.09 g 4-Oxo-tetrahydro-pyran-3-sulfonamid wurden in THF gelöst, 0.23 g Natriumborhydrid zugegeben und 18 Stunden bei Raumtemperatur gerührt. Der Ansatz wurde dann mit Salzsäure auf pH = 2 eingestellt und im Vakuum konzentriert. der Rückstand, gebunden auf 8 g Celite, wurde über eine Kieselgelsäule (50 g) gereinigt: Lösungsmittel A: Dichlormethan, B:
Methanol, Gradient: 0 min 3 % B, 3 min 3 % B, 5 min 5 % B, 35 min 10 % B, 44 min 10 % B; Fluss 20 ml / min; Detektion: 220 nm, Fraktionsgröße: 20 ml. Nach Entfernen des Lösungsmittels im Vakuum erhielt man so das Produkt mit Verunreinigungen. Der Rückstand (615 mg) wurde ohne weitere Aufarbeitung weiterverwendet.

### Cyclohexyl-(4a,8a-dimethyl-1,1-dioxo-4a,5,6,7,8,8a-hexahydro-1H-1lambda6-benzo[1,4,3]oxathiazin-3-yl)-amin

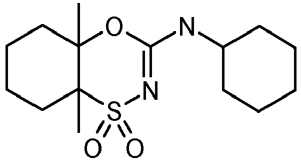

90 mg 2-Hydroxy-1,2-dimethyl-cyclohexansulfonsäureamid wurden in DMF suspendiert und anschließend mit 54 mg Kalium-tert-butylat 5 Minuten im Ultraschallbad behandelt. Dann wurden 67 mg Cyclohexylisothiocyanat zugegeben. Nach 3 Minuten rühren wurden dann 68 mg N-Bromsuccinimid zugesetzt und weitere 5 Minuten gerührt. Die Lösung wurde anschließend im Vakuum konzentriert und mit präparativer HPLC gereinigt. Man erhielt so das Produkt (3.4 mg) mit dem Molekulargewicht 314.4 g / mol; MS (ESI) m / e = 315 (M+H+).

Folgende Produkte wurden auf gleiche Weise hergestellt:
Trans-Cyclohexyl-(1,1-dioxo-5,6,8,8a-tetrahydro-1H,4aH-4,7-dioxa-1lambda*6*-thia-2-aza-naphthalen-3-yl)-amin 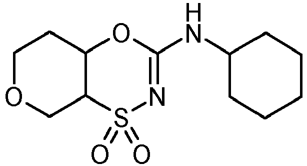
Cis-Cyclohexyl-(1,1-dioxo-5,6,8,8a-tetrahydro-1H,4aH-4,7-dioxa-1lambda*6*-thia-2-aza-naphthalen-3-yl)-amin 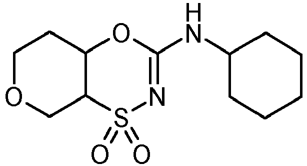
#Cyclohexyl-(1,1-dioxo-4a,5,6,7,8,8a-hexahydro-1H-1lambda*6*-benzo[1,4,3]oxathiazin-3-yl)-amin 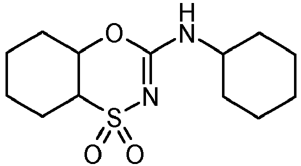 Man erhielt die Trans-Cyclohexyl-(1,1-dioxo-5,6,8,8a-tetrahydro-1H,4aH-4,7-dioxa-1lambda*6*-thia-2-aza-naphthalen-3-yl)-amine durch Trennung auf einer chiralen Säule (siehe Tabelle 1).
Cyclohexyl-(7,7-difluor-1,1-dioxo-4a,5,6,7,8,8a-hexahydro-1H-1lambda*6*-benzo[1,4,3]oxathiazin-3-yl)-amin 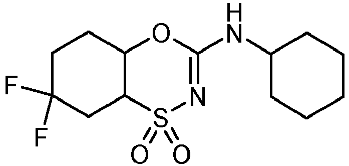
3-(2-Fluor-phenoxy)-4a-methyl-4a,5,6,7,8,8a-hexahydro-benzo[1,4,3]oxathiazin-1,1-dioxid 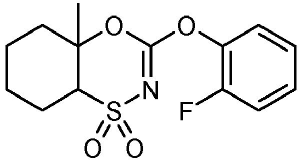 6.42 g Chlorsulfonylisocyanat wurden in 65 ml Methylenchlorid gelöst und 6.7 g 2-Fluorphenol bei Raumtemperatur hinzugegeben. Nach 1 Stunde wurde im Vakuum konzentriert, der Rückstand in 30 ml THF gelöst und auf -78 °C abgekühlt. Zu dieser Lösung wurde unter Inertgas 1.95 g Natriumhydrid 60 % zugegeben und die Kühlung entfernt. Anschließend wurde auf 35 °C langsam erwärmt und dann 1-Methyl-1-cyclohexen zugegeben. Es wurde noch 2 Stunden bei 35 °C gerührt und dann auf 100 g Eis gegossen. Das Gemisch wurde mit Ethylacetat extrahiert, mit Natriumsulfat getrocknet, filtriert und im Vakuum konzentriert. Das Rohprodukt wurde ohne weitere Aufreinigung weiterverwendet.
Cyclohexyl-(4a-methyl-1,1-dioxo-4a,5,6,7,8,8a-hexahydro-1H-1lambda*6*-benzo[1,4,3]oxathiazin-3-yl)-amin 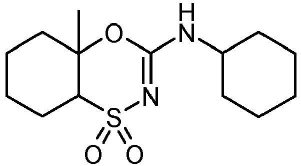 1g 3-(2-Fluor-phenoxy)-4a-methyl-4a,5,6,7,8,8a-hexahydro-benzo[1,4,3]oxathiazine-1,1-dioxid wurden in Methylenchlorid gelöst, 320 mg Cyclohexylamin und 412 mg Diisopropylamin hinzugegeben und über Nacht bei Raumtemperatur gerührt. Die Lösung wurde anschließend im Vakuum konzentriert und mit präparativer HPLC gereinigt. Man erhielt so das Produkt (20 mg) mit dem Molekulargewicht 300.4 g / mol ; MS (ESI) m / e = 301 (M+H+)

## Patentansprüche

1. Verbindungen der Formel I, 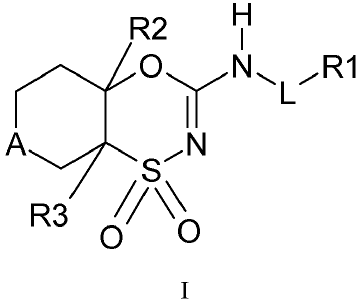 worin bedeuten
A Bindung, CH₂, CF₂, O;
L Bindung, C(R5)(R6), N(R5);
R1 (C₃-C₈)-Carbozyklus,
wobei der Carbozyklusrest ein bis 3-fach substituiert sein kann mit F, Cl, Br, I, OH, -(C₁-C₆)-Alkylen-OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁- C₆)-Alkyl)₂, SO₂-CH₃, SO₂-NH₂, SO₂-NH(C₁-C₆)-Alkyl, SO₂-N((C₁-C₆)- Alkyl)₂, COOH, COO-(C₁-C₆)-Alkyl, CONH₂, CONH(C₁-C₆)-Alkyl, CON((C₁-C₆)-Alkyl)₂, SF₅;
(C₆-C₁₀)-Aryl,
wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁- C₆)-Alkyl, NH₂, NH(C₁-C₆)-Alkyl, N((C₁-C₆)-Alkyl)₂, SO₂-CH₃, SO₂- NH₂, SO₂-NH(C₁-C₆)-Alkyl, SO₂-N((C₁-C₆)-Alkyl)₂, COOH, COO-(C₁- C₆)-Alkyl, CONH₂, CONH(C₁-C₆)-Alkyl, CON((C₁-C₆)-Alkyl)₂, SF₅;
4-12-gliedriger Heterozyklus,
wobei der Heterozyklylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO- (C₁-C₆)-Alkyl, CONH₂, CONH(C₁-C₆)-Alkyl, CON((C₁-C₆)-Alkyl)₂, SF₅;
und wobei der Heterozyklus mit einem weiteren Ring oder Ringsystem anneliert sein kann;
R2, R3 unabhängig voneinander H, (C₁-C₆)-Alkyl;
R5, R6 unabhängig voneinander H, (C₁-C₆)-Alkyl, (C₃-C₈)-Cycloalkyl, (C(R7)(R8))ₙ-O-(CO)-N(R7)(R8);
R7, R8 unabhängig voneinander H, (C₁-C₆)-Alkyl;
n 0, 1, 2;
sowie deren pharmazeutisch verträgliche Salze.

2. Verbindungen der Formel I, gemäß Anspruch 1, **dadurch gekennzeichnet, dass** darin bedeuten
A Bindung, CH₂, CF₂;
L Bindung, C(R5)(R6), N(R5);
R1 (C₃-C₈)-Carbozyklus,
wobei der Carbozyklusrest ein bis 3-fach substituiert sein kann mit F, Cl, Br, I, OH, -(C₁-C₆)-Alkylen-OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁- C₆)-Alkyl)₂, SO₂-CH₃, SO₂-NH₂, SO₂-NH(C₁-C₆)-Alkyl, SO₂-N((C₁-C₆)- Alkyl)₂, COOH, COO-(C₁-C₆)-Alkyl, CONH₂, CONH(C₁-C₆)-Alkyl, CON((C₁-C₆)-Alkyl)₂, SF₅;
Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁- C₆)-Alkyl, NH₂, NH(C₁-C₆)-Alkyl, N((C₁-C₆)-Alkyl)₂, SO₂-CH₃, SO₂- NH₂, SO₂-NH(C₁-C₆)-Alkyl, SO₂-N((C₁-C₆)-Alkyl)₂, COOH, COO-(C₁- C₆)-Alkyl, CONH₂, CONH(C₁-C₆)-Alkyl, CON((C₁-C₆)-Alkyl)₂, SF₅;
ein Heterozyklus, ausgewählt aus der Gruppe Thiophen, Pyran, Tetrahydropyran, Piperidin, Thiopyran, Tetrahydrothiopyran, Pyrrol, Tetrahydropyrrol, Azepan oder (2,3,4,5,6,7,8,9,10,11-Decaborabicyclo[8.1.1.]dodecan,
wobei der Heterozyklylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO- (C₁-C₆)-Alkyl, CONH₂, CONH(C₁-C₆)-Alkyl, CON((C₁-C₆)-Alkyl)₂, SF₅;
und wobei der Heterozyklus mit einem weiteren Ring oder Ringsystem anneliert sein kann;
R2, R3 unabhängig voneinander H, (C₁-C₆)-Alkyl;
R5, R6 unabhängig voneinander H, (C₁-C₆)-Alkyl, (C₃-C₈)-Cycloalkyl, (C₁-C₆)-Alkylen-(C₃-C₈)-Cycloalkylen, (C(R7)(R8))ₙ-O-(CO)-N(R7)(R8);
R7, R8 unabhängig voneinander H, (C₁-C₆)-Alkyl;
n 0, 1, 2;
sowie deren pharmazeutisch verträgliche Salze.

3. Verbindungen der Formel I, gemäß Anspruch 1 oder 2, **dadurch gekennzeichnet, dass** darin bedeuten
A Bindung, CH₂, CF₂;
L Bindung, C(R5)(R6), N(R5);
R1 (C₃-C₈)-Carbozyklus,
wobei der Carbozyklusrest ein bis 3-fach substituiert sein kann mit F, Cl, Br, I, OH, -(C₁-C₆)-Alkylen-OH, CF₃, NO₂, CN, OCF₃, (C₁-C₆)-Alkyl, CONH₂, CONH(C₁-C₆)-Alkyl, CON((C₁-C₆)-Alkyl)₂;
Phenyl, wobei der Phenylrest ein bis 3-fach substituiert sein kann mit F, Cl, Br, I, OH, -(C₁-C₆)-Alkylen-OH, CF₃, NO₂, CN, OCF₃, (C₁-C₆)-Alkyl, CONH₂, CONH(C₁-C₆)-Alkyl, CON((C₁-C₆)-Alkyl)₂;
ein Heterozyklus, ausgewählt aus der Gruppe Carboran, Thiophen, Tetrahydropyran, Piperidin, Thiochroman, Hexahydro-Cyclopenta [c]pyrrol, Azepan oder (2,3,4,5,6,7,8,9,10,11-Decabora bicyclo[8.1.1.]dodecan,
wobei der Heterozyklylrest ein bis 3-fach substituiert sein kann mit F, Cl, Br, I, OH, -(C₁-C₆)-Alkylen-OH, CF₃, NO₂, CN, OCF₃, (C₁-C₆)-Alkyl, CONH₂, CONH(C₁-C₆)-Alkyl, CON((C₁-C₆)-Alkyl)₂;
R2, R3 unabhängig voneinander H, (C₁-C₆)-Alkyl;
R5, R6 unabhängig voneinander H, (C₁-C₆)-Alkyl;
sowie deren pharmazeutisch verträgliche Salze.

4. Verbindungen der Formel I, gemäß einem oder mehreren der Ansprüche 1 bis 3, zur Anwendung als Arzneimittel.

5. Arzneimittel enthaltend Verbindungen der Formel I, gemäß einem oder mehreren der Ansprüche 1 bis 3.

6. Arzneimittel enthaltend Verbindungen der Formel I, gemäß einem oder mehreren der Ansprüche 1 bis 3 und mindestens einen weiteren Wirkstoff.

7. Arzneimittel, gemäß Anspruch 6, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natriumabhängigen Glukosetransporters 1 oder 2, Modulatoren des GPR40, Inhibitoren der hormonsensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten,, CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten, Lipase/Amylase-Inhibitoren, PPAR-Modulatoren, RXR-Modulatoren oder TR-β-Agonisten oder Amphetamine enthält.

8. Verfahren zur Herstellung eines Arzneimittels enthaltend die Verbindungen der Formel I, gemäß einem oder mehreren der Ansprüche 1 bis 3, **dadurch gekennzeichnet, daß** der Wirkstoff mit einem pharmazeutisch geeigneten Träger vermischt wird und diese Mischung in eine für die Verabreichung geeignete Form gebracht wird.

9. Verwendung der Verbindungen der Formel I, gemäß einem oder mehreren der Ansprüche 1 bis 3 zur Herstellung eines Medikaments zur Behandlung von Hyperglykämie.

10. Verwendung der Verbindungen der Formel I, gemäß einem oder mehreren der Ansprüche 1 bis 3 zur Herstellung eines Medikaments zur Behandlung des Diabetes.

11. Verwendung der Verbindungen der Formel I, gemäß einem oder mehreren der Ansprüche 1 bis 3 zur Herstellung eines Medikaments zur Behandlung von Insulin Resistenz.

12. Set (Kit) bestehend aus getrennten Packungen von
a) einer wirksamen Menge einer Verbindung der Formel I, gemäß einem oder mehreren der Ansprüche 1 bis 3 und
b) einer wirksamen Menge eines weiteren Arzneimittelswirkstoffs.

## Claims

1. Compounds of the formula I 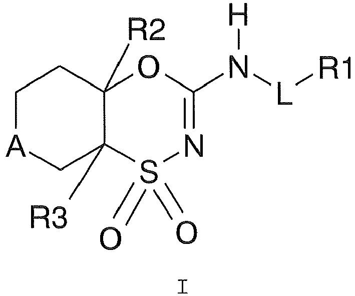 in which
A is a bond, CH₂, CF₂, 0;
L is a bond, C(R5)(R6), N(R5);
R1 (C₃-C₈)-carbocycle,
where the carbocycle radical may be mono- to trisubstituted by F, Cl, Br, I, OH, -(C₁-C₆)-alkylene-OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N ((C₁-C₆)-alkyl)₂, SO₂-CH₃, SO₂-NH₂, SO₂- NH(C₁-C₆)-alkyl, SO₂-N((C₁-C₆)-alkyl)₂, COOH, COO-(C₁-C₆)-alkyl, CONH₂, CONH(C₁- C₆)-alkyl, CON((C₁-C₆)-alkyl)₂, SF₅;
(C₆-C₁₀)-aryl,
where the aryl radical may be mono- to trisubstituted by F, Cl, Br, I, OH, CF₃, CHF₂, CH₂F, NO₂, CN, OCF₃, OCHF₂, O-(C₁- C₆)-alkyl, (C₁-C₆)-alkyl, NH₂, NH(C₁-C₆)- alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁- C₆)-alkyl, CON((C₁-C₆)-alkyl)₂, SF₅;
4-12-membered heterocycle,
where the heterocyclyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂- CH₃, SO₂-NH₂, SO₂-NH(C₁-C₆)-alkyl, SO₂- N ((C₁-C₆)-alkyl)₂, COOH, COO-(C₁-C₆)- alkyl, CONH₂, CONH(C₁-C₆)-alkyl, CON((C₁- C₆)-alkyl)₂, SF₅; and where the heterocycle may be fused to a further ring or ring system;
R2, R3 are each independently H, (C₁-C₆)-alkyl;
R5, R6 are each independently H, (C₁-C₆)-alkyl, (C₃-C₈)-cycloalkyl, (C(R7)(R8))ₙ-O-(CO)-N(R7)(R8);
R7, R8 are each independently H, (C₁-C₆)-alkyl;
n is 0, 1, 2;
and pharmaceutically acceptable salts thereof.

2. Compounds of the formula I according to Claim 1, **characterized in that**
A is a bond, CH₂, CF₂;
L is a bond, C(R5)(R6), N(R5);
R1 (C₃-C₈)-carbocycle,
where the carbocycle radical may be mono- to trisubstituted by F, Cl, Br, I, OH, -(C₁-C₆)-alkylene-OH, CF₃, CHF₂, CH₂F, NO₂, CN, OCF₃, OCHF₂, O-(C₁-C₆)-alkyl, (C₁-C₆)-alkyl , NH₂, NH (C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
phenyl,
where the phenyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
a heterocycle selected from the group of thiophene, pyran, tetrahydropyran, piperidine, thiopyran, tetrahydrothiopyran, pyrrole, tetrahydropyrrole, azepane or 2,3,4,5,6,7,8,9,10,11-decaborabicyclo[8.1.1]dodecane,
where the heterocyclyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅; and where the heterocycle may be fused to a further ring or ring system;
R2, R3 are each independently H, (C₁-C₆)-alkyl;
R5, R6 are each independently H, (C₁-C₆)-alkyl, (C₃-C₈)-cycloalkyl, (C₁-C₆)-alkylene-(C₃-C₈)-cycloalkylene, (C(R7)(R8))ₙ-O-(CO)-N(R7)(R8);
R7, R8 are each independently H, (C₁-C₆)-alkyl;
n is 0, 1, 2;
and pharmaceutically acceptable salts thereof.

3. Compounds of the formula I according to Claim 1 or 2, **characterized in that**
A is a bond, CH₂, CF₂;
L is a bond, C(R5)(R6), N(R5);
R1 (C₃-C₈)-carbocycle, where the carbocycle radical may be mono- to trisubstituted by F, Cl, Br, I, OH, -(C₁-C₆)-alkylene-OH, CF₃, NO₂, CN, OCF₃ , (C₁-C₆)-alkyl, CONH₂, CONH(C₁-C₆)-alkyl, CON((C₁-C₆)-alkyl)₂;
phenyl, where the phenyl radical may be mono- to trisubstituted by F, Cl, Br, I, OH, -(C₁-C₆)-alkylene-OH, CF₃, NO₂, CN, OCF₃, (C₁-C₆)-alkyl, CONH₂, CONH(C₁-C₆)-alkyl, CON((C₁-C₆)-alkyl)₂;
a heterocycle selected from the group of carborane, thiophene, tetrahydropyran, piperidine, thiochromane, hexahydrocyclopenta[c]pyrrole, azepane and 2,3,4,5,6,7,8,9,10,11-decaborabicyclo[8.1.1]dodecane, where the heterocycle radical may be mono- to trisubstituted by F, Cl, Br, I,
OH, -(C₁-C₆)-alkylene-OH, CF₃, NO₂, CN, OCF₃ , (C₁-C₆)-alkyl, CONH₂, CONH(C₁-C₆)-alkyl, CON((C₁-C₆)-alkyl)₂;
R2, R3 are each independently H, (C₁-C₆)-alkyl;
R5, R6 are each independently H, (C₁-C₆)-alkyl;
and pharmaceutically acceptable salts thereof.

4. Compounds of the formula I according to one or more of Claims 1 to 3 for use as medicaments.

5. Medicament comprising compounds of the formula I according to one or more of Claims 1 to 3.

6. Medicament comprising compounds of the formula I according to one or more of Claims 1 to 3 and at least one further active ingredient.

7. Medicament according to Claim 6, **characterized in that** it comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose 1,6-biphosphatase, modulators of glucose transporter 4, inhibitors of glutamine-fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, GPR40 modulators, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, decoupling protein 2 or 3 modulators, leptin agonists, DA agonists, lipase/amylase inhibitors, PPAR modulators, RXR modulators or TR β agonists or amphetamines.

8. Process for producing a medicament comprising the compounds of the formula I according to one or more of Claims 1 to 3, **characterized in that** the active ingredient is mixed with a pharmaceutically suitable carrier and this mixture is converted to a form suitable for administration.

9. Use of the compounds of the formula I according to one or more of Claims 1 to 3 for producing a medicament for treatment of hyperglycemia.

10. Use of the compounds of the formula I according to one or more of Claims 1 to 3 for producing a medicament for treatment of diabetes.

11. Use of the compounds of the formula I according to one or more of Claims 1 to 3 for producing a medicament for treatment of insulin resistance.

12. Set (kit) consisting of separate packages of
a) an effective amount of a compound of the formula I according to one or more of Claims 1 to 3 and
b) an effective amount of a further active medicament ingredient.

## Revendications

1. Composés de formule I, 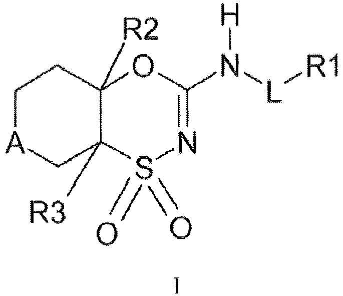 dans laquelle
A représente une liaison, CH₂, CF₂, O ;
L représente une liaison, C(R5)(R6), N(R5) ;
R1 représente un carbocycle en C₃-C₈, le radical carbocyclique pouvant être une à 3 fois substitué par F, Cl, Br, I, OH, -alkylène(C₁-C₆)-OH, CF₃, CHF₂, CH₂F, NO₂, CN, OCF₃, OCHF₂, O-alkyle(C₁-C₆), alkyle en C₁-C₆, NH₂, NH-alkyle(C₁-C₆), N(alkyle(C₁-C₆))₂, SO₂-CH₃, SO₂-NH₂, SO₂-NH-alkyle(C₁-C₆), SO₂-N(alkyle(C₁-C₆))₂, COOH, COO-alkyle(C₁-C₆), CONH₂, CONH-alkyle(C₁-C₆), CON(alkyle(C₁-C₆))₂, SF₅ ;
un radical aryle en C₆-C₁₀,
le radical aryle pouvant être une à 3 fois substitué par F, Cl, Br, I, OH, CF₃, CHF₂, CH₂F, NO₂, CN, OCF₃, OCHF₂, O-alkyle(C₁-C₆), alkyle en C₁-C₆, NH₂, NH-alkyle(C₁-C₆), N(alkyle(C₁-C₆))₂, SO₂-CH₃, SO₂-NH₂, SO₂-NH-alkyle(C₁-C₆), SO₂-N(alkyle(C₁-C₆))₂, COOH, COO-alkyle(C₁-C₆), CONH₂, CONH-alkyle(C₁-C₆), CON(alkyle(C₁-C₆))₂, SF₅ ;
un hétérocycle à 4-12 chaînons,
le radical hétérocyclyle pouvant être une à 3 fois substitué par F, Cl, Br, I, OH, CF₃, CHF₂, CH₂F, NO₂, CN, OCF₃, OCHF₂, O-alkyle(C₁-C₆), alkyle en C₁-C₆, NH₂, NH-alkyle(C₁-C₆), N(alkyle(C₁-C₆))₂, SO₂-CH₃, SO₂-NH₂, SO₂-NH-alkyle(C₁-C₆), SO₂-N(alkyle(C₁-C₆))₂, COOH, COO-alkyle(C₁-C₆), CONH₂, CONH-alkyle(C₁-C₆), CON(alkyle(C₁-C₆))₂, SF₅ ; et l'hétérocycle pouvant être soudé à un autre cycle ou système cyclique ;
R2, R3 représentent indépendamment l'un de l'autre H, un groupe alkyle en C₁-C₆,
R5, R6 représentent indépendamment l'un de l'autre H, un groupe alkyle en C₁-C₆, cycloalkyle en C₃-C₈, (C(R7)(R8))ₙ-O-(CO)-N(R7)(R8) ;
R7, R8 représentent indépendamment l'un de l'autre H, un groupe alkyle en C₁-C₆ ;
n vaut 0, 1, 2 ;
ainsi que leurs sels pharmaceutiquement acceptables.

2. Composés de formule I, selon la revendication 1, **caractérisés en ce que** dans ceux-ci
A représente une liaison, CH₂, CF₂ ;
L représente une liaison, C(R5)(R6), N(R5) ;
R1 représente un carbocycle en C₃-C₈, le radical carbocyclique pouvant être une à 3 fois substitué par F, Cl, Br, I, OH, -alkylène(C₁-C₆)-OH, CF₃, CHF₂, CH₂F, NO₂, CN, OCF₃, OCHF₂, O-alkyle(C₁-C₆), alkyle en C₁-C₆, NH₂, NH-alkyle(C₁-C₆), N(alkyle(C₁-C₆))₂, SO₂-CH₃, SO₂-NH₂, SO₂-NH-alkyle(C₁-C₆), SO₂-N(alkyle( C₁-C₆))₂, COOH, COO-alkyle(C₁-C₆), CONH₂, CONH-alkyle(C₁-C₆), CON(alkyle(C₁-C₆))₂, SF₅ ;
un radical phényle,
le radical phényle pouvant être une à 3 fois substitué par F, Cl, Br, I, OH, CF₃, CHF₂, CH₂F, NO₂, CN, OCF₃, OCHF₂, O-alkyle(C₁-C₆), alkyle en C₁-C₆, NH₂, NH-alkyle(C₁-C₆), N(alkyle(C₁-C₆))₂, SO₂-CH₃, SO₂-NH₂, SO₂-NH-alkyle(C₁-C₆), SO₂-N(alkyle(C₁-C₆))₂, COOH, COO-alkyle(C₁-C₆), CONH₂, CONH-alkyle(C₁-C₆), CON(alkyle(C₁-C₆))₂, SF₅ ;
un hétérocycle choisi dans le groupe constitué par les cycles thiophène, pyrane, tétrahydropyrane, pipéridine, thiopyrane, tétrahydrothiopyrane, pyrrole, tétrahydropyrrole, azépane ou (2,3,4,5,6,7,8,9,10,11-décaborabicyclo[8.1.1.]-dodécane,
le radical hétérocyclyle pouvant être une à 3 fois substitué par F, Cl, Br, I, OH, CF₃, CHF₂, CH₂F, NO₂, CN, OCF₃, OCHF₂, O-alkyle(C₁-C₆), alkyle en C₁-C₆, NH₂, NH-alkyle(C₁-C₆), N(alkyle(C₁-C₆))₂, SO₂-CH₃, SO₂-NH₂, SO₂-NH-alkyle(C₁-C₆), SO₂-N(alkyle(C₁-C₆))₂, COOH, COO-alkyle(C₁-C₆), CONH2, CONH-alkyle(C₁-C₆), CON(alkyle(C₁-C₆))₂, SF₅ ; et l'hétérocycle pouvant être soudé à un autre cycle ou système cyclique ;
R2, R3 représentent indépendamment l'un de l'autre H, un groupe alkyle en C₁-C₆,
R5, R6 représentent indépendamment l'un de l'autre H, un groupe alkyle en C₁-C₆, cycloalkyle en C₃-C₈, alkylène(C₁-C₆)-cycloalkylène(C₃-C₈), (C(R7)(R8))ₙ-O-(CO)-N(R7)(R8) ;
R7, R8 représentent indépendamment l'un de l'autre H, un groupe alkyle en C₁-C₆ ;
n vaut 0, 1, 2 ;
ainsi que leurs sels pharmaceutiquement acceptables.

3. Composés de formule I, selon la revendication 1 ou 2, **caractérisés en ce que** dans ceux-ci
A représente une liaison, CH₂, CF₂ ;
L représente une liaison, C(R5)(R6), N(R5) ;
R1 représente un carbocycle en C₃-C₈, le radical carbocyclique pouvant être une à 3 fois substitué par F, Cl, Br, I, OH, -alkylène(C₁₋C₆)-OH, CF₃, NO₂, CN, OCF₃, alkyle en C₁-C₆, CONH₂, CONH-alkyle(C₁-C₆), CON(alkyle(C₁-C₆))₂ ;
un radical phényle,
le radical phényle pouvant être une à 3 fois substitué par F, Cl, Br, I, OH, -alkylène(C₁-C₆)-OH, CF₃, NO₂, CN, OCF₃, alkyle en C₁-C₆, CONH₂, CONH-alkyle(C₁-C₆), CON(alkyle(C₁-C₆))₂ ;
un hétérocycle choisi dans le groupe constitué par les cycles carborane, thiophène, tétrahydropyrane, pipéridine, thiochromane, hexahydro-cyclopenta[c]-pyrrole, azépane ou (2,3,4,5,6,7,8,9,10,11-décaborabicyclo-[8.1.1.]dodécane,
le radical hétérocyclyle pouvant être une à 3 fois substitué par F, Cl, Br, I, OH, -alkylène(C₁₋C₆)-OH, CF₃, NO₂, CN, OCF₃, alkyle en C₁-C₆, CONH₂, CONH-alkyle(C₁-C₆), CON(alkyle(C₁-C₆))₂ ;
R2, R3 représentent indépendamment l'un de l'autre H, un groupe alkyle en C₁-C₆,
R5, R6 représentent indépendamment l'un de l'autre H, un groupe alkyle en C₁-C₆ ;
ainsi que leurs sels pharmaceutiquement acceptables.

4. Composés de formule I, selon une ou plusieurs des revendications 1 à 3, pour utilisation en tant que médicament.

5. Médicament contenant des composés de formule I, selon une ou plusieurs des revendications 1 à 3.

6. Médicament contenant des composés de formule I, selon une ou plusieurs des revendications 1 à 3, et au moins une autre substance active.

7. Médicament selon la revendication 6, **caractérisé en ce qu'**il contient comme autre substance active un(e) ou plusieurs antidiabétiques, principes actifs hypoglycémiants, inhibiteurs de HMGCoA-réductase, inhibiteurs de la résorption du cholestérol, agonistes de PPAR gamma, agonistes de PPAR alpha, agonistes de PPAR alpha/gamma, agonistes de PPAR delta, fibrates, inhibiteurs de MTP, inhibiteurs de la résorption d'acides biliaires, inhibiteurs de CETP, adsorbeurs polymères d'acides biliaires, inducteurs des récepteurs de LDL, inhibiteurs d'ACAT, antioxydants, inhibiteurs de lipoprotéine lipase, inhibiteurs d'ATP-citrate lyase, inhibiteurs de squalène synthétase, antagonistes de lipoprotéine (a), agonistes de récepteurs HM74A, inhibiteurs de lipase, insulines, sulfonylurées, biguanides, méglitinides, thiazolidinediones, inhibiteurs d'α-glucosidase, principes actifs agissant sur le canal potassique ATP-dépendant des cellules bêta, inhibiteurs de glycogène phosphorylase, antagonistes des récepteurs du glucagon, activateurs de la glucokinase, inhibiteurs de la gluconéogenèse, inhibiteurs de la fructose-1,6-biphosphatase, modulateurs du transporteur de glucose-4, inhibiteurs de la glutamine-fructose-6-phosphate-amidotransférase, inhibiteurs de la dipeptidylpeptidase-IV, inhibiteurs de la 11-bêta-hydroxystéroïde-déshydrogénase-1, inhibiteurs de la protéine-tyrosine-phosphatase-1B, modulateurs du transporteur de glucose dépendant du sodium 1 ou 2, modulateurs du GPR40, inhibiteurs de la lipase hormonosensible, inhibiteurs de l'acétyl-CoA carboxylase, inhibiteurs de la phosphoénolpyruvate carboxykinase, inhibiteurs de la glycogène synthase kinase-3 bêta, inhibiteurs de la protéine kinase C bêta, antagonistes des récepteurs d'endothéline A, inhibiteurs de la I kappaB kinase, modulateurs du récepteur de glucocorticoïdes, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes de β3, antagonistes des récepteurs CB1, agonistes de MSH (hormone simulant les mélanocytes), agonistes de CCK, inhibiteurs de la recapture de sérotonine, composés sérotoninergiques et noradrénergiques mixtes, agonistes de 5HT, agonistes de bombésine, antagonistes de galanine, hormones de croissance, composés libérant l'hormone de croissance, agonistes de TRH, modulateurs de protéine 2 ou 3 découpleurs, agonistes de leptine, agonistes de DA, inhibiteurs de lipase/amylase, modulateurs de PPAR, modulateurs de RXR ou agonistes de TR-β ou amphétamines.

8. Procédé pour la fabrication d'un médicament contenant les composés de formule I, selon une ou plusieurs des revendications 1 à 3, **caractérisé en ce qu'**on mélange la substance active avec un véhicule pharmaceutiquement convenable et on met ce mélange sous une forme appropriée à l'administration.

9. Utilisation des composés de formule I, selon une ou plusieurs des revendications 1 à 3, pour la fabrication d'un médicament destiné au traitement de l'hyperglycémie.

10. Utilisation des composés de formule I, selon une ou plusieurs des revendications 1 à 3, pour la fabrication d'un médicament destiné au traitement du diabète.

11. Utilisation des composés de formule I, selon une ou plusieurs des revendications 1 à 3, pour la fabrication d'un médicament destiné au traitement de la résistance à l'insuline.

12. Nécessaire (kit) constitué d'emballages séparés de
a) une quantité efficace d'un composé de formule I, selon une ou plusieurs des revendications 1 à 3 et
b) une quantité efficace d'une autre substance active médicamenteuse.
